# EUROPÄISCHE PATENTSCHRIFT

(11) **EP 3 826 627 B1**
(45) Veröffentlichungstag und Bekanntmachung des Hinweises auf die Patenterteilung: **18.10.2023**
(21) Anmeldenummer: 19740580.6
(22) Anmeldetag: 19.07.2019
(51) Int. Cl.: A61K 31/196, A61K 9/20, A61K 9/28, A61P 13/12, C07C 233/55

(54) **ORAL APPLIZIERBARE PHARMAZEUTISCHE DARREICHUNGSFORM MIT MODIFIZIERTER FREISETZUNG**
ORALLY ADMINISTERABLE PHARMACEUTICAL DOSAGE FORM WITH MODIFIED RELEASE
FORME D'ADMINISTRATION PHARMACEUTIQUE APPLICABLE PAR VOIE ORALE À LIBÉRATION MODIFIÉE

(30) Priorität: 24.07.2018 EP 18185127; 24.07.2018 US 201816043567
(43) Veröffentlichungstag der Anmeldung: 02.06.2021
(73) Patentinhaber: Bayer Aktiengesellschaft, 51373 Leverkusen (DE); Bayer Pharma Aktiengesellschaft, 13353 Berlin (DE)
(72) Erfinder: STROYER, Anke, 12159 Berlin (DE); LOBBACK, Carmen, 15566 Schöneiche (DE); SERNO, Peter, 51467 Bergisch Gladbach (DE); LOVIS, Kai, 40215 Düsseldorf (DE); RUBENBAUER, Philipp, 64625 Bensheim (DE); SCHIRMER, Heiko, 42655 Solingen (DE); GROSSBACH, Danja, 42329 Wuppertal (DE); JACOBS, Tia, 42115 Wuppertal (DE); OLENIK, Britta, 46242 Bottrop (DE); KÜSEL, Julia, 44879 Bochum (DE); BIERER, Donald, 42781 Haan (DE)
(74) Vertreter: BIP Patents
(86) Internationale Anmeldenummer: PCT/EP2019/069561
(87) Internationale Veröffentlichungsnummer: WO 2020/020789

(56) Entgegenhaltungen:
- US-B2- 10 023 528
- RASHMI SAREEN ET AL: "An Insight to Osmotic Drug Delivery", CURRENT DRUG DELIVERY, Bd. 9, Nr. 3, April 2012 (2012-04), Seiten 285-296, XP055545735, NL ISSN: 1567-2018, DOI: 10.2174/156720112800389106 in der Anmeldung erwähnt

## Beschreibung

Die vorliegende Erfindung betrifft oral applizierbare pharmazeutische Darreichungsformen mit modifizierter Freisetzung enthaltend Natrium-(3S)-3-(4-chlor-3-{[(2S,3R)-2-(4-chlorphenyl)-4,4,4-trifluor-3-methylbutanoyl]amino}phenyl)-3-cyclopropylpropanoat sowie Verfahren zur Herstellung der Darreichungsformen und ihre Verwendung zur Behandlung und/oder Prävention von Krankheiten, insbesondere zur Behandlung und/oder Prävention von kardialen, renalen, pulmonalen und ophthalmologischen Erkrankungen, Erkrankungen des Zentralnervensystems, fibrotischen und inflammatorischen Erkrankungen und Stoffwechselerkrankungen.

In WO 2012/139888 ist die Verbindung (3S)-3-(4-Chlor-3-{[(2S,3R)-2-(4-chlorphenyl)-4,4,4-trifluor-3-methylbutanoyl]amino}phenyl)-3-cyclopropylpropansäure der Formel (I), 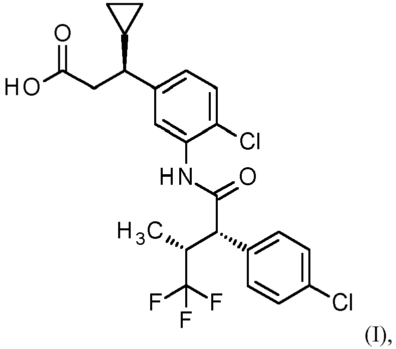 und dessen Herstellung in Beispiel 22 offenbart. Die Verbindung der Formel (I) wirkt als Aktivator der löslichen Guanylatcyclase. Das Dokument offenbart zudem, dass die beschriebenen chemischen Verbindungen generell in Tabletten, oral applizierbare Suspensionen und oral applizierbare Lösungen überführt werden können. Diese pharmazeutischen Darreichungsformen stellen ausschließlich schnell freisetzende pharmazeutische Zusammensetzungen dar.

Für Krankheiten, die über einen längeren Zeitraum behandelt werden müssen, oder zur längerfristigen Prophylaxe von Krankheiten, ist es wünschenswert, die Häufigkeit der Einnahme von Medikamenten so gering wie möglich zu halten. Dies ist nicht nur bequemer für den Patienten, sondern es erhöht auch die Behandlungssicherheit, indem es die Nachteile unregelmäßiger Einnahmen vermindert. Die gewünschte Reduktion der Einnahmefrequenz, beispielsweise von zweimal täglich auf einmal tägliche Gabe, kann über eine Verlängerung der therapeutisch effektiven Plasmaspiegel durch modifizierte Wirkstofffreisetzung aus den Darreichungsformen erreicht werden.

Nach Einnahme von Darreichungsformen mit modifizierter Wirkstofffreisetzung können außerdem durch Glättung des Plasmaspiegelverlaufes Nebenwirkungen vermindert werden. Durch Minimierung des sogenannten *peak-trough-*Verhältnisses, also durch Vermeidung von hohen Plasmawirkstoffkonzentrationen, die häufig nach Gabe schnellfreisetzender Arzneiformen zu beobachten sind, kann das Auftreten unerwünschter, mit den Konzentrationsspitzen korrelierten Nebenwirkungen vermindert werden. Daher sollte eine Arzneiform mit modifizierter Freisetzung entwickelt werden. Hierbei wurde ein osmotisches Freisetzungssystem ausgewählt, um das geforderte Profil einer gleichmäßigen, langanhaltenden und vollständigen Wirkstofffreisetzung über einen variablen zuvor definierten Zeitraum zu gewährleisten. Osmotische Freietzungssysteme zeichen sich gegenüber anderen Darreichungssystemen mit verzögerter Freisetzung beispielsweise dadurch aus, dass sich die Freisetzungsprofile flexibel durch die Einstellung der Dicke der Hülle einstellen lassen (Kaushal, A.M., Garg, S. An Update on Osmotic Drug Delivery Patents. Pharmaceutical Technology. 2003.13(1):8-97).

Osmotische Freisetzungssysteme werden auch als Gastrointestinale Therapeutische Systeme (GITS) oder Orale Osmotische Systeme (OROS) bezeichnet. Die langanhaltende und gleichmäßige Freisetzung eines Wirkstoffes wird vom osmotischen Druck kontrolliert.

Bei osmotischen Freisetzungssystemen kann zwischen Einkammersystemen (*elementary osmotic pump*) und Zweikammersystemen (*push-pull*-*Systeme*) unterschieden werden.

Beim Einkammersystem werden ein oder mehrere osmotisch aktive Stoffe mit dem Wirkstoff vermischt und verpresst. Diese Kerne werden mit einer semipermeablen Membran umgeben, die mindestens eine Öffnung aufweist. Diese semipermeable Membran, im Folgenden als Hülle bezeichnet, ist für die Komponenten des Kerns undurchlässig, erlaubt aber den Eintritt des Wassers von außen über Osmose. Das eingedrungene Wasser setzt dann über den entstehenden osmotischen Druck den Wirkstoff in gelöster oder suspendierter Form aus einer oder mehreren Öffnungen in der Hülle frei. Die Gesamtwirkstofffreisetzung und die Freisetzungsrate können im Wesentlichen über die Dicke und Porosität der Hülle, die Zusammensetzung des Kerns und die Anzahl und Größe der Öffnungen gesteuert werden.

Beim Zweikammersystem enthält eine Kammer den Wirkstoff, die andere Kammer den osmotisch aktiven Stoff. Beide Kammern können durch eine flexible Trennwand getrennt sein. Dieser Kern wird ebenfalls mit einer Hülle umgeben, die mindestens eine Öffnung auf der Seite der wirkstoffhaltigen Kammer aufweist.

Vorteile, Formulierungsaspekte, Anwendungsformen und Informationen zu Herstellverfahren von osmotischen Freisetzungssystemen sind u. a. in folgenden Publikationen beschrieben:
- Kaushal, A.M., Garg, S.: "An Update on Osmotic Drug Delivery Patents", Pharmaceutical Technology 2003, 13, 8-97.
gekennzeichnet, dass die pharmazeutische Darreichungsform auf einem osmotischen Zweikammersystem basiert.

In einer Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II) und mindestens ein hydrophiles quellbares Polymer enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II) und mindestens ein hydrophiles quellbares Polymer, das ausgewählt ist aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder bevorzugt ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II), mindestens einem hydrophilen quellbaren Polymer, das ausgewählt ist aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder bevorzugt ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, gegebenenfalls mindestens einen pharmazeutisch üblichen Hilfsstoff und gegebenenfalls einen osmotisch aktiven Zusatz enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II), mindestens ein hydrophiles quellbares Polymer, ausgewählt aus einer Liste bestehend aus Bei der Weiterverarbeitung des aufwendig gesiebten, wirkstoffhaltigen Walzengranulates traten weitere nachteilige Effekte bei der Verpressung der Tabletten auf. Bereits im Zuführtrichter wurde eine sogenannte Brückenbildung beobachtet, welche das Verhaken der Granulatkörner aufgrund der groben Oberfläche der Granulatkörner beschreibt. Die pressfertige Mischung konnte daher nicht ohne zusätzliche Agitation fließen. Somit war keine durchgängige Tablettierung des Granulates als pressfertige Mischung möglich. Auch hier musste der Herstellprozess abgebrochen werden. Die Geräteteile der Tablettiermaschine wie Stempel, Matritze und Rundläufertisch zeigten starke Anhaftungen der wirkstoffhaltigen Pressmischung. Die wenigen erhaltenen Tabletten zeigten eine sogenannte Deckelneigung, wobei sich der obere oder der untere Teil der Tablette bei Auswurf aus der Tablettenpresse oder während des Bearbeitungsprozesses horizontal ganz oder teilweise vom Hauptteil ablöste und eine Kappe bildete. Solche Tabletten entsprechen nicht den Bedingungen für eine akzeptable pharmazeutische Qualität und sind nicht mehr verwendbar.

Eine Entnahme und Analyse verschiedener Proben der wirkstoffhaltigen Pulvermischung vor Granulation, der kunststoffartigen Masse vor Siebung, der kunststoffartigen Masse nach Zerkleinerung und Siebung und des Rückstands auf dem Mahlsieb zeigten starke Schwankungen im Gehalt der Verbindung der Formel (I). Ausgehend von 100 % des deklarierten Wirkstoffgehaltes in der wirkstoffhaltigen Pulvermischung vor Granulation zeigten die Proben Gehaltswerte von 107 % bis 120 % bezogen auf den deklarierten Wirkstoffgehalt. Die durchweg erhöhten Gehaltswerte sind wahrscheinlich darauf zurückzuführen, dass bei der Herstellung lediglich ein Teil des Walzengranulats schmilzt und die Verbindung der Formel (I) heterogen verteilt vorliegt. Eine pharmazeutische Darreichungsform mit derartigen Abweichungen des Wirkstoffgehalts ist nicht akzeptabel und für die weitere Entwicklung nicht verwendbar. Es ist davon auszugehen, dass die gemessenen Gehaltsschwankungen der Pulvermischung auch zu Gehaltschwankungen in einer daraus hergestellten Tablette führen und dass diese Tabletten somit nicht den Anforderungen des Arzneibuchs, beispielsweise der Gehaltseinheitlichkeit (Ph. Eur. Ausgabewerk 9; 2.9.40 "Uniformity of Dosage Units") entsprechen.

Überraschenderweise konnte durch den Ersatz der Verbindung der Formel (I) durch das Natrium-Salz dieser Verbindung, nämlich Natrium-(3S)-3-(4-chlor-3-{[(2S,3R)-2-(4-chlorphenyl)-4,4,4-trifluor-3-methylbutanoyl]amino}phenyl)-3-cyclopropylpropanoat der Formel (II) 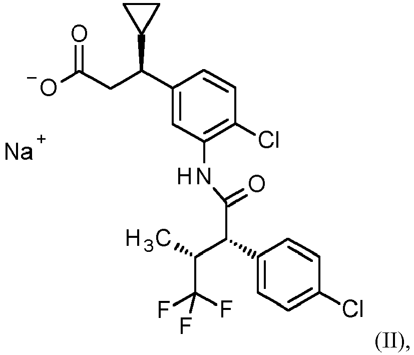 ein osmotisches Freisetzungssystem erhalten werden, das weder die beschriebenen nachteiligen Eigenschaften des osmotischen Freisetzungssystems enthaltend die Verbindung der Formel (I), noch die Nachteile bei der beschriebenen Herstellung des osmotischen Freisetzungssystems enthaltend die Verbindung der Formel (I) aufweist. Beim Einsatz der Verbindung der Formel (II) wurden keinerlei Schmelzphänomene oder weitere nachteilige Beobachtungen während der einzelnen Herstellschritte gemacht. Der Herstellprozess konnte ohne ungeplante Unterbrechungen vollständig ausgeführt werden. Die Gehaltsbestimmung ergab spezifikationsgerechte Resultate im Hinblick auf den deklarierten Wirkstoffgehalt.

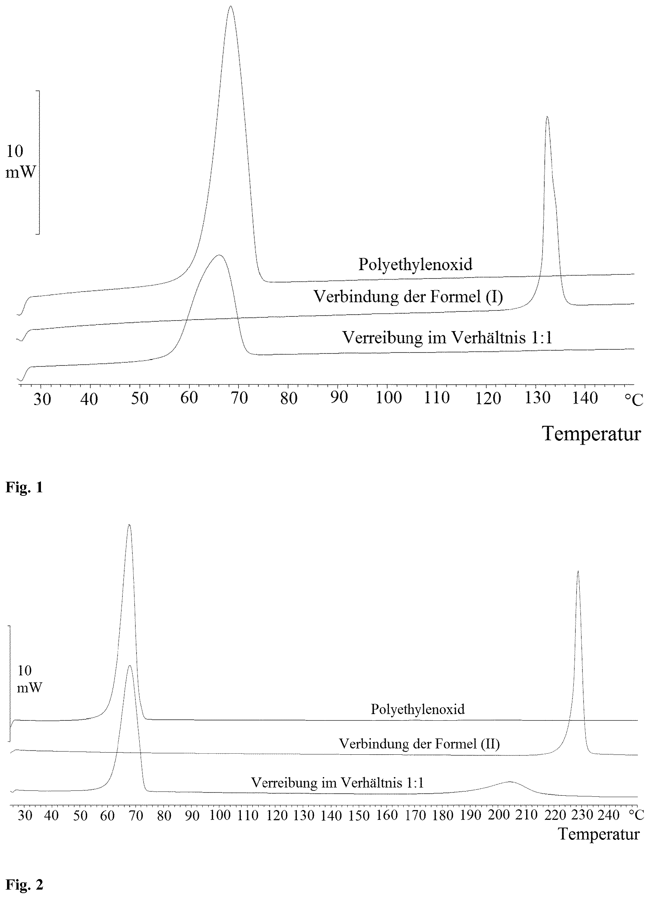

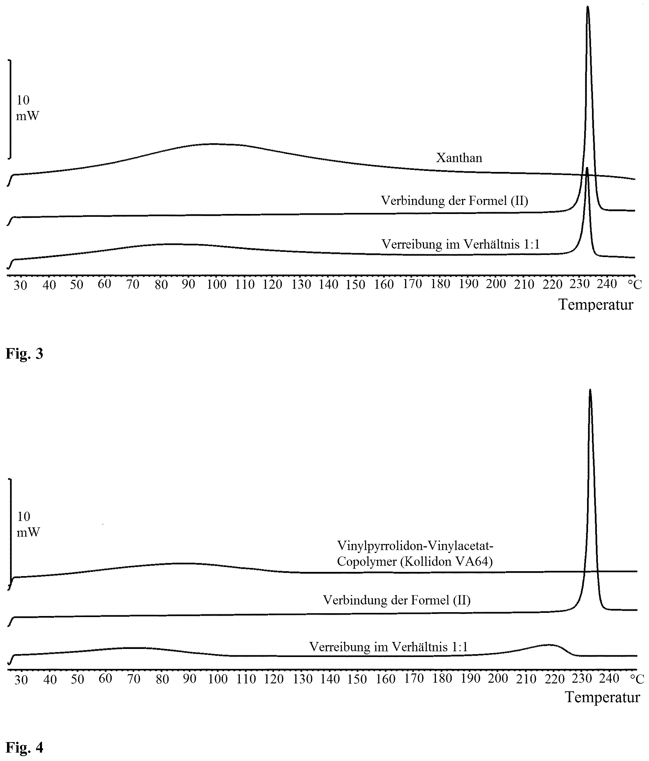

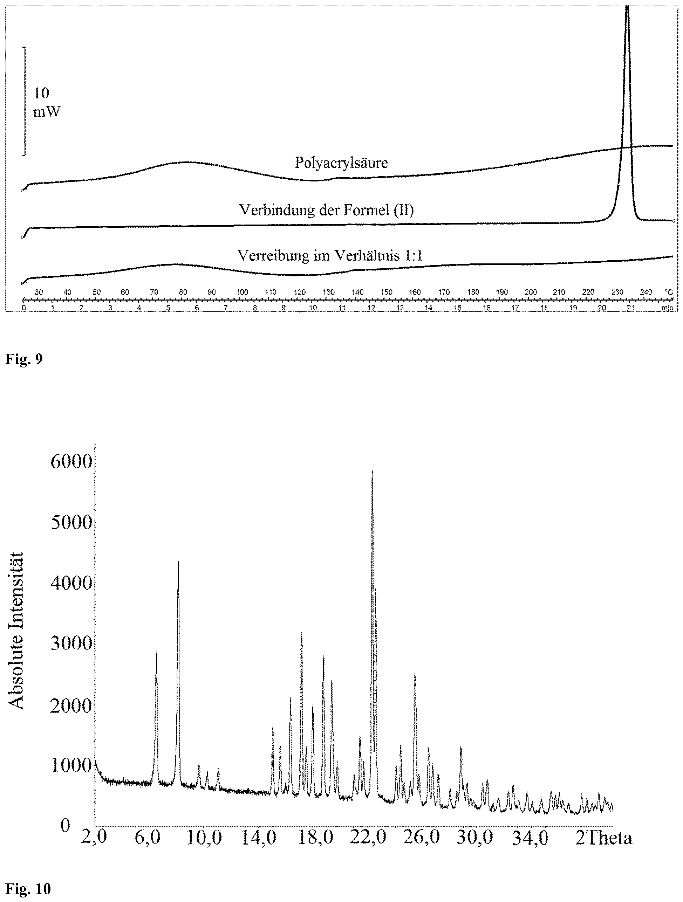

Das unterschiedliche Verhalten einer Mischung der Verbindung der Formel (I) und Polyethylenoxid im Vergleich zu einer Mischung der Verbindung der Formel (II) und Polyethylenoxid kann zusätzlich durch Messung der DSC (*differential scanning calorimetry*) Thermogramme der jeweiligen Stoffe alleine und in Verreibungen im Verhältnis 1:1 (binären Mischungen) gezeigt werden. Die in den Thermogrammen zu beobachtenden Veränderungen sind indikativ für die Prozessierbarkeit der Pulvermischung. Eine Verreibung, die zu gleichen Teilen die Verbindung der Formel (I) und Polyethylenoxid enthält, zeigt keinen Schmelzpeak, welcher der Verbindung der Formel (I) zuzuordnen ist (Fig. 1). Ein Verschwinden des Schmelzpeaks des Wirkstoffes, eine Verbreiterung des Schmelzpeaks des hydrophilen quellbaren Polymers und ein früherer Schmelzbeginn korrelieren mit den geschilderten Mängeln der Verarbeitbarkeit. Der Schmelzvorgang beginnt daher schon bei einer Temperatur zwischen 50°C und 60°C. Diese Temperaturen können bei der Herstellung der osmotischen Freisetzungsysteme entstehen und die beschriebenen Schmelzphänomene verursachen. Eine Verreibung, die zu gleichen Teilen die Verbindung der Formel (II) und Polyethylenoxid enthält, zeigt neben dem Schmelzpeak von Polyethylenoxid auch einen zusätzlichen Schmelzpeak, welcher der Verbindung der Formel (II) zuzuordnen ist (Fig. 2). Der Schmelzbereich der Verbindung der Formel (II) ist in der Verreibung verringert, liegt allerdings in einem Temperaturbereich, der bei der Herstellung der osmotischen Freisetzungsysteme nicht erreicht wird. Zudem verringert die Verbindung der Formel (II) im Gegensatz zur Verbindung der Formel (I) nicht die Schmelztemperatur von Polyethylenoxid. Daher sind die Schmelzphänomene bei dieser Kombination nicht zu beobachten. Verreibungen im Verhältnis 1:1 der Verbindung der Formel (II) mit Xanthan, Vinylpyrrolidon-Vinylacetat-Copolymer (Kollidon VA 64), Polyvinylpyrrolidon (PVP 25), Hydroxypropylcellulose (HPC LM), anionischen Copolymeren von Methacrylsäure und Methylmethacrylaten (Eudragit L100, Eudragit RL PO) zeigen ebenfalls einen Schmelzpeak der Verbindung der Formel (II), welcher in einem hohen Temperaturbereich liegt (Fig. 3 bis Fig. 8). Da diese Polymere amorph vorliegen, ist im gemessenen Bereich kein Schmelzpeak zu erkennen, der den Polymeren zuzuordnen ist. Das Thermogramm einer Verreibung der Verbindung der Formel (II) und Polyacrylsäure zeigt keinen Schmelzpeak, der der Verbindung der Formel (II) zuzuordnen ist (Fig. 9). Es liegt die Vermutung nahe, dass sich die Verbindung der Formel (II) nach der Glasübergangstemperatur von Polyacrylsäure mit steigender Temperatur löst. Da die Glasübergangstemperatur bei ca. 106°C liegt, sind Schmelzphänomene während der Herstellung des osmotischen Freisetzungssystems mit Polyacrylsäure als hydrophiles quellbares Polymeren nicht zu erwarten.

Es wurden Versuche unternommen eine Vielzahl weiterer pharmazeutisch verwendbarer Salze der Verbindung der Formel (I) herzustellen. Darunter befanden sich Kalium-, Cholin-, Bicarbonat-, Natriumcarbonat-, (Diethylamino)ethanol-, L-Lysin-, Tris-, N-Methyl-D-Glucamin-, L-Arginin-, Natriumbicarbonat- und Kaliumbicarbonat-Salze der Verbindung der Formel (I). Für die Entwicklung einer Arzneiform besteht ein hoher Bedarf dafür, dass man den Wirkstoff reproduzierbar in einer definierten kristallinen Form isoliert. Die amorphen Formen sind für die Herstellung von pharmazeutischen Darreichungsformen ungeeignet, da sie häufig eine geringere thermodynamische Stabilität zeigen und nachteilige Eigenschaften für die Formulierbarkeit von pharmazeutischen Darreichungsformen, beispielsweise schlechte Mikronisierbarkeit, Adhäsivität oder schlechte Tablettierbarkeit, aufweisen. Zusätzlich sollte die kristalline Form des Wirkstoffs eine reproduzierbare Bioverfügbarkeit zeigen und während des Mikronisierprozesses stabil bleiben, so dass keine Umwandlung und Rekristallisation stattfindet.

Überraschenderweise stellte sich heraus, dass lediglich das Natrium-Salz der Verbindung der Formel (I) in kristalliner Form erhalten werden konnte und die kristalline Form des Natrium-Salzes der Verbindung der Formel (II) die beschriebenen vorteilhaften Eigenschaften aufweist. Diese kristalline Form wird im Folgenden als Verbindung der Formel (II) in kristalliner Form der Modifikation 1 bezeichnet.

Alle weiteren getesteten Salze der Verbindung der Formel (I) konnten nicht in kristalliner Form erhalten werden, weshalb bevorzugt die Verbindung der Formel (II) für die Herstellung eines osmotischen Freisetzungssystems verwendet wurde.

Ein Gegenstand der vorliegenden Erfindung ist eine feste, oral applizierbare, Natrium-(3S)-3-(4-chlor-3-{[(2S,3R)-2-(4-chlorphenyl)-4,4,4-trifluor-3-methylbutanoyl]amino}phenyl)-3-cyclopropylpropanoat der Formel (II) enthaltende pharmazeutische Darreichungsform mit modifizierter Freisetzung, dadurch gekennzeichnet, dass 80 % der Verbindung der Formel (II) in einem Zeitraum von 2 bis 24, bevorzugt 4 bis 20 Stunden freigesetzt werden, gemessen gemäß USP-Freisetzungsmethode (USP 39; Kapitel <711> Dissolution) mit Apparatur 2 (Paddle) und der Angaben im Kapitel "Freisetzungsverhalten".

Für die Formulierung der Verbindungen der Formel (II) in Form eines osmotisches Freisetzungssystems sind sowohl Zweikammersysteme (*push-pull-Systeme*) als auch Einkammersysteme (*elementary osmotic pump*) geeignet. Sowohl das Zweikammersystem als auch das Einkammersystem bestehen aus einem Kern, der mit einer Hülle und optional mit einem Lack überzogen ist. Die Verbindung der Formel (II) kann in den osmotischen Freisetzungssystemen sowohl in kristalliner, als auch in amorpher Form vorliegen oder in Mischungen mit kristallinen und amorphen Anteilen. Vorzugsweise liegt die Verbindung der Formel (II) im osmotischen Freisetzungssystem in kristalliner Form vor. Vorzugsweise liegt die Verbindung der Formel (II) im osmotischen Freisetzungssystem in mikronisierter Form vor.

Ein Gegenstand der vorliegenden Erfindung ist eine feste, oral applizierbare, Natrium-(3S)-3-(4-chlor-3-{[(2S,3R)-2-(4-chlorphenyl)-4,4,4-trifluor-3-methylbutanoyl]amino}phenyl)-3-cyclopropylpropanoat der Formel (II), 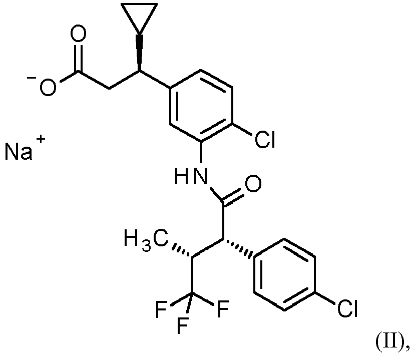 enthaltende pharmazeutische Darreichungsform mit modifizierter Freisetzung, dadurch gekennzeichnet, dass die pharmazeutische Darreichungsform auf einem osmotischen Freisetzungssystem basiert.

Ein Gegenstand der vorliegenden Erfindung ist eine feste, oral applizierbare, die Verbindung der Formel (II) enthaltende pharmazeutische Darreichungsform mit modifizierter Freisetzung, dadurch gekennzeichnet, dass die pharmazeutische Darreichungsform auf einem osmotischen Einkammersystem basiert.

Ein Gegenstand der vorliegenden Erfindung ist eine feste, oral applizierbare, die Verbindung der Formel (II) enthaltende pharmazeutische Darreichungsform mit modifizierter Freisetzung, dadurch gekennzeichnet, dass die pharmazeutische Darreichungsform auf einem osmotischen Zweikammersystem basiert.

In einer Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II) und mindestens ein hydrophiles quellbares Polymer enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II) und mindestens ein hydrophiles quellbares Polymer, bevorzugt ausgewählt aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder bevorzugt ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II), mindestens einem hydrophilen quellbaren Polymer, bevorzugt ausgewählt aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder bevorzugt ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, gegebenenfalls mindestens einen pharmazeutisch üblichen Hilfsstoff und gegebenenfalls einen osmotisch aktiven Zusatz enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II), mindestens ein hydrophiles quellbares Polymer, ausgewählt aus einer Liste bestehend aus Polyethylenoxid, Xanthan und Vinylpyrrolidon-Vinylacetat-Copolymer oder ausgewählt aus einer Liste bestehend aus Polyethylenoxid und Xanthan, gegebenenfalls mindestens ein weiteres hydrophiles quellbares Polymer, gegebenenfalls mindestens einen pharmazeutisch üblichen Hilfsstoff und gegebenenfalls einen osmotisch aktiven Zusatz enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern die Verbindung der Formel (II), das hydrophile quellbare Polymer Polyethylenoxid, gegebenenfalls mindestens ein weiteres hydrophiles quellbares Polymer, gegebenenfalls mindestens einen pharmazeutisch üblichen Hilfsstoff und gegebenenfalls einen osmotisch aktiven Zusatz enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern 0,5 Gew.-% bis 50 Gew.-% der Verbindung der Formel (II), 40 Gew.-% bis 99,5 Gew.-% von mindestens einem hydrophilen quellbaren Polymer, das ausgewählt ist aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, besonders bevorzugt Polyethylenoxid, Xanthan und Vinylpyrrolidon-Vinylacetat-Copolymer, ganz besonders bevorzugt Polyethylenoxid, gegebenenfalls mindestens einen pharmazeutisch üblichen Hilfsstoff und gegebenenfalls einen osmotisch aktiven Zusatz enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern 1 Gew.-% bis 40 Gew.-% der Verbindung der Formel (II), 50 Gew.-% bis 99 Gew.-% von mindestens einem hydrophilen quellbaren Polymer, das ausgewählt ist aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, besonders bevorzugt Polyethylenoxid, Xanthan und Vinylpyrrolidon-Vinylacetat-Copolymer, ganz besonders bevorzugt Polyethylenoxid, gegebenenfalls mindestens einen pharmazeutisch üblichen Hilfsstoff und gegebenenfalls einen osmotisch aktiven Zusatz enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern 2 Gew.-% bis 20 Gew.-% der Verbindung der Formel (II), 60 Gew.-% bis 90 Gew.-% von mindestens einem hydrophilen quellbaren Polymer, das ausgewählt ist aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, besonders bevorzugt Polyethylenoxid Xanthan und Vinylpyrrolidon-Vinylacetat-Copolymer, ganz besonders bevorzugt Polyethylenoxid, gegebenenfalls mindestens einen pharmazeutisch üblichen Hilfsstoff und gegebenenfalls einen osmotisch aktiven Zusatz enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern 2 Gew.-% bis 10 Gew.-% der Verbindung der Formel (II), 70 Gew.-% bis 85 Gew.-% von mindestens einem hydrophilen quellbaren Polymer, das ausgewählt ist aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder bevorzugt ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, besonders bevorzugt Polyethylenoxid, Xanthan und Vinylpyrrolidon-Vinylacetat-Copolymer, ganz besonders bevorzugt Polyethylenoxid, gegebenenfalls mindestens einen pharmazeutisch üblichen Hilfsstoff und gegebenenfalls einen osmotisch aktiven Zusatz enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern
- 0,5 Gew.-% bis 50 Gew.-% der Verbindung der Formel (II),
- 10 Gew.-% bis 50 Gew.-% Xanthan,
- 5 Gew.-% bis 40 Gew.-% eines Vinylpyrrolidon-Vinylacetat-Copolymers,
gegebenenfalls mindestens ein weiteres hydrophiles quellbares Polymer, gegebenenfalls mindestens einen weiteren pharmazeutisch üblichen Hilfsstoff und gegebenenfalls einen osmotisch aktiven Zusatz enthält.

Die Gew.-%-Angaben beziehen sich jeweils auf die Gesamtmasse des Kerns.

Bevorzugt enthält das osmotische Einkammersystem als einen der wesentlichen Bestandteile des Kerns das hydrophile wasserquellbare Polymer Xanthan. Dabei handelt es sich um ein anionisches Heteropolysaccharid, das im Handel beispielsweise unter der Bezeichnung Rhodigel^{®} (hergestellt durch Rhodia) oder "Xanthan FN Lebensmittelqualität normal" (hergestellt durch Jungbunzlauer Ladenburg GmbH) erhältlich ist. Es liegt in einer Menge von 10 bis 50 Gew.-%, bevorzugt von 25 bis 40 Gew.-%, bezogen auf die Gesamtmasse der Kernbestandteile vor.

Ein weiterer wesentlicher Bestandteil des Kerns ist das Vinylpyrrolidon-Vinylacetat-Copolymer. Dieses Copolymer ist an sich bekannt und kann mit beliebigen Mischungsverhältnissen der Monomere hergestellt werden. Das bevorzugt verwendete kommerziell erhältliche Kollidon^{®} VA64 (hergestellt durch BASF) ist beispielsweise ein 60:40-Copolymerisat. Es weist im allgemeinen einen Gewichtsmittelwert des Molekulargewichts, bestimmt durch Lichtstreuungsmessungen, von etwa 45.000 bis etwa 70.000 auf. Die Menge des Vinylpyrrolidon-Vinylacetat-Copolymers im Kern beträgt 5 bis 40 Gew.-%, bevorzugt 15 bis 25 Gew.-%, bezogen auf die Gesamtmasse der Kernbestandteile.

Gegebenenfalls im Kern zusätzlich vorhandene hydrophile quellbare Polymere sind beispielsweise Hydroxypropylcellulose, Hydroxypropylmethylcellulose, Natriumcarboxymethylcellulose, Natriumcarboxymethylstärke, Polyacrylsäuren oder deren Salze.

Weiterer Gegenstand der vorliegenden Erfindung ist ein Verfahren zur Herstellung eines osmotischen Freisetzungssystems dadurch gekennzeichnet, dass die Komponenten des Kerns miteinander vermischt, granuliert und tablettiert werden, der so entstandene Kern mit einer Hülle beschichtet wird und die Hülle abschließend mit einer oder mehreren Öffnungen, geeignet zum Austritt der Verbindung der Formel (II), versehen wird.
vermischt werden, gegebenenfalls feucht oder trocken granuliert werden, anschließend tablettiert werden und der so entstandene Kern mit der Hülle beschichtet wird. Die Hülle wird auf der Wirkstoffseite mit einer oder mehreren Öffnungen versehen. Alternativ dazu kann die Einführung der einen oder mehreren Öffnungen in diesem Prozessschritt unterbleiben und zunächst ein Lack, beispielsweise ein Lichtschutz- und/oder Farblack, aufgetragen werden. In diesem Fall werden erst nach erfolgter Beschichtung mit einem oder mehreren weiteren Lacken beide Seiten der Tablette mit jeweils einer Öffnung versehen, die jeweils von außen bis zum inneren Kern reichen, d.h. Lack und Hülle durchqueren und geeignet sind zum Austritt der Verbindung der Formel (II).

In einer bevorzugten Ausführungsform der vorliegenden Erfindung werden bei der Herstellung des osmotischen Einkammersystems die Kernkomponenten einer Feuchtgranulation unterzogen, da dieser Verfahrensschritt eine bessere Benetzbarkeit der Bestandteile des Tablettenkerns bewirkt, wodurch die eintretende Gastrointestinalflüssigkeit den Kern besser durchdringt, was vielfach zu einer rascheren und vollständigeren Freisetzung des Wirkstoffs führt.

In einer weiteren Ausführungsforin besteht der Kern des osmotischen Freisetzungssystems aus zwei Schichten, einer Wirkstoffschicht und einer Osmoseschicht. Ein derartiges osmotisches Zweikammersystem ist beispielsweise in DE 3417113 C2, WO 2006/072367 oder WO 2010/060564, deren Offenbarung hiermit durch Bezugnahme eingeschlossen ist, ausführlich beschrieben.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht und die Wirkstoffschicht Polyethylenoxid mit einer Viskosität von 40 bis 100 mPa·s (gemessen in 5%iger wässriger Lösung, 25 °C) ist und das mindestens eine hydrophile quellbare Polymer der Osmoseschicht Polyethylenoxid mit einer Viskosität von 5000 bis 8000 mPa·s (gemessen in 1%iger wässriger Lösung, 25 °C) ist.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht und die Wirkstoffschicht 1 Gew.-% bis 50 Gew.-% der Verbindung der Formel (II), 20 Gew.-% bis 99 Gew.-% von mindestens einem hydrophilen quellbaren Polymer, das ausgewählt ist aus einer Liste bestehend aus Polyethylenoxid, Xanthan, Cellulosederivate, beispielsweise der Verbindung der Formel (II), 20 Gew.-% bis 99 Gew.-% von mindestens einem hydrophilen quellbaren Polymer, bevorzugt ausgewählt aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, besonders bevorzugt Polyethylenoxid, Xanthan und Vinylpyrrolidon-Vinylacetat-Copolymer, ganz besonders bevorzugt Polyethylenoxid, gegebenenfalls mindestens einen osmotisch aktiven Zusatz und gegebenenfalls mindestens einen pharmazeutisch üblichen Hilfsstoff enthält.

In einer weiteren Ausführungsform enthält die Wirkstoffschicht 1 Gew.-% bis 45 Gew.-%, bevorzugt 1 Gew.-% bis 30 Gew.-%, besonders bevorzugt 2 Gew.-% bis 20 Gew.-% der Verbindung der Formel (II), 30 Gew.-% bis 99 Gew.-%, bevorzugt 50 Gew.-% bis 99 Gew.-%, besonders bevorzugt 60 Gew.-% bis 98 Gew.-% von mindestens einem hydrophilen quellbaren Polymer, gegebenenfalls mindestens einen osmotisch aktiven Zusatz und gegebenenfalls mindestens einen pharmazeutisch üblichen Hilfsstoff.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht und die Wirkstoffschicht 1 Gew.-% bis 50 Gew.-% der Verbindung der Formel (II), 20 Gew.-% bis 99 Gew.-% Polyethylenoxid, bevorzugt Polyethylenoxid mit einer Viskosität von 40 bis 100 mPa·s (gemessen in 5%iger wässrige Lösung, 25 °C), gegebenenfalls mindestens ein weiteres hydrophiles quellbares Polymer, gegebenenfalls mindestens einen osmotisch aktiven Zusatz und gegebenenfalls mindestens einen pharmazeutisch üblichen Hilfsstoff enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht und die Wirkstoffschicht 1 Gew.-% bis 45 Gew.-% der Verbindung der Formel (II), 30 Gew.-% bis 99 Gew.-% Polyethylenoxid, bevorzugt Polyethylenoxid mit einer Viskosität von 40 bis 100 mPa·s (gemessen in 5%iger wässrige Lösung, 25 °C), gegebenenfalls mindestens ein weiteres hydrophiles quellbares Polymer, gegebenenfalls mindestens einen osmotisch aktiven Zusatz und gegebenenfalls mindestens einen pharmazeutisch üblichen Hilfsstoff enthält.

(II), 50 Gew.-% bis 99 Gew.-% Polyethylenoxid, bevorzugt Polyethylenoxid mit einer Viskosität von 40 bis 100 mPa·s (gemessen in 5%iger wässrige Lösung, 25 °C), gegebenenfalls mindestens ein weiteres hydrophiles quellbares Polymer, gegebenenfalls mindestens einen osmotisch aktiven Zusatz und gegebenenfalls mindestens einen pharmazeutisch üblichen Hilfsstoff enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht und die Wirkstoffschicht 2 Gew.-% bis 20 Gew.-% der Verbindung der Formel (II), 60 Gew.-% bis 98 Gew.-% Polyethylenoxid, bevorzugt Polyethylenoxid mit einer Viskosität von 40 bis 100 mPa·s (gemessen in 5%iger wässrige Lösung, 25 °C), gegebenenfalls mindestens ein weiteres hydrophiles quellbares Polymer, gegebenenfalls mindestens einen osmotisch aktiven Zusatz und gegebenenfalls mindestens einen pharmazeutisch üblichen Hilfsstoff enthält.

Die Gew.-%-Angaben beziehen sich jeweils auf die Gesamtmasse der Wirkstoffschicht.

Die Viskosität von Polyethylenoxid mit einer Viskosität von 40 bis 100 mPa·s (gemessen in 5%iger wässriger Lösung, 25 °C) wird vorzugsweise mit einem geeigneten Brookfield Viskosimeter und einer geeigneten Spindel bei einer geeigneten Drehzahl gemessen, insbesondere wird ein Brookfield Viskosimeter Modell RVT und einer Spindel Nr. 1 bei einer Drehzahl von 50 U/min oder mit einem vergleichbaren Modell unter den entsprechenden Bedingungen (Spindel, Drehzahl) verwendet.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer der zuvor beschriebenen Wirkstoffschichten und einer Osmoseschicht besteht, wobei die Osmoseschicht 40 bis 90 Gew.-%, bevorzugt 50 Gew.-% bis 80 Gew.-% von mindestens einem hydrophilen quellbaren Polymer, das ausgewählt ist aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, bevorzugt Polyethylenoxid, Xanthan und Vinylpyrrolidon-Vinylacetat-Copolymer, ganz besonders bevorzugt Polyethylenoxid, 10 Gew.-% bis 60 Gew.-%, bevorzugt 20 Gew.-% bis 50 Gew.-% von mindestens einem osmotisch aktiven Zusatz und gegebenenfalls mindestens einen pharmazeutisch üblichen Hilfsstoff enthält.

In der Osmoseschicht wird als hydrophiles quellbares Polymere bevorzugt Polyethylenoxid verwendet. Besonders bevorzugt wird Polyethylenoxid mit einer Viskosität von 5000 bis 8000 mPa·s (gemessen in 1%iger wässriger Lösung, 25 °C).

Die Viskosität von Polyethylenoxid mit einer Viskosität von 5000 bis 8000 mPa·s (gemessen in 1%iger wässriger Lösung, 25 °C) wird vorzugsweise gemessen mit einem geeigneten Brookfield Viskosimeter und einer geeigneten Spindel bei einer geeigneten Drehzahl, insbesondere mit einem Brookfield Viskosimeter Modell RVF und einer Spindel Nr. 2 bei einer Drehzahl von 2 U/min oder mit einem vergleichbaren Modell unter den entsprechenden Bedingungen (Spindel, Drehzahl).

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht, wobei die Wirkstoffschicht 0,5 Gew.-% bis 65 Gew.-% der Verbindung der Formel (II), 20 Gew.-% bis 99,5 Gew.-% von mindestens einem hydrophilen quellbaren Polymer, das ausgewählt ist aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, bevorzugt Polyethylenoxid, Xanthan und Vinylpyrrolidon-Vinylacetat-Copolymer, ganz besonders bevorzugt Polyethylenoxid, gegebenenfalls mindestens einen osmotisch aktiven Zusatz und gegebenenfalls mindestens einen pharmazeutisch üblichen Hilfsstoff enthält, und die Osmoseschicht 40 Gew.-% bis 90 Gew.-% von mindestens einem hydrophilen quellbaren Polymer, bevorzugt ausgewählt aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, bevorzugt Polyethylenoxid, Xanthan und Vinylpyrrolidon-Vinylacetat-Copolymer, ganz besonders bevorzugt Polyethylenoxid, 10 Gew.-% bis 60 Gew.-% von mindestens einem osmotisch aktiven Zusatz und gegebenenfalls mindestens einen pharmazeutisch üblichen Hilfsstoff enthält.
einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren, bevorzugt Polyethylenoxid, Xanthan und Vinylpyrrolidon-Vinylacetat-Copolymer, ganz besonders bevorzugt Polyethylenoxid, 10 Gew.-% bis 60 Gew.-% von mindestens einem osmotisch aktiven Zusatz und gegebenenfalls mindestens einen pharmazeutisch üblichen Hilfsstoff enthält.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht, wobei die Wirkstoffschicht 1 Gew.-% bis 50 Gew.- % der Verbindung der Formel (II), 20 Gew.-% bis 99 Gew.-% Polyethylenoxid, bevorzugt Polyethylenoxid mit einer Viskosität von 40 bis 100 mPa·s (gemessen in 5%iger wässriger Lösung, 25 °C), gegebenenfalls mindestens einen osmotisch aktiven Zusatz und gegebenenfalls mindestens einen pharmazeutisch üblichen Hilfsstoff enthält, und die Osmoseschicht 40 Gew.-% bis 90 Gew.-% Polyethylenoxid, bevorzugt Polyethylenoxid mit einer Viskosität von 5000 bis 8000 mPa·s (gemessen in 1%iger wässriger Lösung, 25 °C), 10 Gew.-% bis 60 Gew.-% eines osmotischen aktiven Zusatzes und gegebenenfalls mindestens einen pharmazeutisch üblichen Hilfsstoff enthält.

In einer weiteren Ausführungsform enthält die Wirkstoffschicht 1 Gew.-% bis 45 Gew.-%, bevorzugt 1 Gew.-% bis 30 Gew.-%, besonders bevorzugt 2 Gew.-% bis 20 Gew.-% der Verbindung der Formel (II), 30 Gew.-% bis 99 Gew.-%, bevorzugt 50 Gew.-% bis 99 Gew.-%, besonders bevorzugt 60 Gew.-% bis 98 Gew.-% Polyethylenoxid mit einer Viskosität von 40 bis 100 mPa·s (gemessen in 5%iger wässriger Lösung, 25 °C), gegebenenfalls mindestens einen osmotisch aktiven Zusatz und gegebenenfalls mindestens einen pharmazeutisch üblichen Hilfsstoff, und die Osmoseschicht 40 Gew.-% bis 90 Gew.- %, bevorzugt 50 Gew.-% bis 80 Gew.-% Polyethylenoxid mit einer Viskosität von 5000 bis 8000 mPa·s (gemessen in 1%iger wässriger Lösung, 25 °C), 10 Gew.-% bis 60 Gew.-%, bevorzugt 20 Gew.-% bis 50 Gew.-% von mindestens einem osmotisch aktiven Zusatz und gegebenenfalls mindestens einen pharmazeutisch üblichen Hilfsstoff.

In einer weiteren Ausführungsform besteht das osmotische Freisetzungssystem aus einem Kern und einer Hülle, wobei die Hülle aus einem wasserdurchlässigen, für die Komponenten des Kerns undurchlässigen Material mit mindestens einer Öffnung besteht und wobei der Kern aus einer Wirkstoffschicht und einer Osmoseschicht besteht, wobei die Wirkstoffschicht 2 Gew.-% bis 20 Gew.- % der Verbindung der Formel (II), 60 Gew.-% bis 98 Gew.-% Polyethylenoxid mit einer Viskosität von 40 bis 100 mPa·s (gemessen in 5%iger wässriger Lösung, 25 °C), gegebenenfalls mindestens einen osmotisch aktiven Zusatz und gegebenenfalls mindestens einen pharmazeutisch üblichen Hilfsstoff enthält, und die Osmoseschicht 50 Gew.-% bis 80 Gew.-% Polyethylenoxid mit einer Viskosität von In einer weiteren Ausführungsform ist das osmotische Freisetzungssystem eines der zuvor beschriebenen osmotischen Freisetzungssysteme, wobei 80 % der Verbindung der Formel (II) nach 2 Stunden bis 24 Stunden, bevorzugt 4 Stunden bis 20 Stunden, besonders bevorzugt 5 Stunden bis 16 Stunden freigesetzt werden (gemessen gemäß USP-Freisetzungsmethode (USP 39; Kapitel <711> Dissolution) mit Apparatur 2 (Paddle) und der Angaben im Kapitel "Freisetzungsverhalten").

In einer weiteren Ausführungsform ist das osmotische Freisetzungssystem eines der zuvor beschriebenen osmotischen Freisetzungssysteme, wobei diese den Anforderungen des Arzneibuchs, bezüglich der Gehaltseinheitlichkeit (Ph. Eur. Ausgabewerk 9; 2.9.40 "Uniformity of Dosage Units") entsprechen.

In einer weiteren Ausführungsform ist das osmotische Freisetzungssystem eines der zuvor beschriebenen osmotischen Freisetzungssysteme, wobei die prozentuale Standardabweichung des Gehalts der Verbindung der Formel (II) innerhlab der osmotishcen Freisetzungssysteme unterhalb von 7 %, bevorzugt unterhalb von 6 %, besonders bevorzugt unterhalb von 5 %, ganz besonders bevorzugt unterhalb von 4 %, bei n=10 Einzeldosen, liegt.

Im Rahmen der vorliegenden Erfindung sind hydrophile quellbare Polymere alle dem Fachmann bekannten pharmazeutisch verwendbaren Polymerverbindungen, die durch Aufnahme von Wasser aufquellen. Es wird mindestens ein hydrophiles quellbares Polymer ausgewählt aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren oder ausgewählt aus einer Liste bestehend aus Polyethylenoxid, Xanthan, Cellulosederivate, beispielsweise Hydroxypropylcellulose, Hydroxypropylmethylcellulose oder Natriumcarboxymethylcellulose, Stärkederivate, beispielsweise Natriumcarboxymethylstärke, Vinylpyrrolidon-Vinylacetat-Copolymer, Polyvinylpyrrolidon und Methacrylsäure-Copolymere, beispielsweise Methacrylsäure-methylmethacrylat-Copolymer verwendet.

Weiterhin bevorzugt wird mindestens ein hydrophiles quellbares Polymer ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke und Vinylpyrrolidon-Vinylacetat-Copolymer verwendet, besonders bevorzugt werden Xanthan, Polyethylenoxid und Vinylpyrrolidon-Vinylacetat-Copolymer oder Mischungen davon verwendet.

Weiterhin bevorzugt wird mindestens ein hydrophiles quellbares Polymer ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke, Vinylpyrrolidon-Vinylacetat-Copolymer und Polyacrylsäuren oder ausgewählt aus einer Liste bestehend aus Polyethylenoxid, Xanthan, Hydroxypropylcellulose, Hydroxypropylmethylcellulose, Natriumcarboxymethylcellulose, Natriumcarboxymethylstärke und Vinylpyrrolidon-Vinylacetat-Copolymer verwendet, besonders bevorzugt werden Xanthan, Polyethylenoxid und Vinylpyrrolidon-Vinylacetat-Copolymer oder Mischungen davon verwendet.

Weiterhin bevorzugt wird mindestens ein hydrophiles quellbares Polymer ausgewählt aus einer Liste bestehend aus Polyethylenoxid, Xanthan, Kollidon VA 64, PVP 25, Eudragit L100, Eudragit RL PO, HPC LM und Polyacrylsäure oder ausgewählt aus einer Liste bestehend aus Polyethylenoxid, Xanthan, Kollidon VA 64, PVP 25, Eudragit L100, Eudragit RL PO und HPC LM verwendet, besonders bevorzugt wird Polyethylenoxid verwendet.

Weiterhin besonders bevorzugt wird mindestens ein hydrophiles quellbares Polymer ausgewählt aus einer Liste bestehend aus Polyethylenoxid (entsprechend der Ph. Eur. (Ausgabewerk 9) Monographie "Macrogols, High Molecular Mass"; Viskosität 5000 bis 8000mPa·s; gemessen in 1%iger wässriger Lösung, 25 °C; POLYOX^{™} Water-Soluble Resin NF WSR N-80; Dow) und Polyethylenoxid (entsprechend der Ph. Eur. (Ausgabewerk 9) Monographie "Macrogols, High Molecular Mass"; Viskosität von 40 bis 100 mPa·s; gemessen in 5%iger wässriger Lösung, 25 °C; POLYOX^{™} Water-Soluble Resin NF WSR N-80; Dow) verwendet.

Im Rahmen der vorliegenden Erfindung sind als hydrophile quellbare Polymere geeignete Stärkederivate Mais-, Weizen-, Reis- und Kartoffelstärke, substituierte Stärken wie Carboxymethylstärke und dessen Salz, Hydroxyethylstärke oder Mischungen davon.

Im Rahmen der vorliegenden Erfindung sind als hydrophile quellbare Polymere geeignete Cellulosederivate Methylcellulose (MC), Hydroxymethylpropylcellulose (HPMC), Hydroxypropylcellulose (HPC), Carboxymethylcellulose-Natrium (Na-CMC), Hydroxyethylcellulose (HEC) oder Mischungen davon.

Die genannten hydrophilen quellbaren Polymere können alleine oder in Kombination mit weiteren hydrophilen quellbaren Polymeren verwendet werden.

Einige hydrophile quellbare Polymere können alternativ als pharmazeutisch übliche Hilfsstoffe im Kern, beispielsweise als Binde- oder Sprengmittel, verwendet werden. Beträgt der Anteil eines solchen Stoffs im Kern 10 oder mehr Prozent bezogen auf die Masse des Kerns, gilt ein solcher Stoff im Rahmen der vorliegenden Erfindung als hydrophiles quellbares Polymer.

Osmotisch aktive Zusätze im Rahmen der vorliegenden Erfindung sind beispielsweise alle wasserlöslichen Stoffe, deren Verwendung in der Pharmazie unbedenklich ist, wie z.B. die in Pharmakopöen, in "Hager" und "Remington Pharmaceutical Science" oder weiterer Literatur (Sareen. R., Jain, N., Kumar, D., Current Drug Delivery, 9, (2012), 285-296) erwähnten wasserlöslichen Hilfsstoffe. Insbesondere können wasserlösliche Salze von anorganischen oder organischen Säuren oder nichtionische organische Stoffe mit großer Wasserlöslichkeit wie z.B. Kohlehydrate, insbesondere Zucker, Zuckeralkohole oder Aminosäuren verwendet werden. Zum Beispiel können die osmotisch aktiven Zusätze ausgewählt werden aus anorganischen Salzen wie Chloriden, Sulfaten, Carbonaten und Bicarbonaten von Alkali- oder Erdalkalimetallen, wie Lithium, Natrium, Kalium, Magnesium, Calcium sowie Phosphate, Hydrogen- oder Dihydrogenphosphate, Acetate, Succinate, Benzoate, Citrate oder Ascorbate davon. Des weiteren können Pentosen, wie Arabinose, Ribose oder Xylose, Hexosen, wie Glucose, Fructose, Galactose oder Mannose, Disaccharide wie Sucrose, Maltose oder Lactose oder Trisaccharide wie Raffinose verwendet werden. Zu den wasserlöslichen Aminosäuren zählen Glycin, Leucin, Alanin oder Methionin. Bevorzugt wird Natriumchlorid verwendet.

Pharmazeutisch übliche Hilfsstoffe im Rahmen der vorliegenden Erfindung sind beispielsweise Pufferstoffe wie Natriumbicarbonat, Bindemittel wie Hydroxypropylcellulose, Hydroxypropylmethylcellulose, Polyvinylpyrrolidon oder Vinylpyrrolidon-Vinylacetat-Copolymers (Kollidon^{®} VA64) Sprengmittel wie Natriumcarboxymethylstärke, Schmiermittel wie Magnesiumstearat, Netzmittel wie Natriumlaurylsulfat, Fließregulierungsmittel wie hochdisperses Siliziumdioxid, Schutzkolloide, wie in EP-B-0277092 (S.5, Z. 10-25) beschrieben, Weichmacher, wie z.B. in EP-B-0277092 (S. 5, Z. 29-32) beschrieben, Tensiden, wie z.B. in EP-B-0277092 (S. 5, Z. 33-44) beschrieben, Trägermaterialien, wie z.B. in EP-B-0277092 (S. 5, Z. 45-47) beschrieben sowie ein oder mehere Farbpigmente wie beispielsweise Eisenoxid in einer der beiden Schichten zur Differenzierung von Wirkstoff- und Osmoseschicht. Geeignete Schutzkolloide sind beispielsweise methylierte Cellulosederivate, z.B. Methylcellulose mit einem Methoxygehalt von ca. 27,0 bis 32,0 % und einem Substitutionsgrad von ca. 1,75 bis 2,1 oder Methylhydroxypropylcellulose mit einem Gehalt von ca. 16,0 - 30,0 % Methoxy- und 4,0 - 32,0 % Hydroxypropoxy-Gruppen. Geeignete Weichmacher sind beispielsweise Glycerin, Triethylcitrat, Diethylphthalat oder Diethylsebacat. Geeignete Tenside sind beispielsweise z.B. anionischen Tenside vom Typ Alkylsulfat, beispielsweise Natrium-, Kalium- oder Magnesium-n-dodecylsulfat, -n-tetradecyl-sulfat, -n-hexadecyl-sulfat oder -n-octa-decyl-sulfat, Alkylethersulfat, beispielsweise Natrium-, Kalium- oder Magnesium-n-dodecyloxyethylsulfat, -n-tetradecyloxyethylsulfat, -n-hexadecyl-oxyethylsulfat oder -n-octadecyloxyethylsulfat oder Alkansulfonat, beispielsweise Natrium-, Kalium- oder Magnesium-n-dodecansulonat, -n-tetradecansulfonat, -n-hexadecansulfonat oder -n-octadecansulfonat. Geeignete Tenside sind ferner nichtionische Tenside vom Typ Fettsaure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearatoder -trioleat, Polyethylenglycol-Fettsaureester wie Polyoxyethylstearat, Polyethylenglycol-400-stearat, Polyethylenglycol-2000-stearat, insbesondere Ethylenoxid-Propylenoxid Blockpolymere von Typ Pluronics^{®} (BWC) oder Synperonic^{®} (ICI). Geeignete Trägermaterialien sind beispielsweise Lactose, Saccharose, Sorbit, Mannit, Stärke, beispielsweise Kartoffelstärke, Maisstärke oder Amylopektin, oder Cellulose.

Die Hülle des osmotischen Wirkstofffreisetzungssystems besteht sowohl beim Einkammer- als auch beim Zweikammersystem aus einem wasserdurchlässigen, für die Komponenten des Kerns undurchlässigen filmbildenden Material. Solche Hüllmaterialien sind im Prinzip bekannt und beispielsweise in EP1024793 beschrieben. Als Hüllmaterialien können beispielsweise acylierte Cellulosederivate verwendet werden.

Acylierte Cellulosederivate (Celluloseester) sind durch Acetylgruppen ein- bis dreifach oder durch Acetylgruppen ein- bis zweifach und einen weiteren von Acetyl verschiedenen Acylrest substituierte Cellulosen, z.B. Celluloseacetat, Cellulosetriacetat, Celluloseacetatethylcarbamat, Celluloseacetatphthalat, Celluloseacetatmethylcarbamat, Celluloseacetatsuccinat, Celluloseacetatdimethylaminoacetat, Celluloseacetatethylcarbonat, Celluloseacetchloracetat, Celluloseacetatethyloxalat, Celluloseacetatmethylsulfonat, Cellulose-acetatbutylsulfonat, Celluloseacetatpropionat, Celluloseacetatdiethylaminoacetat, Celluloseacetatoctat, Celluloseacetatlaurat, Celluloseacetat-p-toluolsulfonat, Celluloseacetatbutyrat, und Hüllmaterialien aus der Gruppe der Celluloseether wie z.B. Ethylcellulose oder andere Celluloseacetatderivate sowie Agaracetat und Amyloseacetat.

Als Material für die Hülle eignen sich auch Ethylcellulose und polymere Epoxide, Copolymere aus Alkylenoxid und Alkylglycidylethern, Polyglykole und Polymilchsäurederivate und weitere Derivate davon. Ferner können auch Mischungen von an sich wasserunlöslichen Acrylaten (z.B. ein Copolymerisat von Acrylsäureethylester und Methacrylsäuremethylester) verwendet werden.

Im Rahmen der vorliegenden Erfindung werden als Hüllmaterial bevorzugt Celluloseacetat oder Gemische von Celluloseacetat und Polyethylenglycol eingesetzt.

Die Mengen und die verwendeten Bestandteile für die Herstellung der Hülle des osmotischen Arzneimittelfreisetzungssystems beeinflussen in bekannter Weise die Eintrittsgeschwindigkeit der gastrointestinalen Flüssigkeit. Grundsätzlich nimmt die Eintrittsgeschwindigkeit der gastrointestinalen Flüssigkeit mit zunehmender Hüllmaterialmenge ab.

Auf die Hülle kann bei Bedarf ein Lack, beispielsweise ein Lichtschutz- und/oder Farblack aufgebracht werden. Besonders geeignete Materialien sind beispielsweise Polymere wie Polyvinylalkohol, Hydroxypropylcellulose und/oder Hydroxypropylmethylcellulose, gegebenenfalls in Kombination mit geeigneten Weichmachern wie beispielsweise Polyethylenglycol oder Polypropylenglycol und Pigmenten wie beispielsweise Titandioxid oder Eisenoxide. Beispielhaft genannt sei die Lackierung mit einem Filmüberzug, für den zunächst Polyvinylalkohol und Polyethylenglykol 3350 in Wasser bei Raumtemperatur gelöst und unter Rühren gemischt wird. Unter Rühren wird schrittweise Talkum, Titandioxid und Eisenoxid zugegeben. Lacksuspensionen können beispielsweise mit einer geeigneten Lackiereinheit, z.B. einem Glatt-Coater, auf die Tablettenkerne aufgetragen werden. Alternativ kann anstatt der Lackierung auch eine Dragierung erfolgen. Im Allgemeinen wird ein solcher Lack durch ein wässriges oder organisches Auftragsmedium aufgebracht. Im Rahmen der vorliegenden Erfindung bezeichnet der Begriff Lack zusätzlich auch Beschichtungen der Hülle, welche durch ein alternatives Verfahren, beispielsweise ein lösungsmittelfreies Verfahren, aufgetragen werden.

Als Lacke können ebenfalls sogenannte "Fertiglacke" verwendet werden. Diese enthalten bereits ein Mischung von Hilfsstoffen und werden in Wasser gelöst und aufgetragen. Beispielhaft ist Opadry II 85F230009 Orange (Colorcon PVA-basierter Fertiglack) genannt, welcher partiell hydrolysiertes Polyvinylalkohol, Talkum, Polyethylenglykol (PEG 3350), Titandioxid, rotes Eisenoxid, gelbes Eisenoxid und Polysorbat 80 (Tween 80) enthält.

Die Hülle des osmotischen Arzneimittelfreisetzungssystems der vorliegenden Erfindung weist mindestens eine Öffnung bzw. Passage auf, durch die der Wirkstoff zusammen mit den weiteren Kernbestandteilen allmählich austritt. Die Öffnung wird durch Laserbohren, mechanisches Bohren oder z.B. Stanzen in die Hülle eingebracht. Es können ein oder mehrere Öffnungen in der Hülle vorhanden sein. Die Größe der Öffnung (Durchmesser) beträgt bevorzugt 0,2 bis 1,6 mm, besonders bevorzugt 0,3 bis 1,2 mm. Die Beschaffenheit und die Herstellverfahren der Öffnung sind an sich bekannt und beispielsweise beschrieben in US 4063064, US 4088864, US 3916899 oder EP-B-0277092. Der optional vorhandene Lack kann ebenfalls ein oder mehrere Öffnung aufweisen.

Als osmotisch aktiver Zusatz wird in den beschriebenen Ausführungsformen bevorzugt mindestens ein wasserlösliches Salze von anorganischen oder organischen Säuren, besonders bevorzugt Natriumchlorid, eingesetzt.

Als pharmazeutisch übliche Hilfsstoffe werden in den beschriebenen Ausführungsformen bevorzugt Bindemittel, wie beispielsweise Hydroxypropylcellulose, Schmiermittel, wie beispielsweise Magnesiumstearat, Fließregulierungsmittel, wie beispielsweise hochdisperses Siliziumdioxid und Farbpigmente, wie beispielsweise Eisenoxid, verwendet.

Zur Herstellung des osmotischen Zweikammersystems können beispielsweise die Komponenten der Wirkstoffschicht gemischt und feucht oder trocken, bevorzugt trocken granuliert werden, die Komponenten der Osmoseschicht gemischt und granuliert werden und anschließend beide Granulate auf einer Zweischichttablettenpresse zu einer Zweischichttablette verpresst werden. Der so entstandene innere Kern wird dann mit einer Hülle beschichtet. Die Hülle wird auf der Wirkstoffseite mit einer oder mehreren Öffnungen versehen. Alternativ dazu kann die Einführung der einen oder mehreren Öffnungen in diesem Prozessschritt unterbleiben. In diesem Fall werden erst nach erfolgter Beschichtung mit einem oder mehreren Lacken beide Seiten der Tablette mit jeweils einer Öffnung versehen, die jeweils von außen bis zum inneren Kern reichen, d.h. Lack und Hülle durchqueren.

Bevorzugt werden bei der Herstellung des osmotischen Zweikammersystems sowohl die Komponenten der Wirkstoffschicht als auch die Komponenten der Osmoseschicht jeweils granuliert, insbesondere mit Hilfe der Walzengranulation.

Ein weiterer Gegenstand der vorliegenden Erfindung ist ein Verfahren zur Herstellung der genannten Osmotischen Freisetzungssysteme, dadurch gekennzeichnet, dass die Komponenten des Kerns miteinander vermischt, granuliert und tablettiert werden, der so entstandene Kern mit einer Hülle beschichtet wird und die Hülle abschließend mit einer oder mehreren Öffnungen, geeignet zum Austritt der Verbindung der Formel (II), versehen wird.

Ein weiterer Gegenstand der vorliegenden Erfindung ist ein Verfahren zur Herstellung der genannten Osmotischen Freisetzungssysteme, dadurch gekennzeichnet, dass die Komponenten der Wirkstoffschicht gemischt und granuliert werden und die Komponenten der Osmoseschicht gemischt und granuliert werden, anschließend auf einer Zweischichttablettenpresse beide Granulate zu einer Zweischichttablette verpresst werden, der so entstandene Kern dann mit der Hülle beschichtet wird und die Hülle auf der Wirkstoffseite mit einer oder mehreren Öffnungen versehen wird.

Erfindungsgemäß bevorzugt sind aufgrund der physikalisch-chemischen Eigenschaften des Wirkstoffs osmotische Zweikammersysteme (*push-pull-Systeme*), bei denen die Wirkstoff- und Osmoseschicht getrennt vorliegen, und beispielhaft und vorzugsweise als Zweischichttablette formuliert sind. Die Vorteile gegenüber osmotischen Einkammersystemen sind hierbei die über einen längeren Zeitraum gleichmäßigere Freisetzungsrate sowie die Möglichkeit, den systembedingt notwendigen Wirkstoffüberschuss zu verringern.

Ein weiterer Gegenstand der vorliegenden Erfindung ist die Verbindung Natrium-(3S)-3-(4-chlor-3-{[(2S,3R)-2-(4-chlorphenyl)-4,4,4-trifluor-3-methylbutanoyl]amino}phenyl)-3-cyclopropylpropanoat der Formel (II)

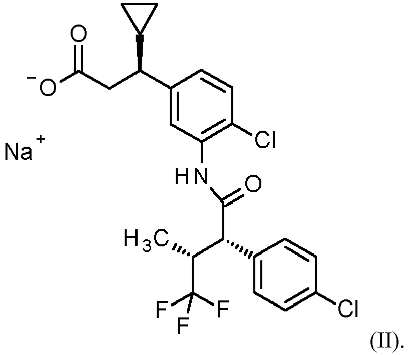

Ein weiterer Gegenstand ist die Verbindung der Formel (II) in kristalliner Form der Modifikation 1, dadurch gekennzeichnet, dass das Röntgendiffraktogramm der Verbindung Peakmaxima des 2 Theta Winkels bei 8,1; 22,3; 22,6° zeigt.

Ein weiterer Gegenstand ist die Verbindung der Formel (II) in kristalliner Form der Modifikation 1, dadurch gekennzeichnet, dass das Röntgendiffraktogramm der Verbindung Peakmaxima des 2 Theta Winkels bei 8,1; 17,2; 18,8; 22,3; 22,6° zeigt.

Ein weiterer Gegenstand ist die Verbindung der Formel (II) in kristalliner Form der Modifikation 1, dadurch gekennzeichnet, dass das Röntgendiffraktogramm der Verbindung Peakmaxima des 2 Theta Winkels bei 6,5; 8,1; 17,2; 18,8; 22,3; 22,6; 25,5° zeigt.

Ein weiterer Gegenstand ist die Verbindung der Formel (II) in kristalliner Form der Modifikation 1, dadurch gekennzeichnet, dass das Röntgendiffraktogramm der Verbindung Peakmaxima des 2 Theta Winkels bei 6,5; 8,1; 16,4; 17,2; 18,0; 18,8; 19,4; 22,3; 22,6 and 25,5° zeigt.

Ein weiterer Gegenstand ist die Verbindung der Formel (II) in kristalliner Form der Modifikation 1, dadurch gekennzeichnet, dass das IR-Spektrum der Verbindung Bandenmaxima bei 3381, 1691, 1565 cm⁻¹ zeigt.

Ein weiterer Gegenstand ist die Verbindung der Formel (II) in kristalliner Form der Modifikation 1, dadurch gekennzeichnet, dass das IR-Spektrum der Verbindung Bandenmaxima bei 3381, 1691, 1565, 1524, 1419 cm⁻¹ zeigt.

Ein weiterer Gegenstand ist die Verbindung der Formel (II) in kristalliner Form der Modifikation 1, dadurch gekennzeichnet, dass das IR-Spektrum der Verbindung Bandenmaxima bei 3381, 3066, 1691, 1565, 1524, 1419, 1101 cm⁻¹ zeigt.

Ein weiterer Gegenstand ist die Verbindung der Formel (II) in kristalliner Form der Modifikation 1, dadurch gekennzeichnet, dass das IR-Spektrum der Verbindung Bandenmaxima bei 3381, 3066, 2975, 1691, 1565, 1524, 1419, 1135, 1101, 817 cm⁻¹ zeigt.

Die Verbindung der Formel (II) in der kristallinen Modifikation 1 lässt sich aus der Verbindung der Formel (I) herstellen. Die Herstellung der Verbindung der Formel (I) in amorpher Form ist in WO 2012/139888 als Beispiel 22 offenbart. Die Herstellung der Verbindung der Formel (I) in kristalliner Form ist in EP17204842.3 (veröffentlicht als WO 2019/105881) offenbart. Sowohl die Verbindung der Formel (I) in amorpher Form als auch in kristalliner Form eignen sich in gleicher Weise zur Herstellung der Verbindung der Formel (II) in der kristallinen Modifikation 1 in den im Folgenden dargestellten Verfahren.

Bei der Herstellung der Verbindung der Formel (II) aus der Verbindung der Formel (I) besteht die Gefahr, dass die Verbindung der Formel (I) zu (3S)-3-(4-Chlor-3-{[(2R,3R)-2-(4-chlorphenyl)-4,4,4-trifluor-3-methylbutanoyl]amino}phenyl)-3-cyclopropylpropansäure der Formel (III), 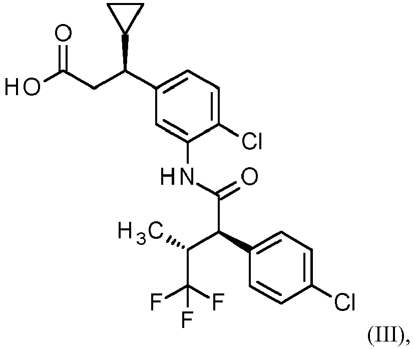 epimerisiert oder die Verbindung der Formel (II) zu Natrium-(3S)-3-(4-Chlor-3-{[(2R,3R)-2-(4-chlorphenyl)-4,4,4-trifluor-3-methylbutanoyl]amino}phenyl)-3-cyclopropylpropanoat der Formel (IV), 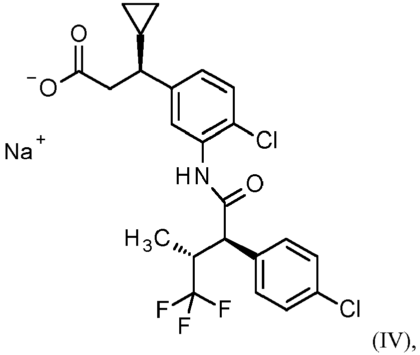 epimerisiert. So kommt es beispielsweise bei der Herstellung der Verbindung der Formel (II) mit Natronlauge oder bei der Verwendung von Methanol oder Ethanol als Lösungsmittel zu einem signifikanten Ausmaß zur Epimerisierung. In der Folge entstehen bei solchen Reaktionen relevante Mengen der Verbindung der Formel (III) und (IV), wodurch die Ausbeute der erwünschten Verbindung der Formel (II) verringert wird. Daher sollte eine Epimerisierung vermieden werden. Überraschenderweise zeigte sich bei den im Folgenden beschriebenen Herstellverfahren ein geringes Ausmaß dieser Nebenreaktion.

Zur Herstellung der Verbindung der Formel (II) in kristalliner Modifikation 1 wird die Verbindung der Formel (I), bevorzugt unter einer Schutzgasatmosphäre, beispielsweise unter eine Stickstoffatmosphäre, in einem polaren und aprotischen Lösungsmittel gelöst. Als polares und aprotisches Lösungsmittel kann beispielsweise Acetonitril, Toluol, Methyl-tert-butylether (MTBE) oder Tetrahydrofuran (THF) verwendet werden, bevorzugt wird Acetonitril verwendet. Anschließend wird Natriumhydroxid, bevorzugt in fester Form verwendet. Das Gemisch wird, bevorzugt über mehrere Stunden, gerührt. Nach Filtration wird der erhaltene Feststoff mit einem polaren und aprotischen Lösungsmittel gewaschen und getrocknet.

Insbesondere die Auswahl des Lösungsmittels, die verwendete Menge an Natriumhydroxid sowie die Verwendung von Natriumhydroxid in fester Form führen zu einer Reduktion der unerwünschten Epimerisierung der Verbindung der Formel (I) während der Synthese.

Insbesondere für die Herstellung größerer Mengen (Kilo-Maßstab) der Verbindung der Formel (II) besteht die Möglichkeit, dass ein Teil des festen Natriumhydoxids während der Reaktion nicht umgesetzt wird und nach der Filtration im erhaltenen Feststoff zurückbleibt. Daher wurde ein alternatives Herstellverfahren (Herstellverfahren 2) entwickelt.

In einer alternativen Herstellmethode wird die Verbindung der Formel (I), bevorzugt unter einer Schutzgasatmosphäre, beispielsweise unter einer Stickstoffatmosphäre, in einem polaren und aprotischen Lösungsmittel gelöst und vorzugsweise gefiltert. Als polares und aprotisches Lösungsmittel kann beispielsweise Acetonitril, Toluol, Methyl-tert-butylether (MTBE) oder Tetrahydrofuran (THF) verwendet werden, bevorzugt wird Acetonitril verwendet. Die Lösung wird abgekühlt und bevorzugt bei einer Tempertaur von -20 °C bis 50 °C, besonders bevorzugt -10 °C bis 10 °C, ganz besonders bevorzugt 0 °C mit einem sterisch anspruchsvollen Natriumalkoholat, wie beispielsweise Natrium-tert-butanolat oder Natrium-2-methyl-butan-2-olat, gelöst in einem geeigneten polaren und aprotischen Lösungsmittel, versetzt. Das sterisch anspruchsvolle Natriumalkoholat wird bevozugt in einer Menge von 0,7 bis 1,0 molaren Äquivalenten, besonders bevorzugt 0,9 bis 1,0 molaren Äquivalenten und ganz besonders bevorzugt 0,98 molaren Äquivalenten, bezogen auf die Verbindung der Formel (I) verwendet. Als polares und aprotisches Lösungsmittel kann beispielsweise Acetonitril, Toluol, Methyl-tert-butylether (MTBE), 2-Methyltetrahydrofuran oder Tetrahydrofuran (THF) verwendet werden, bevorzugt wird THF verwendet. Während der Hinzugabe des sterisch anspruchsvollen Natriumalkoholats können Impfkristalle der Verbindung der Formel (II) in der kristallinen Modifikation 1 hinzugegeben werden. Dies führt zu einer effizienteren Ausfällung und höheren Ausbeute. Diese Impfkristalle können beispielsweise durch das Herstellverfahren 1 hergestellt werden. Das Gemisch wird bei -20 °C bis 20 °C, bevorzugt -5 °C bis 5 °C, besonders bevorzugt 0 °C, bevorzugt über mehrere Stunden, gerührt. Nach Filtration wird der erhaltene Feststoff mit einem polaren und aprotischen Lösungsmittel gewaschen und getrocknet.

Überraschenderweise hat die Menge an Base in Bezug auf die verwendetete Menge der Verbindung der Formel (I) einen starken Einfluss auf das Ausmaß der Epimierisierung. 0,7 bis 1,0 molare Äquivalente, bevorzugt 0,9 bis 1,0 molare Äquivalente und besonders bevorzugt 0,98 molare Äquivalente an Base, bezogen auf die Verbindung der Formel (I), sind vorteilhaft.

Alternativ lässt sich die Verbindung der Formel (II) in der kristallinen Modifikation 1 durch Lösen der amorphen Form der Verbindung der Formel (II) oder einer weiteren Modifikation der Verbindung der Formel (II) in einem polarem Lösungsmittel, beispielsweise Tetrahydrofuran, Isopropanol oder Methanol, und nachfolgender Kristallisation herstellen.

Ein Gegenstand der vorliegenden Erfindung ist die Herstellung der Verbindung der Formel (II) in kristalliner Modifikation 1, dadurch gekennzeichnet, dass die Verbindung der Formel (I) in einem polaren und aprotischen Lösungsmittel gelöst wird, mit einer Base, ausgewählt aus einer Liste bestehend aus Natriumhydroxid oder einem sterisch anspruchsvollen Natriumalkoholat, versetzt wird und nach Rühren der ausgefällte Feststoff isoliert und getrocknet wird.

Ein Gegenstand der vorliegenden Erfindung ist die Herstellung der Verbindung der Formel (II) in kristalliner Modifikation 1, dadurch gekennzeichnet, dass die Verbindung der Formel (I), gelöst vorliegend in einem polaren und aprotischen Lösungsmittel, mit einer Base, ausgewählt aus einer Liste bestehend aus Natriumhydroxid oder einem sterisch anspruchsvollen Natriumalkoholat, versetzt wird und nach Rühren der ausgefällte Feststoff isoliert und getrocknet wird.

Ein Gegenstand der vorliegenden Erfindung ist die Herstellung der Verbindung der Formel (II) in kristalliner Modifikation 1, dadurch gekennzeichnet, dass die Verbindung der Formel (I), gelöst vorliegend in einem polaren und aprotischen Lösungsmittel, bevorzugt Acetonitril, Toluol, Methyl-tert-butylether (MTBE) oder Tetrahydrofuran (THF), besonders bevorzugt Acetonitril, mit einer Base, ausgewählt aus einer Liste bestehend aus Natriumhydroxid oder einem sterisch anspruchsvollen Natriumalkoholat, versetzt wird und nach Rühren der ausgefällte Feststoff isoliert und getrocknet wird.

Wird als Base Natriumhydroxid verwendet, wird bevorzugt Natriumhydroxid in fester Form verwendet.

Ein Gegenstand der vorliegenden Erfindung ist die Herstellung der Verbindung der Formel (II) in kristalliner Modifikation 1, dadurch gekennzeichnet, dass die Verbindung der Formel (I), gelöst vorliegend in Acetonitril, mit Natriumhydroxid in fester Form versetzt wird und nach Rühren der ausgefällte Feststoff isoliert und getrocknet wird.

Die Base wird bevozugt in einer Menge von 0,7 bis 1,0 molaren Äquivalenten, besonders bevorzugt 0,9 bis 1,0 molaren Äquivalenten und ganz besonders bevorzugt 0,98 molaren Äquivalenten, bezogen auf die Verbindung der Formel (I) verwendet.

Sterisch anspruchsvolle Natriumalkoholate umfassen alle dem Fachmann bekannten geeigneten Natriumalkoholate deren chemische Struktur komplexer als Natriummethanolat oder Natriumethanolat ist. Bevorzugte sterisch anspruchsvolle Natriumalkoholate, sind beispielsweise Natrium-tert-butanolat oder Natrium-2-methyl-butan-2-olat.

Ein Gegenstand der vorliegenden Erfindung ist die Herstellung der Verbindung der Formel (II) in kristalliner Modifikation 1, dadurch gekennzeichnet, dass das sterisch anspruchsvolle Natriumalkoholat Natrium-tert-butanolat oder Natrium-2-methyl-butan-2-olat ist.

Ein Gegenstand der vorliegenden Erfindung ist die Herstellung der Verbindung der Formel (II) in kristalliner Modifikation 1, dadurch gekennzeichnet, dass die Verbindung der Formel (I), gelöst vorliegend in einem polaren und aprotischen Lösungsmittel, bevorzugt Acetonitril, Toluol, Methyl-tert-butylether (MTBE) oder Tetrahydrofuran (THF), besonders bevorzugt Acetonitril, mit einer Base, ausgewählt aus einer Liste bestehend aus Natriumhydroxid, Natrium-tert-butanolat und Natrium-2-methyl-butan-2-olat versetzt wird und nach Rühren der ausgefällte Feststoff isoliert und getrocknet wird.

Ein Gegenstand der vorliegenden Erfindung ist die Herstellung der Verbindung der Formel (II) in kristalliner Modifikation 1, dadurch gekennzeichnet, dass die Verbindung der Formel (I), gelöst vorliegend in Acetonitril, mit Natrium-*tert*-butanolat versetzt wird und nach Rühren der ausgefällte Feststoff isoliert und getrocknet wird.

Ein Gegenstand der vorliegenden Erfindung ist die Herstellung der Verbindung der Formel (II) in kristalliner Modifikation 1, dadurch gekennzeichnet, dass die Verbindung der Formel (I), gelöst vorliegend in Acetonitril, mit Natrium-2-methyl-butan-2-olat versetzt wird und nach Rühren der ausgefällte Feststoff isoliert und getrocknet wird.

Die Base wird bevozugt in einer Menge von 0,7 bis 1,0 molaren Äquivalenten, besonders bevorzugt 0,9 bis 1,0 molaren Äquivalenten und ganz besonders bevorzugt 0,98 molaren Äquivalenten, bezogen auf die Verbindung der Formel (I) verwendet.

Ein Gegenstand der vorliegenden Erfindung ist die Herstellung der Verbindung der Formel (II) in kristalliner Modifikation 1, dadurch gekennzeichnet, dass die Verbindung der Formel (I), gelöst vorliegend in Acetonitril, mit Natrium-*tert*-butanolat in einer Menge von 0,7 bis 1,0 molaren Äquivalenten, besonders bevorzugt 0,9 bis 1,0 molaren Äquivalenten und ganz besonders bevorzugt 0,98 molaren Äquivalenten, bezogen auf die Verbindung der Formel (I) versetzt wird und nach Rühren der ausgefällte Feststoff isoliert und getrocknet wird.

Ein Gegenstand der vorliegenden Erfindung ist die Herstellung der Verbindung der Formel (II) in kristalliner Modifikation 1, dadurch gekennzeichnet, dass die Verbindung der Formel (I), gelöst vorliegend in Acetonitril, mit Natrium-2-methyl-butan-2-olat in einer Menge von 0,7 bis 1,0 molaren Äquivalenten, besonders bevorzugt 0,9 bis 1,0 molaren Äquivalenten und ganz besonders bevorzugt 0,98 molaren Äquivalenten, bezogen auf die Verbindung der Formel (I) versetzt wird und nach Rühren der ausgefällte Feststoff isoliert und getrocknet wird.

Das Versetzen mit der Base sowie das anschließende Rühren finden unabhängig voneinander bei einer Tempertaur von -20 °C bis 50 °C, bevorzugt -20 °C bis 20 °C, besonders bevorzugt -10 °C bis 10 °C, ganz besonders bevorzugt 0 °C statt.

Gegebenenfalls können während der Reaktion Impfkristalle der Verbindung der Formel (II) in der kristallinen Modifikation 1 hinzugegeben werden.

Die erfindungsgemäße Verbindung der Formel (II) und die erfindungsgemäßen Darreichungsformen besitzen wertvolle pharmakologische Eigenschaften und können zur Behandlung und/oder Prävention von Erkrankungen bei Menschen und Tieren verwendet werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die erfindungsgemäße Verbindung der Formel (II) und die erfindungsgemäßen Darreichungsformen führen zu einer Gefäßrelaxation, zu einer Thrombozytenaggregationshemmung, zu einer Blutdrucksenkung sowie zu einer Steigerung des koronaren Blutflusses und der Mikrozirkulation. Diese Wirkungen sind über eine direkte, Häm-unabhängige Aktivierung der löslichen Guanylatcyclase und einen Anstieg der intrazellulären cGMP-Spiegel vermittelt.

Die erfindungsgemäße Verbindung der Formel (II) und die erfindungsgemäßen Darreichungsformen eignen sich in besonderem Maße zur Behandlung und/oder Prävention von renalen und kardiorenalen Erkrankungen, insbesondere des chronischen Nierenversagens (CKD) und der diabetischen Nierenkrankheit (DKD), kardialen und kardiovaskulären Erkrankungen, insbesondere der Herzinsuffizienz (HFpEF und HFrEF), Herzinfarkt, Angina pectoris, Kardiomyopathien, Hypertonie und Arteriosklerose, pulmonaler und kardiopulmonaler Erkrankungen, insbesondere pulmonaler Hypertonie (PH), Erkrankungen des Zentralnervensystems, insbesondere der Demenz, Knochenerkrankungen, insbesondere Osteogenesis imperfecta, thromboembolischen Erkrankungen, Muskeldystrophien, Ischämien, Gefäßerkrankungen, Mikrozirkulationsstörungen, fibrotischen Erkrankungen insbesondere der Systemischen Sklerose, insbesondere der altersbedingten Makuladegeneration, inflammatorischen Erkrankungen, und Stoffwechselerkrankungen, insbesondere des Metabolischen Syndroms, der Dyslipidämie und Diabetes.

Die erfindungsgemäße Verbindung der Formel (II) und die erfindungsgemäßen Darreichungsformen können eingesetzt werden zur Behandlung und/oder Prävention von kardialen, kardiovaskulären und kardiopulmonalen Erkrankungen, beispielsweise Bluthochdruck (Hypertonie), Herzinsuffizienz, koronare Herzerkrankung, stabile und instabile Angina pectoris, pulmonale arterielle Hypertonie (PAH) und sekundäre Formen der pulmonalen Hypertonie (PH), chronisch thromboembolische pulmonale Hypertonie (CTEPH), renale Hypertonie, periphere und kardiale Gefäßerkrankungen, Arrhythmien, Rhythmusstörungen der Vorhöfe und der Kammern sowie Überleitungsstörungen, beispielsweise atrioventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade-de-pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock.

Die erfindungsgemäße Verbindung der Formel (II) und die erfindungsgemäßen Darreichungsformen können zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung, beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, perkutan-transluminalen Angioplastien (PTA), perkutan-transluminalen Koronarangioplastien (PTCA), Herztransplantationen, Bypass-Operationen und mikround makrovaskulären Schädigungen (Vaskulitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff "pulmonale Hypertonie" sowohl primäre als auch sekundäre Unterformen hiervon, wie sie nach der Dana-Point-Klassifikation gemäß ihrer jeweiligen Ätiologie definiert worden sind [siehe D. Montana und G. Simonneau, in: A.J. Peacock et al. (Hrsg.), Pulmonary Circulation. Diseases and their treatment, 3rd edition, Hodder Arnold Publ., 2011, S. 197-206; M.M. Hoeper et al., J. Am. Coll. Cardiol. 2009, 54 (1), S85-S96]. Hierzu gehört insbesondere in Gruppe 1 die pulmonal-arterielle Hypertonie (PAH), zu der unter anderem die idiopathischen und die familiären Formen zählen (IPAH bzw. FPAH),akute pulmonale Hypertonien, insbesondere das Akuten Atemnotsyndrom (*acute respiratory distress sydrom* - ARDS), der Akute Lungenschadens (*acute lung injury* - ALI) und das Atemnotsyndrom des Neugeborenen (*infant respiratory distress syndrom -* IRDS). Des Weiteren umfasst PAH auch die persistierende pulmonale Hypertonie bei Neugeborenen sowie die assoziierte pulmonal-arterielle Hypertonie (APAH), welche assoziiert ist mit Kollagenosen, kongenitalen systemisch-pulmonalen Shuntvitien, portaler Hypertension, HIV-Infektionen, der Einnahme bestimmter Drogen und Medikamente (beispielsweise von Appetitzüglern), mit Erkrankungen mit einer signifikanten venösen/kapillären Beteiligung wie der pulmonal-venookklusiven Erkrankung und der pulmonal-kapillären Hämangiomatose, oder mit anderen Erkrankungen wie Schilddrüsenerkrankungen, Glykogenspeicherkrankheiten, Morbus Gaucher, hereditärer Teleangiektasie, Hämoglobinopathien, myeloproliferativen Erkrankungen und Splenektomie. In Gruppe 2 der Dana-Point-Klassifikation werden PH-Patienten mit einer ursächlichen Linksherzerkrankung, wie ventrikulären, atrialen oder valvulären Erkrankungen, zusammengefasst. Gruppe 3 umfasst Formen der pulmonalen Hypertonie, die mit einer Lungenerkrankung, beispielsweise chronisch-obstruktiver Lungenerkrankung (COPD), interstitieller Lungenkrankheit (ILD), Lungenfibrose (IPF), und/oder einer Hypoxämie, Schlafapnoe, alveoläre Hypoventilation, chronische Höhenkrankheit, anlagebedingte Fehlbildungen assoziiert sind. Zur Gruppe 4 zählen PH-Patienten mit chronisch-thrombotischen und/oder embolischen Erkrankungen, beispielsweise bei thromboembolischer Obstruktion von proximalen und distalen Lungenarterien (CTEPH) oder bei nicht-thrombotischen Embolisierungen (z.B. infolge von Tumorerkrankungen, Parasiten, Fremdkörpern). Seltenere Formen der pulmonalen Hypertonie, wie bei Patienten mit Sarkoidose, Histiozytose X oder Lymphangiomatose, sind in der Gruppe 5 zusammengefasst.

Im Sinne der vorliegenden Erfindung umfasst der Begriff "Herzinsuffizienz" sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz, Herzinsuffizienz mit reduzierter Ejektionsfraktion (HFrEF), Herzinsuffizienz mit erhaltener Ejektionsfraktion (HFpEF).

Die erfindungsgemäße Verbindung der Formel (II) und die erfindungsgemäßen Darreichungsformen eignen sich auch zur Behandlung und/oder Prävention von Stoffwechselerkrankungen. Im Rahmen der vorliegenden Erfindung sind Stoffwechselerkrankungen beispielsweise Erkrankungen des Glukose-Stoffwechsels und Erkrankungen und Komplikationen die mit einem gestörtem Glukose-Stoffwechsel assoziiert sind. Erkrankungen des Glukose-Stoffwechsels sind beispielsweise Diabetes Mellitus (Typ 1 oder Typ 2), Insulinresistenz, gestörte Glukosetolerenz, Hyperglykämie, Hypoglykämie, Hyperinsulinämie oder Hypoinsulinämie. Erkrankungen die mit einem gestörtem Glukose-Stoffwechsel assoziiert sind, sind beispielsweise Mikro- und Makroangiopathien, diabetische Retinopathien, diabetische Neuropathien, diabetische Nephropathien, verzögerte/gestörte Wundheilung, diabetischer Fuß, Gewebeischämien, Geschwüre an den Extremitäten, Gangrän, metabolische Azidose, Ketose, Dyslipidämien, Myokardinfarkt, akutes Koronarsyndrom, stabile oder instabile Angina Pectoris, Kardiomyopathien, Herzinsuffizienz, Herzrhythmusstörungen, vaskuläre Restenose, periphere arterielle Verschlusskrankheit, Fettleibigkeit, Syndrom X, Fettstoffwechselstörungen, Arteriosklerose oder Bluthochdruck. Die erfindungsgemäßen Verbindung der Formel (II) und die erfindungsgemäßen Darreichungsformen eignen sich ebenfalls für die Erhaltung, Verbesserung und Wiederherstellung der Funktionen von Zellen des Pankreas, insbesondere für die Erhaltung, Verbesserung und Wiederherstellung der Anzahl und Größe der β-Zellen des Pankreas.

Im Rahmen der vorliegenden Erfindung sind Stoffwechselerkrankungen außerdem Erkrankungen des Fettstoffwechsels wie beispielsweise Lipidstoffwechselstörungen, Hypolipoproteinämien, Dyslipidämien, Hypertriglyceridämien, Hyperlipidämien, kombinierten Hyperlipidämien, Hypercholesterolämien, Abetalipoproteinämie, Sitosterolämie, Xanthomatose, Tangier-Krankheit, Fettsucht (Adipositas), Fettleibigkeit (Obesitas), Arteriosklerose sowie das Metabolische Syndrom. Die erfindungsgemäße Verbindung der Formel (II) und die erfindungsgemäßen Darreichungsformen eignen sich auch zur Behandlung und/oder Prävention von kardiovaskulären Erkrankungen, die mit einer Stoffwechselerkrankung assoziiert sind.

Die erfindungsgemäße Verbindung der Formel (II) und die erfindungsgemäßen Darreichungsformen eignen sich auch zur Behandlung und/oder Prävention von muskulären- oder neuromuskulären Erkrankungen. Der Ausdruck "muskuläre- oder neuromuskuläre Erkrankungen" bezieht sich auf einen medizinischen Zustand, der die Muskeln und/oder deren direkte Steuerung des Nervensystems betrifft. Sie können erworben werden oder genetischen Ursprungs sein. Insbesondere sind muskuläre oder neuromuskuläre Erkrankungen Duchenne-Muskeldystrophie (DMD), Becker-Muskeldystrophie (BMD), kongenitale Muskeldystrophie, Miyoshi-Myopathie, Emery-Dreifuss-Muskeldystrophie, Facioscapulohumerale Muskeldystrophie, Muskeldystrophie des Schenkelgürtels, Myotonic charakterisiert Muskeldystrophie, Okulopharyngeale Muskeldystrophie, Myasthenia gravis, Lambert-Eaton myasthenisches Syndrom und Charcot-Marie-Tooth-Krankheit.

Weiterhin können die erfindungsgemäße Verbindung der Formel (II) und die erfindungsgemäßen Darreichungsformen zur Behandlung und/oder Prävention von primärem und sekundärem Raynaud-Phänomen, Mikrozirkulationsstörungen, Claudicatio, Hörstörungen, Tinnitus, peripheren und autonomen Neuropathien, diabetischen Mikroangiopathien, diabetischer Retinopathie, CREST-Syndrom, Erythematose, Onychomykose sowie rheumatischen Erkrankungen verwendet werden.

Die erfindungsgemäße Verbindung der Formel (II) und die erfindungsgemäßen Darreichungsformen können darüber hinaus zur Behandlung und/oder Prävention von ischämie- und/oder reperfusionsbedingten Schädigungen von Organen oder Geweben sowie als Zusatzstoffe für Perfusionsund Konservierungslösungen von Organen, Organteilen, Geweben oder Gewebeteilen menschlichen oder tierischen Ursprungs, insbesondere bei chirurgischen Eingriffen oder im Bereich der Transplantationsmedizin, Verwendung finden.

Die erfindungsgemäße Verbindung der Formel (II) und die erfindungsgemäßen Darreichungsformen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen (chronic kidney disease; CKD) hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, diabetische Nierenkrankheit (DKD), Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen, beispielsweise Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Darreichungsformen zur Behandlung und/oder Prävention von Folgeerscheinungen einer Niereninsuffizienz, beispielsweise Hypertonie, Lungenödem, Herzinsuffizienz, Urämie, Anämie, Elektrolytstörungen (beispielsweise Hyperkalämie, Hyponaträmie) und Störungen im Knochen- und Kohlenhydrat-Metabolismus.

Darüber hinaus sind die erfindungsgemäße Verbindung der Formel (II) und die erfindungsgemäßen Darreichungsformen zur Behandlung und/oder Prävention von Erkrankungen des Urogenitalsystems geeignet, beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), interstitielle Zystitis, neurogene überaktive Blase (OAB), Inkontinenz, beispielsweise Misch-, Drang-, Stress- oder Überlauf-Inkontinenz (MUI, UUI, SUI, OUI), Beckenschmerzen sowie erektile Dysfunktion, weibliche sexuelle Dysfunktion, vaginale Atrophy, Dyspareunie oder atrophische Vaginitis.

Die erfindungsgemäße Verbindung der Formel (II) und die erfindungsgemäßen Darreichungsformen eignen sich auch zur Behandlung und/oder Prävention von asthmatischen Erkrankungen, chronisch-obstruktiven Lungenerkrankungen (COPD), des akuten Atemwegssyndroms (ARDS) und der akuten Lungenschädigung (ALI), alpha-1-Antitrypsin-Defizienz (AATD), Lungenfibrose, Lungenemphysem (beispielsweise durch Zigarettenrauch induziertes Lungenemphysem), pulmonalvenöse Hypertonie, interstitielle Lungenerkrankung, Schlafapnoe, alveoläre Hypoventilationsstörungen, chronische Exposition gegenüber Höhenlagen, neonatale Lungenerkrankung, alveolar-kapillare Dysplasie, Sichelzellenanämie, Koagulationsstörungen, chronische Thromboembolie, Tumor-assoziierte Lungenembolie, Bindegewebserkrankungen, Lupus, Schistosomiasis, Sarkoidose, chronische Bronchitis, kapilläre pulmonale Hämangiomatose; Histiozytose X, Lymphangiomatose und komprimierte Lungengefäße aufgrund von Adenopathie, fibrotisierender Mediastinitis und zystischer Fibrose (CF).

Die in der vorliegenden Erfindung beschriebene erfindungsgemäße Verbindung der Formel (II) und die erfindungsgemäßen Darreichungsformen stellen auch Wirkstoffe und Darreichungsformen zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierte Lern- und Gedächtnisstörungen, altersassoziierte Gedächtnisverluste, Demenz, vaskuläre Demenz, Mischformen der Demenz, Demenz, die nach Schlaganfällen auftritt ("post stroke dementia"), post-traumatisches Schädel-Hirn-Trauma, allgemeine Konzentrationsstörungen, Konzentrationsstörungen bei Kindern mit Lern- und Gedächtnisproblemen, Alzheimer'sche Demenz, Demenz mit Lewy-Körperchen, Demenz mit Degeneration der Frontallappen einschließlich des Pick's-Syndroms, Parkinson'sche Krankheit, progressiver nuclear palsy, Demenz mit corticobasaler Degeneration, Amyolateralsklerose (ALS), Huntington'sche Krankheit, Demyelinisation, Multiple Sklerose, thalamische Degeneration, Creutzfeld-Jacob-Demenz, HIV-Demenz, Schizophrenie mit Demenz oder Korsakoff-Psychose, Binswanger-Demenz (subkortikale arteriosklerotische Enzephalopathie), zerebrale autosomal-dominante Arteriopathie mit subkortikalen Infarkten und Leukenzephalopathie (iCADASIL oder CADASIL-Syndrom), asymptomatische neurokognitive Störung (ANI), Multiple Sklerose (MS) (einschließlich des klinisch isolierten Syndroms (CIS), schubförmige MS (RRMS), primär progrediente MS (PPMS) und sekundär progrediente MS (SPMS), Multisystematrophie (MSA), Parkinson-Krankheit, Parkinsonismus Plus, progressive supranukleäre Blickparese (PSP, Steele-Richardson-Olszewski-Syndrom), Aufmerksamkeitsdefizitsyndrom (ADS) und Aufmerksamkeitsdefizit-Hyperaktivitätsstörung (ADHS). Sie eignen sich auch zur Behandlung und/oder Prävention von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme. Sie eignen sich auch zur Behandlung und/oder Prävention von Verletzungen, beispielsweise Schädel-Hirn-Traumen (TBI), einschließlich z. B. Gehirnerschütterungen und traumatische Enzephalopathien (CTE), oder nichttraumatische Schlaganfällen (einschließlich ischämischen Schlaganfällen, Aneurysmen oder Hypoxien), Schädigungen des Gehirns, kognitiven Beeinträchtigungen, Hirnverletzungen, neurodegenerativen Erkrankungen oder neuropathischen Schmerzen. Sie eignen sich auch zur Behandlung und/oder Prävention von Dystonien, beispielsweise generalisierte, fokale, segmentale, vegetative, akute dystonische Reaktionen und genetische/primäre Dystonien und Dyskinesien, einschließlich akute, chronische/tardive und nicht-motorische und levo-Dopa-induzierte Dyskinesien (LID). Sie eignen sich auch zur Behandlung und/oder Prävention von Erkrankungen, die sich durch eine Verringerung der synaptischen Plastizität und der synaptischen Prozesse auszeichnen, beispielsweise Fragil-X-Syndrom, Rett-Syndrom, Williams Syndrom, Renpenning-Syndrom, Autismus-Spektrum-Störungen einschließlich Autismus, Asperger-Syndrom oder tiefgreifende Entwicklungsstörungen. Sie eignen sich auch zur Behandlung und/oder Prävention von mentalen, affektiven oder psychischen Störung, beispielsweise bipolare Störung, Schizophrenie, allgemeine Psychose, Drogen-induzierte Psychose, Wahnvorstellung, schizoaffektive Störung, Zwangsstörung (OCD), depressiver Störungen , Angststörungen, Panikstörungen oder posttraumatische Belastungsstörung (PTSD).

Weiterhin eignen sich die erfindungsgemäße Verbindung der Formel (II) und die erfindungsgemäßen Darreichungsformen auch zur Regulation der cerebralen Durchblutung und stellen somit wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Darreichungsformen zur Bekämpfung von Schmerzzuständen eingesetzt werden.

Zudem besitzen die erfindungsgemäße Verbindung der Formel (II) und die erfindungsgemäßen Darreichungsformen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, UC), Pankreatitis, Peritonitis, rheumatoiden Erkrankungen und entzündlichen Hauterkrankungen eingesetzt werden.

Die erfindungsgemäße Verbindung der Formel (II) und die erfindungsgemäßen Darreichungsformen sind ferner geeignet zur Behandlung und/oder Prävention von akutem Schmerz, zentralem Schmerzsyndrom, Chemotherapie-induzierter Neuropathie und neuropathischen Schmerzen, diabetischer Neuropathie, Fibromyalgie, inflammatorischen Schmerzen, neuropaischen Schmerzen, postoperativen Schmerzen, tonischen Schmerzen oder viszeralen Schmerzen.

Die erfindungsgemäße Verbindung der Formel (II) und die erfindungsgemäßen Darreichungsformen sind ferner zur Behandlung und/oder Prävention von fibrotischen Erkrankungen der inneren Organe, wie beispielsweise der Lunge, des Herzens, der Nieren, des Knochenmarks und insbesondere der Leber, sowie von dermatologischen Fibrosen und fibrotischen Erkrankungen des Auges geeignet. Im Sinne der vorliegenden Erfindung umfasst der Begriff "fibrotische Erkrankungen" insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, systemische Sklerose, Morphaea, Keloide, hypertrophe Narbenbildung, Naevi, diabetische Retinopathie, proliferative Vitroretinopathie und Erkrankungen des Bindegewebes (beispielsweise Sarkoidose). Die erfindungsgemäßen Darreichungsformen können ebenso verwendet werden zur Behandlung der Steatohepatitis, insbesondere der Nichtalkoholischen Steatohepatitis (NASH), zur Förderung der Wundheilung, zur Bekämpfung postoperativer Narbenbildung, beispielsweise nach Glaukom-Operationen, und zu kosmetischen Zwecken bei alternder und verhornter Haut.

Des Weiteren eignen sich die erfindungsgemäße Verbindung der Formel (II) und die erfindungsgemäßen Darreichungsformen zur Behandlung und/oder Prävention von Knochenerkrankungen wie beispielsweise und bevorzugt Osteogenesis Imperfecta (OI), Knochenfrakturen, Knochenheilungsstörungen, Rachitis, Osteomalazie, avaskuläre Knochennekrose, Paget-Krankheit, Osteodystrophie, Osteopenie, osteolytische Läsionen durch Knochenmetastasen, Strahlentherapie oder Chemotherapie, Parodontitis, Hyperkalzämie, Osteonekrose, Osteosarkom, osteolytische Metastasen, familiäre expansive Osteolyse, expansiles skelettales und idiopathisches Hyperplasie, Hyperostosis corticalis deformans juvenilis, Camurati-Engelmann-Krankheit, Prothesenlockerung, periprostetische Osteolyse, kleidokraniale Dysplasie (CCD), multiples Myelom, alveolärer Knochenverlust, durch Immobilisierung oder Sexualhormonmangel bedingter Knochenschwund, Knochenschwund assoziiert mit einer aus der Gruppe ausgewählten Krankheit bestehend aus Kachexie, Anorexie, Alopezie und entzündlichen Erkrankungen, ausgewählt aus der Gruppe bestehend aus rheumatoider Arthritis, psoriatischer Arthritis, Psoriasis, Spondyloarthritis, SLE, systemischer Sklerose, metastasierendem Krebs und entzündlicher Darmerkrankung, Osteoarthritis, beeinträchtigter Knochenheilung nach Osteotomie, idiopathischer Knochenschwund in der Kindheit, Krümmung der Wirbelsäule, Osteoporose, primäre Osteoporose, sekundäre Osteoporose und insbesondere Osteoporose, primäre Osteoporose oder sekundäre Osteoporose, die nicht durch einen Mangel an Sexualhormonen verursacht wird.

Des Weiteren eignen sich die erfindungsgemäße Verbindung der Formel (II) und die erfindungsgemäßen Darreichungsformen zur Behandlung und/oder Prävention von Dysfunktionen gastrointestinaler Sphinkter, wie Achalasie, Sphinkterspasmen und hypertensive Sphinkter, insbesondere untere Ösophagussphinkter (LES)-Achalasie, Ösophagus-Achalasie, spastischer LES, hypertensiver LES (HTNLES), Pylorussphinkter (Pylorus)-Achalasie, Pylorusspasmus (Pylorospasmus), hypertensiver Pylorus, Ileozökalsphincter- oder Valve (ICV)-Achalasie, hypertensiver ICV, spastischer ICV- oder ICV-Spasmus, Sphinkter von Oddi-Dysfunktion (SOD), Sphinkter von Oddi-Achalasie, spastischer Sphinkter von Oddi, hypertensiver Sphinkter von Oddi, interner Analsphinkter (IAS)-Achalasie, hypertensiver IAS, spastischer IAS oder lAS-Krampf. In einer weiteren Ausführungsform sind die genannten Dysfunktionen gastrointestinaler Sphinkter auf eine eine neurologische, metabolische, endokrine oder neurodegenerative Erkrankung zurückzuführen.

Des Weiteren eignet sich die erfindungsgemäße Verbindung der Formel (II) und die erfindungsgemäßen Darreichungsformen zur Behandlung und/oder Prävention von ophthalmologischen Erkrankungen, worunter im Sinne der Erfindung beispielsweise die folgenden Erkrankungen zu verstehen sind: altersbedingte Makuladegeneration (AMD) einschließlich trockener (nicht-exsudativer) und feuchter (exsudativer, neovaskulärer) AMD, choroidale Neovaskularisierung (CNV), choroidale neovaskuläre Membranen (CNVM), zystoides Makula-Ödem (CME), epiretinale Membranen (ERM) und Makula-Perforationen, Myopie-assoziierte choroidale Neovaskularisierung, angioide und vaskuläre Streifen, Retina-Ablösung, diabetische Retinopathie, nicht proliferative diabetische Retinopathie (NPDR), diabetisches Makula-Ödem (DME), atrophische und hypertrophische Veränderungen des retinalen Pigmentepithels, retinaler Venenverschluss, choroidaler retinaler Venenverschluss, Makula-Ödem, Makula-Ödem assoziiert mit retinalem Venenverschluss, Retinitis pigmentosa, Stargardt'sche Erkrankung, Frühgeborenen-Retinopathie, Glaukom, entzündliche Erkrankungen des Auges, beispielsweise Uveitis, Skleritis oder Endophthalmitis, Katarakt, Refraktionsanomalien, beispielsweise Myopie, Hyperopie, Astigmatismus oder Keratokonus, korneale Angiogenese als Folge von beispielsweise Keratitis, Hornhaut-Transplantation oder Keratoplastie, korneale Angiogenese als Folge von Hypoxie (beispielsweise durch extensives Tragen von Kontaktlinsen), Pterygium conjunctivae, subkorneales Ödem und intrakomeales Ödem.

Aufgrund ihres Wirkprofils eignen sich die erfindungsgemäße Verbindung der Formel (II) und die erfindungsgemäßen Darreichungsformen insbesondere zur Behandlung und/oder Prävention von kardiovaskulären und kardiopulmonalen Erkrankungen wie primären und sekundären Formen der pulmonalen Hypertonie, Herzinsuffizienz, Angina pectoris und Hypertonie sowie von thromboembolischen Erkrankungen, Ischämien, Gefäßerkrankungen, Mikrozirkulationsstörungen, Niereninsuffizienz, fibrotischen Erkrankungen und Arteriosklerose.

Bevorzugt eignet sich die erfindungsgemäße Verbindung der Formel (II) und die erfindungsgemäßen Darreichungsformen zur Behandlung und/oder Prävention von renalen und kardiorenalen Erkrankungen, insbesondere des chronischen Nierenversagens (CKD) und der diabetischen Nierenkrankheit (DKD), kardialen und kardiovaskulären Erkrankungen, insbesondere der Herzinsuffizienz (HFpEF und HFrEF), Herzinfarkt, Angina pectoris, Kardiomyopathien, Hypertonie und Arteriosklerose, pulmonaler und kardiopulmonaler Erkran¬kungen, insbesondere pulmonaler Hypertonie (PH), ophthalmologischen Erkrankungen, insbesondere der nicht proliferativen diabetischen Retinopathie (NPDR) und des diabetisches Makula-Ödems (DME), Erkrankungen des Zentralnervensystems, insbesondere der Demenz, Knochenerkrankungen, insbesondere Osteogenesis imperfecta, thromboembolischen Erkrankun¬gen, Muskeldystrophien, Ischämien, Gefäßerkrankungen, Mikro¬zirkulationsstörungen, fibrotischen Erkrankungen insbesondere der Systemischen Sklerose, inflammatorischen Erkrankungen, und Stoffwechselerkrankungen, insbesondere des Metabolischen Syndroms, der Dyslipidämie und Diabetes.

Bevorzugt eignet sich die erfindungsgemäße Verbindung der Formel (II) und die erfindungsgemäßen Darreichungsformen zur Behandlung und/oder Prävention von renalen und kardiorenalen Erkrankungen, insbesondere des chronischen Nierenversagens (CKD).

Bevorzugt eignet sich die erfindungsgemäße Verbindung der Formel (II) und die erfindungsgemäßen Darreichungsformen zur Behandlung und/oder Prävention von ophthalmologischen Erkrankungen, insbesondere der nicht proliferativen diabetischen Retinopathie (NPDR) und des diabetisches Makula-Ödems (DME).

Bevorzugt eignen sich die erfindungsgemäße Verbindung der Formel (II) und die erfindungsgemäßen Darreichungsformen zur Behandlung und/oder Prävention von kardiovaskulären Erkrankungen, insbesondere der Herzinsuffizienz einschließlich der Herzinsuffizienz mit reduzierter Ejektionsfraktion (HFrEF) und der Herzinsuffizienz mit erhaltener Ejektionsfraktion (HFpEF).

Bevorzugt eignen sich die erfindungsgemäße Verbindung der Formel (II) und die erfindungsgemäßen Darreichungsformen zur Behandlung und/oder Prävention von kardiopulmonalen Erkrankungen, insbesondere der pulmonalen Hypertonie.

Bevorzugt eignen sich die erfindungsgemäße Verbindung der Formel (II) und die erfindungsgemäßen Darreichungsformen zur Behandlung und/oder Prävention von Erkrankungen des Zentralnervensystems, insbesondere der Demenz, einschießlich der vaskulären Demenz und Mischformen der Demenz.

Bevorzugt eignen sich die erfindungsgemäße Verbindung der Formel (II) und die erfindungsgemäßen Darreichungsformen zur Behandlung und/oder Prävention von "muskuläre- oder neuromuskuläre Erkrankungen", insbesondere Duchenne-Muskeldystrophie (DMD) und Becker-Muskeldystrophie (BMD).

Weiterer Gegenstand der vorliegenden Erfindung ist die Verwendung der erfindungsgemäßen Verbindung der Formel (II) und die erfindungsgemäßen Darreichungsformen zur Behandlung und/oder Prävention von Sichelzellenanämie, wobei traumatisierte Patienten einen synthetischen Blutersatzstoff erhalten, und zur Konservierung von Blutersatzstoffen.

Weiterer Gegenstand der vorliegenden Erfindung ist die Verwendung der erfindungsgemäßen Verbindung der Formel (II) und die erfindungsgemäßen Darreichungsformen zur Behandlung und/oder Prävention von Polyzystischem Ovar-Syndrom (PCOS).

Weiterer Gegenstand der vorliegenden Erfindung ist die Verwendung der erfindungsgemäßen Verbindung der Formel (II) und die erfindungsgemäßen Darreichungsformen zur Behandlung und/oder Prävention von Präeklampsie.

Weiterer Gegenstand der vorliegenden Erfindung ist die Verwendung der erfindungsgemäßen Verbindung der Formel (II) und die erfindungsgemäßen Darreichungsformen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindung der Formel (II) und die erfindungsgemäßen Darreichungsformen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge der erfindungsgemäße Verbindung der Formel (II) oder von mindestens einer erfindungsgemäßen Darreichungsform.

Die erfindungsgemäße Verbindung der Formel (II) oder die erfindungsgemäßen Darreichungsformen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Darreichungsformen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Weitere Substanzen, die die cGMP-Konzentration erhöhen, wie beispielsweise Protoporphyrin IX, Arachidonsäure oder Phenylhydrazinderivate;
- NO Synthase-Substrate, wie beispielsweise N-hydroxyguanidin-Derivate, L-arginin-Derivate, N-alkyl-N'-hydroxyguanidin-Derivate, N-aryl-N'-hydroxyguanidin-Derivate oder Guanidin-Derivate;
- Verbindungen, die den Abbau von cyclischem Guanosinmonophosphat (cGMP) und/oder cyclischem Adenosinmonophosphat (cAMP) inhibieren, beispielsweise Inhibitoren der Phosphodiesterasen (PDE) 1, 2, 3, 4, 5, 9 und/oder 10, insbesondere PDE-4-Inhibitoren wie Roflumilast oder Revamilast und PDE-5-Inhibitoren wie Sildenafil, Vardenafil, Tadalafil, Udenafil, Dasantafil, Avanafil, Mirodenafil oder Lodenafil;
- NO-unabhängige, jedoch Häm-abhängige Stimulatoren der Guanylatcyclase, insbesondere Riociguat, Nelociguat, Vericiguat, Praliciguat (IW-1973), Olinciguat (IW-1701) sowie die in WO 00/06568, WO 00/06569, WO 02/42301, WO 03/095451, WO 2011/147809, WO 2012/004258, WO 2012/028647 und WO 2012/059549 beschriebenen Verbindungen;
- Prostacyclin-Analoga und IP-Rezeptor-Agonisten, beispielhaft und vorzugsweise Iloprost, Beraprost, Treprostinil, Epoprostenol, NS-304, Selexipag oder Ralinepag;
- Endothelin-Rezeptor-Antagonisten, beispielhaft und vorzugsweise Bosentan, Darusentan, Ambrisentan, Macicentan oder Sitaxsentan;
- Inhibitoren der humanen neutrophilen Elastase (HNE), beispielhaft und vorzugsweise Sivelestat oder DX-890 (Reltran);
- die Signaltransduktionskaskade inhibierende Verbindungen, insbesondere aus der Gruppe der Tyrosinkinase-Inhibitoren, beispielhaft und vorzugsweise Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib, Masitinib oder Tandutinib;
- Rho-Kinase-Inhibitoren, beispielhaft und vorzugsweise Fasudil, Y-27632, SLx-2119, BF-66851, BF-66852, BF-66853, KI-23095 oder BA-1049;
- anti-obstruktiv wirkende Mittel, wie sie beispielsweise zur Therapie einer chronisch-obstruktiven Lungenerkrankung (COPD) oder eines Asthma bronchiale eingesetzt werden, beispielhaft und vorzugsweise inhalativ oder systemisch angewendete beta-Rezeptor-Mimetika (z.B. Bedoradrin) oder inhalativ angewendete anti-muscarinerge Substanzen;
- entzündungshemmmende und/oder immunsuppressive Mittel, wie sie beispielsweise zur Therapie einer chronisch-obstruktiven Lungenerkrankung (COPD), eines Asthma bronchiale oder einer Lungenfibrose eingesetzt werden, beispielhaft und vorzugsweise systemisch oder inhalativ angewendete Corticosteroide, Flutiform, Pirfenidon, Acetylcystein, Azathioprin oder BIBF-1120;
- Chemotherapeutika, wie sie beispielsweise zur Therapie von Neubildungen (Neoplasien) der Lunge oder anderer Organe eingesetzt werden;
- Wirkstoffe, die zur systemischen und/oder inhalativen Behandlung von Lungenerkrankungen eingesetzt werden, beispielsweise bei zystischer Fibrose (alpha-1-Antitrypsin, Aztreonam, Ivacaftor, Lumacaftor, Ataluren, Amikacin, Levofloxacin), chronisch-obstruktiven Lungenerkrankungen (COPD) (LAS40464, PT003, SUN-101), akutem Atemwegssyndrom (ARDS) und akuter Lungenschädigung (ALI) (Interferon-beta-1a, Traumakine), obstruktiver Schlafapnoe (VI-0521), Bronchiektasie (Mannitol, Ciprofloxacin), Bronchiolitis obliterans (Cyclosporin, Aztreonam) und Sepsis (Pagibaximab, Voluven, ART-123);
- Wirkstoffe, die zur Behandlung von Muskeldystrophie eingesetzt werden, beispielsweise Idebenon;
- antithrombotisch wirkende Mittel, beispielhaft und vorzugsweise aus der Gruppe der Thrombozytenaggregationshemmer, der Antikoagulantien oder der profibrinolytischen Substanzen;
- den Fettstoffwechsel verändernde Wirkstoffe, beispielhaft und vorzugsweise aus der Gruppe der Thyroidrezeptor-Agonisten, Cholesterinsynthese-Inhibitoren, beispielhaft und vorzugsweise HMG-CoA-Reduktase- oder Squalensynthese-Inhibitoren, ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten;
- Wirkstoffe, die die Neoangiogenese hemmen, beispielhaft und vorzugsweise Inhibitoren der VEGF- und/oder PDGF-Signalwege, Inhibitoren der Integrin-Signalwege, Inhibitoren der Angiopoietin-Tie-Signalwege, Inhibitoren der PI3K-Akt-mTor-Signalwege, Inhibitoren des Ras-Raf-Mek-Erk-Signalwegs, Inhibitoren der MAPK-Signalwege, Inhibitoren der FGF-Signalwege, Inhibitoren der Sphingosin-1-phosphat-Signalwege, Hemmer der Endothelzellproliferation oder Apoptose-induzierende Wirkstoffe;
- Wirkstoffe, die die Gefäßwanddurchlässigkeit (Ödembildung) verringern, beispielhaft und vorzugsweise Corticosteroide, Inhibitoren des ALK1-Smad1/5-Signalwegs, Inhibitoren der VEGF- und/oder PDGF-Signalwege, Cyclooxygenase-Inhibitoren, Hemmer des Kallikrein-Kinin-Systems oder Inhibitoren der Sphingosin-1-phosphat-Signalwege;
- Wirkstoffe, die die Schädigung der Netzhaut bei oxidativem Stress verringern, beispielhaft und vorzugsweise Inhibitoren des Komplementsystems, insbesondere Antagonisten des Komplement-C5a-Rezeptors, oder Agonisten des 5-HT_{1A}-Rezeptors;
- Antioxidantien und Radikalfänger;
- den Blutdruck senkende Wirkstoffe, beispielhaft und vorzugsweise aus der Gruppe der Calcium-Antagonisten, Angiotensin-AII-Antagonisten, ACE-Hemmer, beta-Rezeptoren-Blocker, alpha-Rezeptoren-Blocker, Diuretika, Phosphodiesterase-Inhibitoren, sGC-Stimulatoren, Verstärker der cGMP-Spiegel, ECE-Inhibitoren, Vasopeptidase-Inhibitoren und/oder Mineralocorticoid-Rezeptor-Antagonisten;
- Antiarrhythmika, wie beispielsweise Natriumkanalblocker, Beta-Rezeptor-Blocker, Kaliumkanalblocker oder Calciumkanalblocker;
- Alpha-1-adrenozeptor Antagonisten;
- Zentral wirkende Alpha-2-adrenozeptor Agonisten;
- Imidazolin I-1 Rezeptor Agonisten;
- Dopamin D1 Rezeptor Agonisten;
- 5-HT2 Antagonisten;
- Vasopressin Antagonisten;
- Calciumkanal-Sensitizer;
- Bronchodilatoren, wie beispielsweise Beta-2-Adrenozeptor-Agonisten, Anticholinergica, Theopyllin oder PDE Inhibitoren;
- Corticosteroide, wie beispielsweise Prednisolon;
- PGD2 Rezeptor Antagonisten;
- Nichtsteroidale Antiasthmatika, wie beispielsweise Beta-2-Adrenozeptor Agonisten oder Kombinationen von Beta-2-Adrenozeptor Agonisten und Corticosteroiden;
- Nicht steroidale Antirheumatika (NSAIDs) und selektive Cyclooxigenase-2 (COX-2)-Inhibitoren;
- Arzenimittel gegen Übergewicht und Fettleibigkeit wie beispielsweise Methamphetamin, Amfepramon, Phentermin, Benzphetamin, Phendimetrazin, Mazindol, Orlistat, Sibutramin oder Rimonabant und Kombinationen wie beispielsweise Phentermin/Topiramat, Bupropion/Naltrexon, Sibutramin/Metformin, Bupropion SR/Zonisamid SR, Salmeterol, Xinafoate/Fluticason; Lorcaserin, Phentermin/Topiramat, Cetilistat, Exenatid, Liraglutid, Metformin, CORT-108297, Canagliflozin, Chromium Picolinat, GSK-1521498, LY-377604, Metreleptin, Obinepitide, P-S7AS3, PSN-821, Salmeterol Xinafoat/Fluticason, Somatropin (rekombinant), Tesamorelin, Tesofensin, Velneperit, Zonisamid, Beloranib, Resveratrol, Sobetirom, Tetrahydrocannabivarin und Beta-Lapachon;
- Adenylatcyclase Inhibitoren, wie beispielsweise Colforsin dapropat;
- Positiv inotrope Substanzen, wie beispielsweise Digoxin;
- Arzneistoffe zur Behandlung der erektilen Dysfunktion, wie beispielsweise Alprostadil;
- Antidementiva wie Acetylcholinesterase-Inhibitoren, wie beispielsweise Donepezil, Galantamin und Rivastigmin; oder NMDA-Rezeptor Antagonisten, wie beispielsweise Memantin;
- Arzneimittel zur Behandlung von psychischen Störungen, wie beispielsweise Dopamin-D4-Rezeptor-Antagonisten wie Clozapin, Dopamin D2-Rezeptorantagonisten, wie Nemonaprid, gemischte Dopamin-D1/D2-Rezeptor-Antagonisten wie Zuclopenthixol, GABA A-Rezeptormodulatoren wie Carbamazepin, Natriumkanalinhibitoren wie Lamotrigin, Monoamin-Oxidase-Inhibitoren wie Moclobemid, trizyklische Antidepressiva wie Amitriptylin, Desipramin, Imipramin, Amoxapin, Nortriptylin oder Clomipramin, selektive Serotonin-Wiederaufnahmehemmer (SSRIs) wie Paroxetin, Fluoxetin oder Citralopram, Doxepin, Trazodonc oder Agomelatin, selektive Noradrenalin-Wiederaufnahmehemmer (SNRls) wie Venlafaxin oder dopaminerge Antidepressiva wie Bupropion;
- Inhibitoren der neuralen Endopeptidase (NEP Inhibitoren) wie Sacubitril, Omapatrilat oder Methylenblau, AVE-7688, oder in dualer Kombination (,ARNIs') mit Angiotensin Rezeptor Blockern (z.B. Valsartan), wie z.B. LCZ696;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Antidiabetika, beispielhaft und vorzugsweise aus der Gruppe der Insuline und Insulin-Derivate, Sulfonylharnstoffe, Biguanide, Meglitinid-Derivate, Glukosidase-Inhibitoren, PPAR-gamma-Agonisten, GLP 1-Rezeptor-Agonisten, Glukagon-Antagonisten, Insulin-Sensitizer, CCK1-Rezeptor-Agonisten, Inhibitoren der Dipeptidylpeptidase 4 (Gliptine), SGLT-2-Inhibitoren, Leptin-Rezeptor-Agonisten, Kaliumkanal-Antagonisten und der Inhibitoren von Leberenzymen, die an der Stimulation der Glukoneogenese und/oder Glykogenolyse beteiligt sind;
- Antiinfektiva, beispielhaft und vorzugsweise aus der Gruppe der antibakteriell, antifungisch und/oder antiviral wirksamen Substanzen; und/oder
- Substanzen zur Behandlung des Glaukoms, beispielhaft und vorzugsweise aus der Gruppe der Adrenergika, Beta-Rezeptor-Blocker, Carboanhydrase-Inhibitoren, Parasympathomimetika und der Prostaglandine; und/oder
- Substanzen zur Behandlung von Knochenerkrankungen, beispielhaft und vorzugsweise Bisphosphonate, Vitamin D oder dessen Metabolite, Strontiumranelat, selektive Östrogen Rezeptormodulatoren (SERM), Parathormon oder dessen Analoga und/oder RANKL (*receptor activator of nuclear factor kappa-B ligand*)-Modulatoren.

Unter antithrombotisch wirkenden Mitteln werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäße Verbindung der Formel (II) oder die erfindungsgemäßen Darreichungsformen in Kombination mit einem Thrombozytenaggregationshemmer, beispielhaft und vorzugsweise Aspirin, Clopidogrel, Ticlopidin oder Dipyridamol, verabreicht.

Bei einer bevorzugten Ausführungsform der Erfindung die erfindungsgemäße Verbindung der Formel (II) oder die erfindungsgemäßen Darreichungsformen in Kombination mit einem Thrombin-Inhibitor, beispielhaft und vorzugsweise Ximelagatran, Melagatran, Dabigatran, Bivalirudin oder Clexane, verabreicht.

Bei einer bevorzugten Ausführungsform der Erfindung die erfindungsgemäße Verbindung der Formel (II) oder die erfindungsgemäßen Darreichungsformen in Kombination mit einem GPIIb/IIIa-Antagonisten, beispielhaft und vorzugsweise Tirofiban oder Abciximab, verabreicht.

Bei einer bevorzugten Ausführungsform der Erfindung die erfindungsgemäße Verbindung der Formel (II) oder die erfindungsgemäßen Darreichungsformen in Kombination mit einem Faktor Xa-Inhibitor, beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäße Verbindung der Formel (II) oder die erfindungsgemäßen Darreichungsformen in Kombination mit Heparin oder einem niedermolekularen (low molecular weight, LMW) Heparin-Derivat verabreicht.

Bei einer bevorzugten Ausführungsform der Erfindung werden die erfindungsgemäße Verbindung der Formel (II) oder die erfindungsgemäßen Darreichungsformen in Kombination mit einem Vitamin K-Antagonisten, beispielhaft und vorzugsweise Coumarin, Phenprocumon oder Warfarin verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäße Verbindung der Formel (II) oder die erfindungsgemäßen Darreichungsformen in Kombination mit einem Calcium-Antagonisten, beispielhaft und vorzugsweise Nifedipin, Amlodipin, Verapamil oder Diltiazem, verabreicht.

Bei einer bevorzugten Ausführungsform der Erfindung werden die erfindungsgemäße Verbindung der Formel (II) oder die erfindungsgemäßen Darreichungsformen in Kombination mit einem Alpha-1-Adrenozeptor Antagonisten, beispielhaft und vorzugsweise Prazosin, verabreicht.

Bei einer bevorzugten Ausführungsform der Erfindung werden die erfindungsgemäße Verbindung der Formel (II) oder die erfindungsgemäßen Darreichungsformen in Kombination mit einem beta-Rezeptoren-Blocker, beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäße Verbindung der Formel (II) oder die erfindungsgemäßen Darreichungsformen in Kombination mit einem Angiotensin AII-Antagonisten, beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Darreichungsformen in Kombination mit einem ACE-Hemmer, beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäße Verbindung der Formel (II) oder die erfindungsgemäßen Darreichungsformen in Kombination mit einem Endothelin-Antagonisten, beispielhaft und vorzugsweise Bosentan, Darusentan, Ambrisentan oder Sitaxsentan, verabreicht.

Bei einer bevorzugten Ausführungsform der Erfindung werden die erfindungsgemäße Verbindung der Formel (II) oder die erfindungsgemäßen Darreichungsformen in Kombination mit einem Renin-Inhibitor, beispielhaft und vorzugsweise Aliskiren, SPP-600 oder SPP-800, verabreicht.

Bei einer bevorzugten Ausführungsform der Erfindung werden die erfindungsgemäße Verbindung der Formel (II) oder die erfindungsgemäßen Darreichungsformen in Kombination mit einem Mineralocorticoid-Rezeptor-Antagonisten, beispielsweise Spironolacton oder Eplerenon, besonders bevorzugt mit einem nicht-steroidalen Mineralocorticoid-Rezeptor-Antagonisten wie Finerenon, verabreicht.

Bei einer bevorzugten Ausführungsform der Erfindung werden die erfindungsgemäße Verbindung der Formel (II) oder die erfindungsgemäßen Darreichungsformen in Kombination mit einem Diuretikum, beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäße Verbindung der Formel (II) oder die erfindungsgemäßen Darreichungsformen in Kombination mit einem CETP-Inhibitor, beispielhaft und vorzugsweise Torcetrapib (CP-5294/4), JJT-705 oder CETP-vaccine (Avant), verabreicht.

Bei einer bevorzugten Ausführungsform der Erfindung werden die erfindungsgemäße Verbindung der Formel (II) oder die erfindungsgemäßen Darreichungsformen in Kombination mit einem Thyroidrezeptor-Agonisten, beispielhaft und vorzugsweise D-Thyroxin, 3,5,3'-Triiodothyronin (T3), CGS 23425 oder Axitirom (CGS 26214), verabreicht.

Bei einer bevorzugten Ausführungsform der Erfindung werden die erfindungsgemäße Verbindung der Formel (II) oder die erfindungsgemäßen Darreichungsformen in Kombination mit einem HMG-CoA-Reduktase-Inhibitor aus der Klasse der Statine, beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäße Verbindung der Formel (II) oder die erfindungsgemäßen Darreichungsformen in Kombination mit einem Squalensynthese-Inhibitor, beispielhaft und vorzugsweise BMS-188494 oder TAK-475, verabreicht.

Bei einer bevorzugten Ausführungsform der Erfindung werden die erfindungsgemäße Verbindung der Formel (II) oder die erfindungsgemäßen Darreichungsformen in Kombination mit einem ACAT-Inhibitor, beispielhaft und vorzugsweise Avasimib, Melinamid, Pactimib, Eflucimib oder SMP-797, verabreicht.

Bei einer bevorzugten Ausführungsform der Erfindung werden die erfindungsgemäße Verbindung der Formel (II) oder die erfindungsgemäßen Darreichungsformen in Kombination mit einem MTP-Inhibitor, beispielhaft und vorzugsweise Implitapid, BMS-201038, R-103757 oder JTT-130, verabreicht.

Bei einer bevorzugten Ausführungsform der Erfindung werden die erfindungsgemäße Verbindung der Formel (II) oder die erfindungsgemäßen Darreichungsformen in Kombination mit einem PPAR-gamma-Agonisten, beispielhaft und vorzugsweise Pioglitazon oder Rosiglitazon, verabreicht.

Bei einer bevorzugten Ausführungsform der Erfindung werden die erfindungsgemäße Verbindung der Formel (II) oder die erfindungsgemäßen Darreichungsformen in Kombination mit einem PPAR-delta-Agonisten, beispielhaft und vorzugsweise GW 501516 oder BAY 68-5042, verabreicht.

Bei einer bevorzugten Ausführungsform der Erfindung werden die erfindungsgemäße Verbindung der Formel (II) oder die erfindungsgemäßen Darreichungsformen in Kombination mit einem Cholesterin-Absorptionshemmer, beispielhaft und vorzugsweise Ezetimib, Tiquesid oder Pamaquesid, verabreicht.

Bei einer bevorzugten Ausführungsform der Erfindung werden die erfindungsgemäße Verbindung der Formel (II) oder die erfindungsgemäßen Darreichungsformen in Kombination mit einem Lipase-Inhibitor, beispielhaft und vorzugsweise Orlistat, verabreicht.

Bei einer bevorzugten Ausführungsform der Erfindung werden die erfindungsgemäße Verbindung der Formel (II) oder die erfindungsgemäßen Darreichungsformen in Kombination mit einem polymeren Gallensäureadsorber, beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäße Verbindung der Formel (II) oder die erfindungsgemäßen Darreichungsformen in Kombination mit einem Gallensäure-Reabsorptionshemmer, beispielhaft und vorzugsweise ASBT (= IBAT)-Inhibitoren, z.B. AZD-7806, S-8921, AK-105, BARI-1741, SC-435 oder SC-635, verabreicht.

Bei einer bevorzugten Ausführungsform der Erfindung werden die erfindungsgemäße Verbindung der Formel (II) oder die erfindungsgemäßen Darreichungsformen in Kombination mit einem Lipoprotein(a)-Antagonisten, beispielhaft und vorzugsweise Gemcabene calcium (CI-1027) oder Nicotinsäure, verabreicht.

Bei einer bevorzugten Ausführungsform der Erfindung werden die erfindungsgemäße Verbindung der Formel (II) oder die erfindungsgemäßen Darreichungsformen in Kombination mit Acetylcholinesterase-Inhibitoren, beispielhaft und vorzugsweise Donepezil, Galantamin oder Rivastigmin, verabreicht.

Bei einer bevorzugten Ausführungsform der Erfindung werden die erfindungsgemäße Verbindung der Formel (II) oder die erfindungsgemäßen Darreichungsformen in Kombination mit NMDA-Rezeptor Antagonisten, beispielhaft und vorzugsweise Memantin, verabreicht.

Bei einer bevorzugten Ausführungsform der Erfindung werden die erfindungsgemäße Verbindung der Formel (II) oder die erfindungsgemäßen Darreichungsformen in Kombination mit sGC Stimulatoren, beispielhaft und vorzugsweise Riociguat, Nelociguat, Vericiguat, Praliciguat (IW-1973) oder Olinciguat (IW-1701) verabreicht.

Bei einer bevorzugten Ausführungsform der Erfindung werden die erfindungsgemäße Verbindung der Formel (II) oder die erfindungsgemäßen Darreichungsformen in Kombination mit Antidiabetika, beispielhaft und vorzugsweise Metformin verabreicht.

Bei einer bevorzugten Ausführungsform der Erfindung werden die erfindungsgemäße Verbindung der Formel (II) oder die erfindungsgemäßen Darreichungsformen in Kombination mit SGLT-2-Inhibitoren, beispielhaft und vorzugsweise Dapagliflozin, Empagliflozin, Canagliflozin, Ipragliflozin und/oder Tofogliflozin verabreicht.

Bei einer bevorzugten Ausführungsform der Erfindung werden die erfindungsgemäße Verbindung der Formel (II) oder die erfindungsgemäßen Darreichungsformen in Kombination mit Substanzen zur Behandlung von Knochenerkrankungen wie beispielhaft und vorzugsweise Vitamin D oder dessen Metabolite, Strontiumranelat, selektive Östrogen Rezeptormodulatoren (SERM) und/oder RANKL-Modulatoren verabreicht.

Bei einer bevorzugten Ausführungsform der Erfindung werden die erfindungsgemäße Verbindung der Formel (II) oder die erfindungsgemäßen Darreichungsformen in Kombination mit Bisphosphonaten, wie beispielhaft und vorzugsweise Etidronate, Clodronate, Tiludronate, Teriparatide, Pamidronate, Neridronate, Olpadronate, Alendronate, Ibandronate, Risedronate oder Zoledronate verabreicht.

Ein weiterer Gegenstand ist die Verbindung der Formel (II), bevorzugt in kristalliner Form der Modifikation 1, zur Behandlung und/oder Prävention von Krankheiten.

Ein weiterer Gegenstand ist die Verbindung der Formel (II), bevorzugt in kristalliner Form der Modifikation 1, zur Behandlung und/oder Prävention von renalen und kardiorenalen Erkrankungen, insbesondere des chronischen Nierenversagens (CKD) und der diabetischen Nierenkrankheit (DKD), kardialen und kardiovaskulären Erkrankungen, insbesondere der Herzinsuffizienz (HFpEF und HFrEF), Herzinfarkt, Angina pectoris, Kardiomyopathien, Hypertonie und Arteriosklerose, pulmonaler und kardiopulmonaler Erkrankungen, insbesondere pulmonaler Hypertonie (PH), Erkrankungen des Zentralnervensystems, insbesondere der Demenz, Knochenerkrankungen, insbesondere Osteogenesis imperfecta, thromboembolischen Erkrankungen, Muskeldystrophien, Ischämien, Gefäßerkrankungen, Mikrozirkulationsstörungen, fibrotischen Erkrankungen insbesondere der Systemischen Sklerose, ophthalmologischen Erkrankungen, inflammatorischen Erkrankungen, und Stoffwechselerkrankungen, insbesondere des Metabolischen Syndroms, der Dyslipidämie und Diabetes.

Ein weiterer Gegenstand ist die Verbindung der Formel (II), bevorzugt in kristalliner Form der Modifikation 1, zur Behandlung und/oder Prävention von renalen und kardiorenalen Erkrankungen, insbesondere des chronischen Nierenversagens (CKD) und der diabetischen Nierenkrankheit (DKD).

Ein weiterer Gegenstand ist die Verbindung der Formel (II), bevorzugt in kristalliner Form der Modifikation 1, zur Behandlung und/oder Prävention von kardialen und kardiovaskulären Erkrankungen, insbesondere der Herzinsuffizienz (HFpEF und HFrEF), Herzinfarkt, Angina pectoris, Kardiomyopathien, Hypertonie und Arteriosklerose.

Ein weiterer Gegenstand ist die Verbindung der Formel (II), bevorzugt in kristalliner Form der Modifikation 1, zur Behandlung und/oder Prävention von pulmonalen und kardiopulmonalen Erkrankungen, insbesondere pulmonaler Hypertonie (PH).

Ein weiterer Gegenstand ist die Verbindung der Formel (II), bevorzugt in kristalliner Form der Modifikation 1, zur Behandlung und/oder Prävention von Erkrankungen des Zentralnervensystems, insbesondere der Demenz.

Ein weiterer Gegenstand ist die Verbindung der Formel (II), bevorzugt in kristalliner Form der Modifikation 1, zur Behandlung und/oder Prävention von Erkrankungen des Zentralnervensystems, insbesondere der vaskulären und Alzheimer Demenz.

Ein weiterer Gegenstand ist die Verbindung der Formel (II), bevorzugt in kristalliner Form der Modifikation 1, zur Behandlung und/oder Prävention von Stoffwechselerkrankungen, insbesondere des Metabolischen Syndroms, der Dyslipidämie und Diabetes.

Ein weiterer Gegenstand ist die Verwendung eines erfindungsgemäßen osmotischen Freisetzungssystems enthaltend die Verbindung der Formel (II), bevorzugt in kristalliner Form der Modifikation 1, zur Behandlung und/oder Prävention von Krankheiten.

Ein weiterer Gegenstand ist die Verwendung eines erfindungsgemäßen osmotischen Freisetzungssystems enthaltend die Verbindung der Formel (II), bevorzugt in kristalliner Form der Modifikation 1, zur Behandlung und/oder Prävention von renalen und kardiorenalen Erkrankungen, insbesondere des chronischen Nierenversagens (CKD) und der diabetischen Nierenkrankheit (DKD), kardialen und kardiovaskulären Erkrankungen, insbesondere der Herzinsuffizienz (HFpEF und HFrEF), Herzinfarkt, Angina pectoris, Kardiomyopathien, Hypertonie und Arteriosklerose, pulmonaler und kardiopulmonaler Erkrankungen, insbesondere pulmonaler Hypertonie (PH), Erkrankungen des Zentralnervensystems, insbesondere der Demenz, Knochenerkrankungen, insbesondere Osteogenesis imperfecta, thromboembolischen Erkrankungen, Muskeldystrophien, Ischämien, Gefäßerkrankungen, Mikrozirkulationsstörungen, fibrotischen Erkrankungen insbesondere der Systemischen Sklerose, ophthalmologischen Erkrankungen, inflammatorischen Erkrankungen, und Stoffwechselerkrankungen, insbesondere des Metabolischen Syndroms, der Dyslipidämie und Diabetes.

Ein weiterer Gegenstand ist die Verwendung eines erfindungsgemäßen osmotischen Freisetzungssystems enthaltend die Verbindung der Formel (II), bevorzugt in kristalliner Form der Modifikation 1, zur Behandlung und/oder Prävention von renalen und kardiorenalen Erkrankungen, insbesondere des chronischen Nierenversagens (CKD) und der diabetischen Nierenkrankheit (DKD).

Ein weiterer Gegenstand ist die Verwendung eines erfindungsgemäßen osmotischen Freisetzungssystems enthaltend die Verbindung der Formel (II), bevorzugt in kristalliner Form der Modifikation 1, zur Behandlung und/oder Prävention von kardialen und kardiovaskulären Erkrankungen, insbesondere der Herzinsuffizienz (HFpEF und HFrEF), Herzinfarkt, Angina pectoris, Kardiomyopathien, Hypertonie und Arteriosklerose.

Ein weiterer Gegenstand ist die Verwendung eines erfindungsgemäßen osmotischen Freisetzungssystems enthaltend die Verbindung der Formel (II), bevorzugt in kristalliner Form der Modifikation 1, zur Behandlung und/oder Prävention von pulmonalen und kardiopulmonalen Erkrankungen, insbesondere pulmonaler Hypertonie (PH).

Ein weiterer Gegenstand ist die Verwendung eines erfindungsgemäßen osmotischen Freisetzungssystems enthaltend die Verbindung der Formel (II), bevorzugt in kristalliner Form der Modifikation 1, zur Behandlung und/oder Prävention von Erkrankungen des Zentralnervensystems, insbesondere der Demenz.

Ein weiterer Gegenstand ist die Verwendung eines erfindungsgemäßen osmotischen Freisetzungssystems enthaltend die Verbindung der Formel (II), bevorzugt in kristalliner Form der Modifikation 1, zur Behandlung und/oder Prävention von Erkrankungen des Zentralnervensystems, insbesondere der vaskulären und Alzheimer Demenz.

Ein weiterer Gegenstand ist die Verwendung eines erfindungsgemäßen osmotischen Freisetzungssystems enthaltend die Verbindung der Formel (II), bevorzugt in kristalliner Form der Modifikation 1, zur Behandlung und/oder Prävention von Stoffwechselerkrankungen, insbesondere des Metabolischen Syndroms, der Dyslipidämie und Diabetes.

Ein weiterer Gegenstand sind Arzneimittel enthaltend die Verbindung der Formel (II) in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln, MR-Antagonisten, IP-Rezeptor-Agonisten, entzündungshemmenden Wirkstoffen, Antidementiva, Antidiabetika, den Fettstoffwechsel verändernden Wirkstoffen sowie Wirkstoffen zur Behandlung von Knochen- und Muskelerkrankungen.

In den erfindungsgemäßen Darreichungsformen ist die Verbindung der Formel (II) bevorzugt in einer Menge von etwa 1 bis 240 mg, besonders bevorzugt in einer Menge von etwa 1 mg bis 120 mg, ganz besonders bevorzugt in einer Menge von etwa 2,5 mg bis 50 mg enthalten. Gegenstand der vorliegenden Erfindung sind die zuvor erwähnten erfindungsgemäßen pharmazeutischen Darreichungsformen, wobei die Verbindung der Formel (II) bevorzugt in einer Menge von 1 mg, 2 mg, 2,5 mg, 3 mg, 4 mg, 5 mg, 6 mg, 7 mg, 8 mg, 9 mg, 10 mg, 12 mg, 15 mg, 20 mg, 25 mg, 30 mg, 35 mg, 40 mg, 50 mg, 60 mg, 70 mg, 75 mg, 80 mg, 90 mg, 100 mg, 110 mg, 120 mg, 125 mg, 150 mg, 175 mg, 200 mg, 225 mg und 240 mg enthalten ist. Die Mengen der Verbindung der Formel (II) bezeichnen die nominalen Mengen in der pharmazeutischen Darreichungsform, unter Umständen kann zusätzlich ein Überschuss von bis zu 20 % der Wirkstoffmenge enthalten sein.

Im Allgemeinen hat es sich als vorteilhaft erwiesen etwa 0,01 bis 10 mg/kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Experimenteller Teil

### Abkürzungen und Akronyme

- cp: Centipoise
- HPLC: Hochdruck-, Hochleistungsflüssigchromatographie
- K: Kelvin
- min: Minute
- ml: Milliliter
- µl: Mikroliter
- mm: Millimeter
- µm: Mikrometer
- mPa: Millipascal
- Ph. Eur.: European Pharmakopoe
- s: Sekunde
- U: Umdrehung
- USP: United States Pharmacopeia
- UV: Ultraviolett

### Ausführungsbeispiele

### Beispielverbindung 1

### Natrium-(3S)-3-(4-chlor-3-{[(2S,3R)-2-(4-chlorphenyl)-4,4,4-trifluor-3-methylbutanoyl]amino}phenyl)-3-cyclopropylpropanoat in kristalliner Modifikation 1

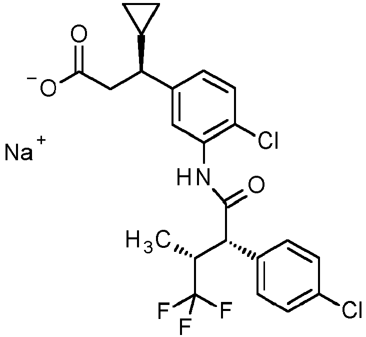

### Herstellungsmethode 1

Ein Reaktionsgefäß wurde mit 1425 g der Verbindung der Formel (I) (Herstellung offenbart in WO 2012/139888, Beispiel 22 und EP17204842.3 (veröffentlicht als WO 2019/105881)) und 13,3 kg Acetonitril unter Stickstoffatmosphäre befüllt. Das Gemisch wird gerührt, bis eine Lösung gebildet ist. 117 g festes Natriumhydroxid werden zugegeben und die erhaltene Suspension wird 25 Stunden gründlich gerührt. Die Suspension wird filtriert. Die gewonnenen Feststoffe werden mit 1,2 kg Acetonitril gewaschen und unter verringertem Druck bei 30 °C 19 Stunden getrocknet.
Ausbeute: 1375 g (92 %)
Gehalt der Verbindung der Formel (II): 96,4 % (HPLC-Methode 1)
Gehalt der Verbindung der Formel (III): < 0,20 % (HPLC-Methode 2)
Natriumgehalt: 4,8 %
XRPD: Modifikation 1
Herstellungsmethode 2

Ein Reaktionsgefäß wird mit 34,4 kg Acetonitril und 4,0 kg der Verbindung der Formel (I) (Gehaltsbestimmung 99,1 %) (Herstellung offenbart in WO 2012/139888, Beispiel 22 und EP17204842.3 (veröffentlicht als WO 2019/105881)) unter Stickstoffatmosphäre befüllt. Das Gemisch wird bei 20 °C gerührt. Die erhaltene Lösung wird filtriert und der Filter wird mit 3 kg Acetonitril gewaschen. Das Filtrat wird auf 0 °C abgekühlt. 3,9 kg einer Tetrahydrofuran-Lösung von Natrium-tert-butanolat (Gehaltsbestimmung 19,6 %) werden bei einer Temperatur von -5 °C bis +5 °C langsam zugegeben. Nach dem Zugeben von etwa 2/3 der Natrium-tert-butanolatlösung werden Impfkristalle der Verbindung der Formel (II) in kristalliner Modifikation 1 zugegeben. Nach dem Abschluss der Dosierung wird die Dosierungsleitung mit zusätzlichen 3,0 kg Tetrahydrofuran gespült. Das erhaltene Gemisch wird bei 0 °C 17 Stunden gerührt. Die Suspension wird filtriert und die gewonnenen Feststoffe werden zweimal mit 5,6 kg kaltem Acetonitril gewaschen. Das Produkt wird unter verringertem Druck bei 40 °C 16 Stunden getrocknet.
Ausbeute: 4,0 kg (97 %)
Gehalt der Verbindung der Formel (II): 98,7 % (HPLC-Methode 1)
Gehalt der Verbindung der Formel (III): 0,19 % (HPLC-Methode 2)
Natriumgehalt: 4,4 %
XRPD: Modifikation 1

### Analytische Methoden

### HPLC Methode 1:

Die Prüfungen zur Gehaltsbestimmung und auf die Verunreinigungen werden auf einer Umkehrphasen-HPLC-Säule mit UV-Nachweis bei 210 nm durchgeführt. Die stationäre Phase ist eine Zorbax Eclipse Plus RRHD C18 HPLC-Säule (50 mm × 2,1 mm, 1,8 µm Partikelgröße) oder eine geeignete Alternative.

Für die optimale Trennung der Maxima wurde eine Gradientenelution gewählt. Der Gradient der mobilen Phase wird in der nachstehenden Tabelle 1 gezeigt.

Die mobile Phase A ist Wasser mit 0,1 % Trifluoressigsäure, die mobile Phase B ist Acetonitril mit 0,1 % Trifluoressigsäure.

**Tabelle 1**

| **Zeit [min]** | **% A** | **% B** |
|---|---|---|
| 0,0 | 95 | 5 |
| 25,0 | 20 | 80 |

Die Flussrate beträgt 1,0 ml/min, die Säulentemperatur beträgt 20 °C, das Einspritzvolumen beträgt 2 µl. Prüflösungen werden durch Auflösen in einem Gemisch von gleichen Teilen Acetonitril und Wasser auf eine Konzentration von 0,46 mg/ml hergestellt.

Das Quantifizieren erfolgt entweder über externe Kalibrierung unter Verwendung eines Referenzstandards oder über Massebilanz. Die Retensionszeit der Verbindung der Formel (II) beträgt ca. 16,2 min., die Retensionszeit der Verbindung der Formel (III) beträgt ca. 12,0 min.

### HPLC-Methode 2:

Die Prüfungen auf Verunreinigungen werden auf einer Normalphasen-HPLC-Säule mit UV-Nachweis bei 220 nm durchgeführt. Die stationäre Phase ist eine Chiralpak AD-H HPLC-Säule (250 mm × 4,6 mm, 5 µm Partikelgröße) oder eine geeignete Alternative.

Für die optimale Trennung der Maxima wurde eine isokratische Elution gewählt.

Die mobile Phase besteht aus 93 Volumen-% Isohexan und 7 Volumen-% an einem Gemisch von 2-Propanol mit 0,2 % Trifluoressigsäure und 1 % Wasser.

Die Flussrate beträgt 1,25 ml/min, die Säulentemperatur beträgt 30 °C, das Einspritzvolumen beträgt 5 µl. Prüflösungen werden durch Auflösen in einem Gemisch von Isohexan und 2-Propanol (3/1, Volumen/Volumen) auf eine Konzentration von 0,5 mg/ml hergestellt.

Das Quantifizieren erfolgt über externe Kalibrierung unter Verwendung eines Referenzstandards. Die Retensionszeit der Verbindung der Formel (II) beträgt ca. 11,4 min., die Retensionszeit der Verbindung der Formel (III) beträgt ca. 9,7 min.

### Methode 3 (Analytik Natrium):

Natrium wird durch ein ICP-MS-Verfahren als halbquantitative Übersichtsanalyse analysiert. Die Probenvorbereitung erfolgt durch Mikrowellenaufschluss mit Salpetersäure.

### Methode 4 - Röntgendiffraktometrie für die Messung der Verbindung der Formel (I) in kristalliner Form der Modifikation 1:

| | |
|---|---|
| Probenvorbereitung: | Probe als flache Pulverschicht zwischen zwei Folien. |
| Gerät: | Röntgenpulverdiffraktometer (STOE STADI P) |
| Generator: | 40 kV / 40 mA |
| Detektor: | ortsempfindlicher Detektor |
| Strahlung: | germanium-monochromatized CuKa1-Strahlung |
| Messmodus: | Transmission |
| Messbereich: | 2° ≤ 2θ ≤ 40° |
| Schrittweite: | 0.5° |
| Messzeit: | 15 sec/step |

**Tabelle 2: Röntgendiffraktometrie Verbindung der Formel (I) in der kristallinen Modifikation 1**

| **Reflexe** | | | | | |
|---|---|---|---|---|---|
| Modifikation 1 | | | | | |
| 6,5 | 17,2 | 24,1 | 28,6 | 32,7 | 38,1 |
| 7,6 | 17,5 | 24,4 | 28,8 | 33,1 | 38,5 |
| 8,1 | 18,0 | 24,7 | 29,0 | 33,7 | 38,7 |
| 9,6 | 18,8 | 25,1 | 29,3 | 34,0 | 38,9 |
| 10,3 | 19,4 | 25,2 | 29,5 | 34,7 | 39,4 |
| 11,0 | 19,8 | 25,5 | 29,8 | 35,5 | 39,6 |
| 14,7 | 21,0 | 25,8 | 30,4 | 35,8 | 39,8 |
| 15,1 | 21,4 | 26,5 | 30,8 | 36,1 | |
| 15,6 | 21,7 | 26,8 | 31,2 | 36,3 | |
| 16,0 | 22,3 | 27,2 | 31,6 | 36,7 | |
| 16,4 | 22,6 | 28,1 | 32,3 | 37,7 | |

Das Röntgendiffraktogramm der Verbindung der Formel (I) in der kristallinen Modifikation 1 ist in Fig. 10 wiedergegeben.

### Methode 5 - IR-Spektroskopie für die Messung der Verbindung der Formel (I) in kristalliner Form der Modifikation 1:

| | |
|---|---|
| Probenpräparation: | Probe wurde als KBr-Scheibe präpariert |
| Gerät | Bruker Vertex 80v |
| Anzahl Scans | 32 |
| Auflösung | 2 cm⁻¹ |
| Technik | Transmission |

**Tabelle 3: IR-Spektren der Verbindung der Formel (I) in der kristallinen Modifikation 1**

| **Bandenmaximum [cm⁻¹]** | | | | | |
|---|---|---|---|---|---|
| Modifikation 1 | | | | | |
| 3381 | 1524 | 1245 | 975 | 735 | 532 |
| 3066 | 1492 | 1185 | 937 | 721 | 516 |
| 2997 | 1458 | 1169 | 906 | 712 | 492 |
| 2975 | 1419 | 1135 | 895 | 669 | 447 |
| 2954 | 1389 | 1108 | 844 | 654 | 422 |
| 2914 | 1376 | 1101 | 827 | 628 | |
| 1691 | 1312 | 1069 | 817 | 593 | |
| 1595 | 1286 | 1044 | 788 | 566 | |
| 1565 | 1263 | 1022 | 753 | 546 | |

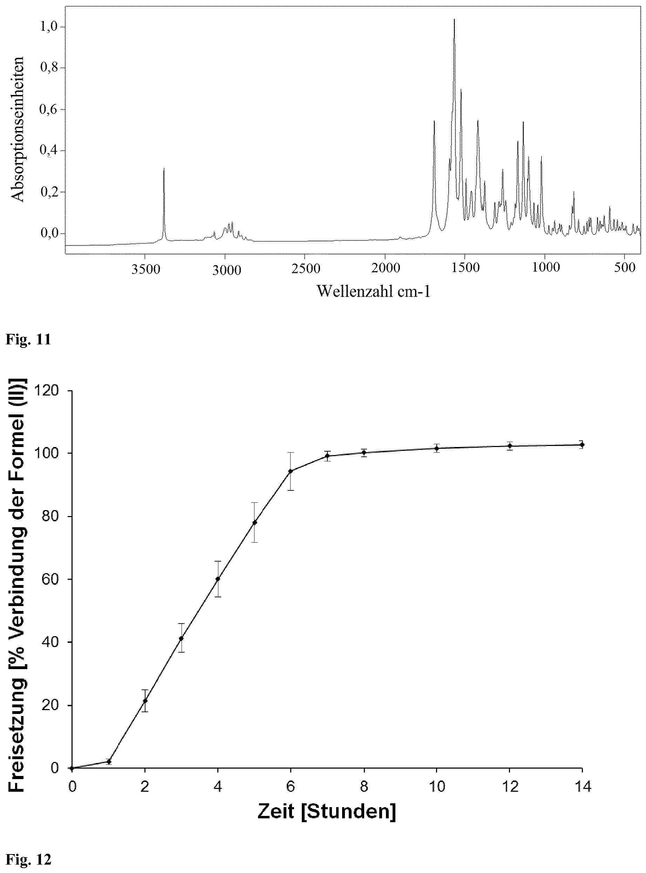

Das IR-Spektrum der Verbindung der Formel (I) in der kristallinen Modifikation 1 ist in Fig. 11 wiedergegeben.

Im folgenden werden osmotische Freisetzungssysteme sowie deren Herstellung dargestellt. Die osmotischen Freisetzungssysteme enthalten jeweils einen Wirkstoffüberschuss, da technisch bedingt ein Teil des Wirkstoffs im osmotischen Freisetzungssystem zurückbleibt.

### Osmotisches Freisetzungssystem 1 (Zweikammersystem, Polyethylenoxid als hydrophiles quellbares Polymer)

Tablettenzusammensetzung in mg/Tablette:

### Kern

### Wirkstoffschicht

| | |
|---|---|
| Verbindung der Formel (II), mikronisiert | 2,75 mg |
| Hydroxypropylmethylcellulose (5 cp) | 5,70 mg |
| Polyethylenoxid * | 100,45 mg |
| Hochdisperses Siliziumdioxid (Aerosil 200, Degussa) | 0,90 mg |
| Magnesiumstearat | 0,30 mg |
| | 110,1 mg |

### Osmoseschicht

| | |
|---|---|
| Hydroxypropylmethylcellulose (5 cp) | 3,69 mg |
| Natriumchlorid | 21,51 mg |
| Polyethylenoxid ** | 47,60 mg |
| Eisenoxid rot | 0,72 mg |
| Magnesiumstearat | 0,18 mg |
| | 73,70 mg |
| Gesamt (Kern) | 183,8 mg |

### Hülle

| | |
|---|---|
| Celluloseacetat | 12,40 mg |
| Polyethylenglycol 3350 | 1,60 mg |
| | 14,0 mg |
| Gesamt (osmotisches Freisetzungssystem) | 197,8 mg |

80 % der Verbindung der Formel (II) wurden nach etwa 5 bis 6 Stunden freigesetzt. Das Freisetzungsprofil der Verbindung der Formel (II) aus dem Osmotischen Freisetzungssystem 1 ist in Fig. 12 dargestellt.

### Osmotisches Freisetzungssystem 2 (Zweikammersystem, Polyethylenoxid als hydrophiles quellbares Polymer)

### Tablettenzusammensetzung in mg/Tablette:

### Kern

### Wirkstoffschicht

| | |
|---|---|
| Verbindung der Formel (II), mikronisiert | 6,00 mg |
| Hydroxypropylmethylcellulose (5 cp) | 5,70 mg |
| Polyethylenoxid * | 97,40 mg |
| Hochdisperses Siliziumdioxid (Aerosil 200, Degussa) | 0,90 mg |
| Magnesiumstearat | 0,30 mg |
| | 110,3 mg |

### Osmoseschicht

| | |
|---|---|
| Hydroxypropylmethylcellulose (5 cp) | 3,69 mg |
| Natriumchlorid | 21,51 mg |
| Polyethylenoxid ** | 47,60 mg |
| Eisenoxid rot | 0,72 mg |
| Magnesiumstearat | 0,18 mg |
| | 73,7 mg |
| Gesamt (Kern) | 184,0 mg |

### Hülle

| | |
|---|---|
| Celluloseacetat | 12,60 mg |
| Polyethylenglycol 3350 | 1,40 mg |
| | 14,0 mg |
| Gesamt (osmotisches Freisetzungssystem) | 198,0 mg |

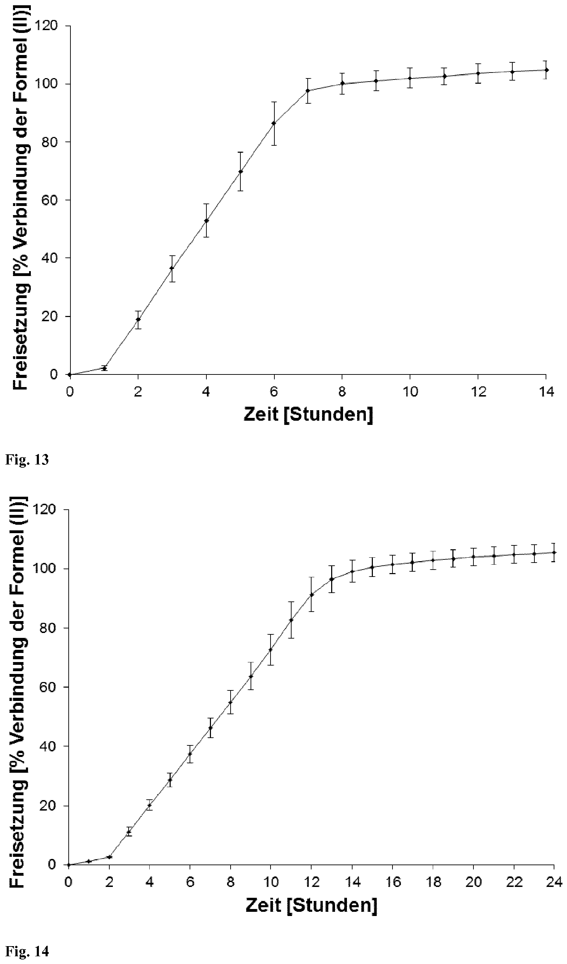

80 % der Verbindung der Formel (II) wurden nach etwa 6 Stunden freigesetzt. Das Freisetzungsprofil der Verbindung der Formel (II) aus dem Osmotischen Freisetzungssystem 2 ist in Fig. 13 dargestellt.

### Osmotisches Freisetzungssystem 3 (Zweikammersystem, Polyethylenoxid als hydrophiles quellbares Polymer)

### Tablettenzusammensetzung in mg/Tablette:

Die Zusammensetzung der Wirkstoffschicht und Osmoseschicht (Kern) entspricht dem Ausführungsbeispiel 2.

### Hülle

| | |
|---|---|
| Celluloseacetat | 25,20 mg |
| Polyethylenglycol 3350 | 2,80 mg |
| | 28,0 mg |
| Gesamt (osmotisches Freisetzungssystem) | 212,0 mg |

80 % der Verbindung der Formel (II) wurden nach etwa 11 Stunden freigesetzt. Das Freisetzungsprofil der Verbindung der Formel (II) aus dem Osmotischen Freisetzungssystem 3 ist in Fig. 14 dargestellt.

### Osmotisches Freisetzungssystem 4 (Zweikammersystem, Polyethylenoxid als hydrophiles quellbares Polymer)

### Tablettenzusammensetzung in mg/Tablette:

Die Zusammensetzung der Wirkstoffschicht und Osmoseschicht (Kern) entspricht dem Ausführungsbeispiel 2.

### Hülle

| | |
|---|---|
| Celluloseacetat | 34,20 mg |
| Polyethylenglycol 3350 | 3,80 mg |
| | 38,0 mg |
| Gesamt (osmotisches Freisetzungssystem) | 222,0 mg |

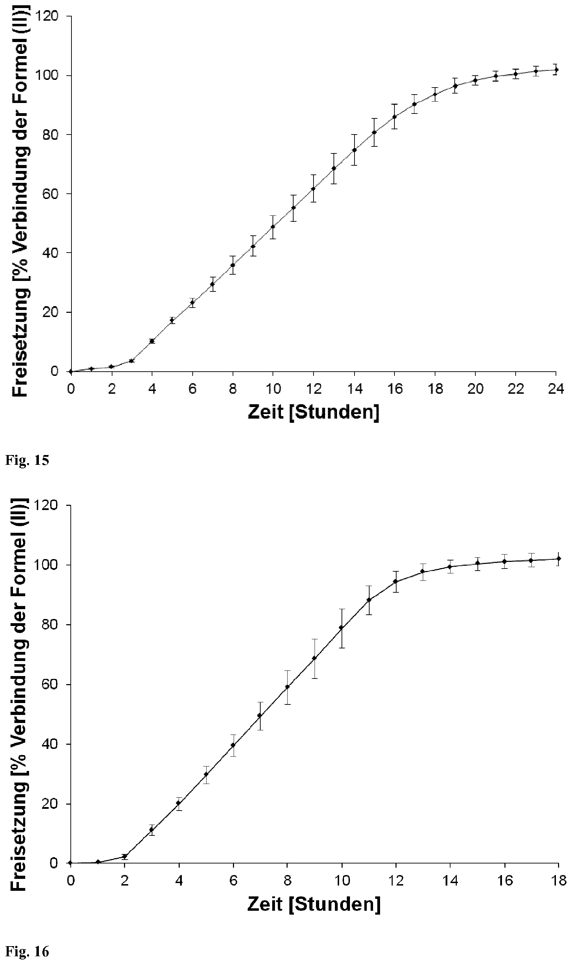

80 % der Verbindung der Formel (II) wurden nach etwa 15 Stunden freigesetzt. Das Freisetzungsprofil der Verbindung der Formel (II) aus dem Osmotischen Freisetzungssystem 4 ist in Fig. 15 dargestellt.

### Osmotisches Freisetzungssystem 5 (Zweikammersystem, Polyethylenoxid als hydrophiles quellbares Polymer)

### Tablettenzusammensetzung in mg/Tablette:

### Kern

### Wirkstoffschicht

| | |
|---|---|
| Verbindung der Formel (II), mikronisiert | 5,75 mg |
| Hydroxypropylmethylcellulose (5 cp) | 5,70 mg |
| Polyethylenoxid * | 97,65 mg |
| Hochdisperses Siliziumdioxid (Aerosil 200, Degussa) | 0,90 mg |
| Magnesiumstearat | 0,30 mg |
| | 110,3 mg |

### Osmoseschicht

| | |
|---|---|
| Hydroxypropylmethylcellulose (5 cp) | 3,69 mg |
| Natriumchlorid | 21,51 mg |
| Polyethylenoxid ** | 47,60 mg |
| Eisenoxid rot | 0,72 mg |
| Magnesiumstearat | 0,18 mg |
| | 73,7 mg |
| Gesamt (Kern) | 184,0 mg |

### Hülle

| | |
|---|---|
| Celluloseacetat | 27,00 mg |
| Polyethylenglycol 3350 | 3,00 mg |
| | 30,0 mg |
| Gesamt (osmotisches Freisetzungssystem) | 214,0 mg |

80 % der Verbindung der Formel (II) wurden nach etwa 10 Stunden freigesetzt. Das Freisetzungsprofil der Verbindung der Formel (II) aus dem Osmotischen Freisetzungssystem 5 ist in Fig. 16 dargestellt.

Das Osmotische Freisetzungssystem 5 wurde auf den Gehalt der Verbindung der Formel (II) (Wirkstoffgehalt) geprüft (n=10). Ausgehend von 100 % des deklarierten Wirkstoffgehaltes wurde ein minimaler Wirkstoffgehalt von 93.8 % und ein maximaler Wirkstoffgehalt von 103,7 % gemessen. Die Standardabweichung lag bei 3,1 %.

### Osmotisches Freisetzungssystem 6 (Zweikammersystem, Polyethylenoxid als hydrophiles quellbares Polymer)

### Tablettenzusammensetzung in mg/Tablette:

### Kern

### Wirkstoffschicht

| | |
|---|---|
| Verbindung der Formel (II), mikronisiert | 17,24 mg |
| Hydroxypropylmethylcellulose (5 cp) | 5,70 mg |
| Polyethylenoxid * | 86,16 mg |
| Hochdisperses Siliziumdioxid (Aerosil 200, Degussa) | 0,90 mg |
| Magnesiumstearat | 0,30 mg |
| | 110,3 mg |

### Osmoseschicht

| | |
|---|---|
| Hydroxypropylmethylcellulose (5 cp) | 3,69 mg |
| Natriumchlorid | 21,51 mg |
| Polyethylenoxid ** | 47,60 mg |
| Eisenoxid rot | 0,72 mg |
| Magnesiumstearat | 0,18 mg |
| | 73,70 mg |
| Gesamt (Kern) | 184,0 mg |

### Hülle

| | |
|---|---|
| Celluloseacetat | 27,00 mg |
| Polyethylenglycol 3350 | 3,00 mg |
| | 30,00 mg |
| Gesamt (osmotisches Freisetzungssystem) | 214,0 mg |

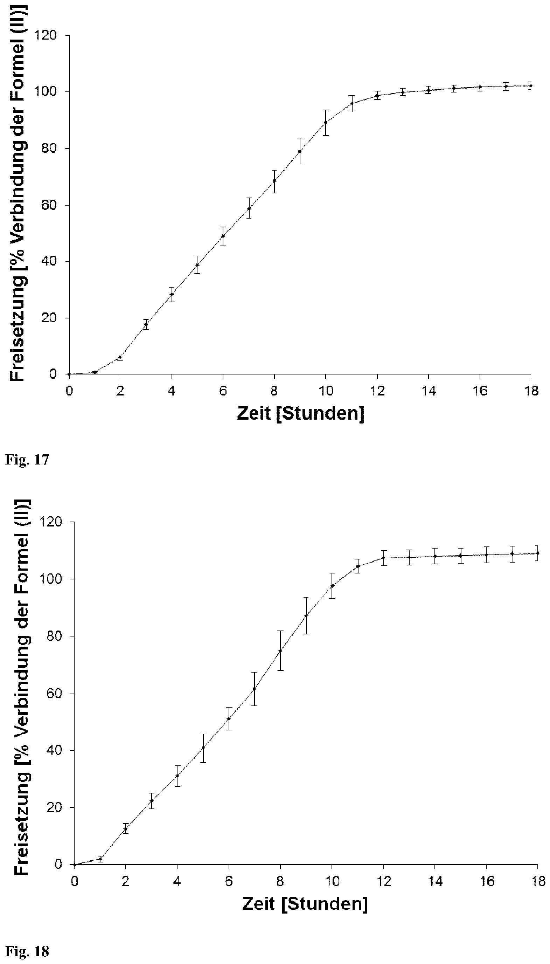

80 % der Verbindung der Formel (II) wurden nach etwa 10 Stunden freigesetzt. Das Freisetzungsprofil der Verbindung der Formel (II) aus dem Osmotischen Freisetzungssystem 6 ist in Fig. 17 dargestellt.

Das Osmotische Freisetzungssystem 6 wurde auf den Gehalt der Verbindung der Formel (II) (Wirkstoffgehalt) geprüft (n=10). Ausgehend von 100 % des deklarierten Wirkstoffgehaltes wurde ein minimaler Wirkstoffgehalt von 96,3 % und ein maximaler Wirkstoffgehalt von 101,2 % gemessen. Die Standardabweichung lag bei 1,4 %.

### Osmotisches Freisetzungssystem 7 (Zweikammersystem, Kollidon VA 64 als hydrophiles quellbares Polymer)

### Tablettenzusammensetzung in mg/Tablette:

### Kern

### Wirkstoffschicht

| | |
|---|---|
| Verbindung der Formel (II), mikronisiert | 5,75 mg |
| Hydroxypropylmethylcellulose (5 cp) | 5,70 mg |
| Kollidon VA 64 | 97,65 mg |
| Hochdisperses Siliziumdioxid (Aerosil 200, Degussa) | 0,90 mg |
| Magnesiumstearat | 0,30 mg |
| | 110,3 mg |

### Osmoseschicht

| | |
|---|---|
| Hydroxypropylmethylcellulose (5 cp) | 3,69 mg |
| Natriumchlorid | 21,51 mg |
| Polyethylenoxid ** | 47,60 mg |
| Eisenoxid rot | 0,72 mg |
| Magnesiumstearat | 0,18 mg |
| | 73,70 mg |
| Gesamt (Kern) | 184,0 mg |

### Hülle

| | |
|---|---|
| Celluloseacetat | 27,00 mg |
| Polyethylenglycol 3350 | 3,00 mg |
| | 30,00 mg |
| Gesamt (osmotisches Freisetzungssystem) | 214,0 mg |

80 % der Verbindung der Formel (II) wurden nach etwa 8,5 Stunden freigesetzt. Das Freisetzungsprofil der Verbindung der Formel (II) aus dem Osmotisches Freisetzungssystem 7 (welches zusätzlich mit 6 mg einer Lackzusammensetzung, wie nachfolgend beschrieben, überzogen ist) ist in Fig. 18 dargestellt.

### Osmotisches Freisetzungssystem 8 (Einkammersystem, Kollidon VA 64 und Xanthangummi als hydrophile quellbare Polymere)

### Tablettenzusammensetzung in mg/Tablette:

### Kern

| | |
|---|---|
| Verbindung der Formel (II), mikronisiert | 6,00 mg |
| Xanthangummi (60 mesh) | 100,0 mg |
| Kollidon VA 64 | 55,0 mg |
| Natriumchlorid | 55,0 mg |
| Natriumbicarbonat | 17.0 mg |
| Natriumcarboxymethylstärke (Explotab) | 23,0 mg |
| Hydroxypropylmethylcellulose (3 cp) | 10,0 mg |
| Hochdisperses Siliziumdioxid (Aerosil 200, Degussa) | 1.50 mg |
| Magnesiumstearat | 1.50 mg |
| Gesamt (Kern) | 269,0 mg |

### Hülle

| | |
|---|---|
| Celluloseacetat | 12,00 mg |
| Polyethylenglycol 3350 | 8,00 mg |
| | 20,00 mg |
| Gesamt (osmotisches Freisetzungssystem) | 289,0 mg |

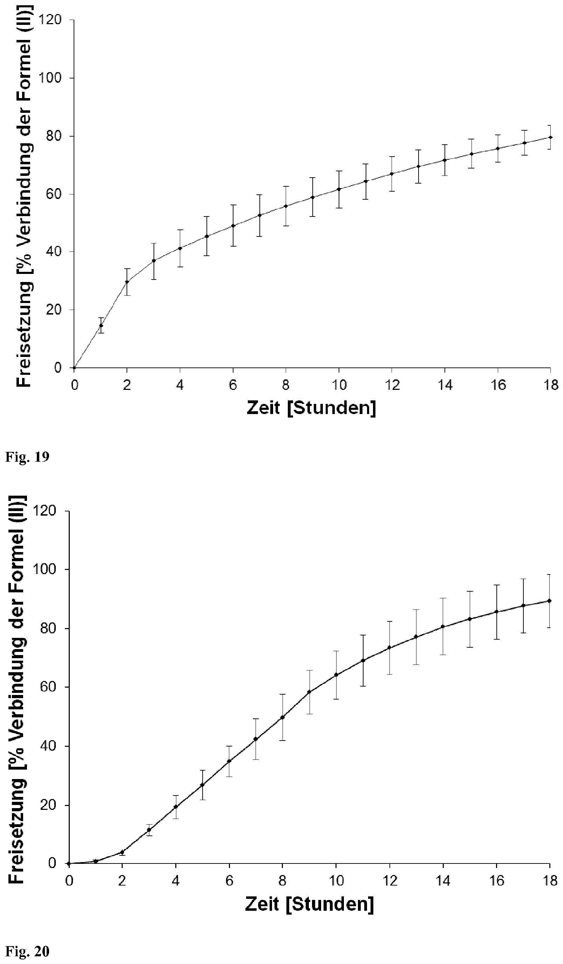

80 % der Verbindung der Formel (II) wurden nach etwa 18 Stunden freigesetzt. Das Freisetzungsprofil der Verbindung der Formel (II) aus dem Osmotisches Freisetzungssystem 8 ist in Fig. 19 dargestellt.

### Osmotisches Freisetzungssystem 9 (Einkammersystem, Polyethylenoxid als hydrophiles quellbares Polymer)

### Tablettenzusammensetzung in mg/Tablette:

### Kern

| | |
|---|---|
| Verbindung der Formel (II), mikronisiert | 5,75 mg |
| Hydroxypropylmethylcellulose (5 cp) | 10,0 mg |
| Polyethylenoxid * | 60,65 mg |
| Polyethylenoxid ** | 57,0 mg |
| Natriumchlorid | 25,0 mg |
| Hochdisperses Siliziumdioxid (Aerosil 200, Degussa) | 1,0 mg |
| Magnesiumstearat | 0,6 mg |
| Gesamt (Kern) | 160,0 mg |

### Hülle

| | |
|---|---|
| Celluloseacetat | 21,6 mg |
| Polyethylenglycol 3350 | 2,4 mg |
| | 24,00 mg |
| Gesamt (osmotisches Freisetzungssystem) | 184,0 mg |

80 % der Verbindung der Formel (II) wurden nach etwa 14 Stunden freigesetzt. Das Freisetzungsprofil der Verbindung der Formel (II) aus dem Osmotisches Freisetzungssystem 9 ist in Fig. 20 dargestellt.
* Viskosität 5 %ige wässrige Lösung (25 °C, Brookfield Viskosimeter Modell RVT, Spindel Nr. 1, Drehzahl: 50 U/min): 40-100 mPa·s (z. B. POLYOX^{™} Water-Soluble Resin NF WSR N-80; Dow)
** Viskosität 1 %ige wässrige Lösung (25 °C, Brookfield Viskosimeter Modell RVF, Spindel Nr. 2, Drehzahl: 2 U/min): 5000-8000 mPa·s (z. B. POLYOX^{™} Water-Soluble Resin NF WSR Coagulant; Dow)

Optional kann auf die dargestellten Osmotischen Freisetzungssysteme ein Lack aufgetragen werden. Für die Osmotischen Freisetzungssysteme 5, 6 und 7 wurde ein Lack mit der im Folgenden dargestellten Zusammensetzung hergestellt und mit einer Menge von 6 mg pro osmotischem Freisetzungssystem aufgetragen.

### Lack

| | |
|---|---|
| Polyvinylalkohol | 2,4 mg |
| Polyethylenglykol 3350 | 1,212 mg |
| Talkum | 0,888 mg |
| Titandioxid | 1,02 mg |
| Eisenoxid gelb | 0,2784 mg |
| Eisenoxid rot | 0,2016 mg |
| Gesamt (Lack) | 6,0 mg |

Dem Fachmann ist bekannt, dass die Lackmenge angepasst werden kann, beispielsweise in Abhängigkeit der Größe und Oberfläche des osmotischen Freisetzungssystems. Die prozentuale Zusammensetzung der Komponenten des Lacks bleibt dabei gleich.

### Herstellung der Osmotischen Freisetzungssysteme 1 bis 6:

Zur Herstellung der Wirkstoffschicht wurde die Verbindung der Formel (II) in mikronisierter Form, Hydroxypropylmethylcellulose (entsprechend der Ph. Eur. (Ausgabewerk 9) Monographie "Hypromellose"; Viskosität 5 mPa·s; gemessen in 2 %iger wässrige Lösung, 25 °C) und Polyethylenoxid (entsprechend der Ph. Eur. (Ausgabewerk 9) Monographie "Macrogols, High Molecular Mass"; Viskosität von 40 bis 100 mPa·s; gemessen in 5%iger wässriger Lösung, 25 °C; POLYOX^{™} Water-Soluble Resin NF WSR N-80; Dow) in einem Mischer gemischt. Diese Vormischung wurde gesiebt, erneut gemischt und anschließend mittels Walzengranulation trocken granuliert und abschließend gesiebt. Das erhaltene Granulat wurde mit hochdispersem Silika (entsprechend der Ph. Eur. (Ausgabewerk 9) Monographie "Silica, colloidal anhydrous"; Siliciumdioxid, Aerosil^{®} 200) gemischt. Nach Zugabe von vorher gesiebtem Magnesiumstearat (entsprechend der Ph. Eur. (Ausgabewerk 9) Monographie "Magnesium Stearate") erfolgte eine abschließende Mischung zur pressfertigen Mischung.

Zur Herstellung der Osmoseschicht wurden Eisenoxid rot (beispielsweise CAS Nummer 1309-37-1), Hydroxypropylmethylcellulose (entsprechend der Ph. Eur. (Ausgabewerk 9) Monographie "Hypromellose"; Viskosität 5 mPa·s; gemessen in 2 %iger wässrige Lösung, 25 °C), Polyethylenoxid (entsprechend der Ph. Eur. (Ausgabewerk 9) Monographie "Macrogols, High Molecular Mass"; Viskosität 5000 bis 8000 mPa·s; gemessen in 1%iger wässriger Lösung, 25 °C; POLYOX^{™} Water-Soluble Resin NF WSR Coagulant; Dow) und Natriumchlorid (entsprechend der Ph. Eur. (Ausgabewerk 9) Monographie "Sodium Chloride") in einem Mischer gemischt. Diese Vormischung wurde trocken granuliert und anschließend gesiebt. Nach Zugabe von vorher gesiebtem Magnesiumstearat (entsprechend der Ph. Eur. (Ausgabewerk 9) Monographie "Magnesium Stearate") erfolgte eine abschließende Mischung zur pressfertigen Mischung.

Die Herstellung der Zweischichttabletten erfolgte durch Tablettierung auf einer Zweischichttablettenpresse. Zuerst wurde die Tablettenpresse auf das Tablettengewicht der Wirkstoffschicht (unterer Tablettenteil) eingestellt. Anschließend wurde das Granulat für die Osmoseschicht (oberer Tablettenteil) dem vorgepressten unteren Tablettenteil zugeführt, um das jeweilige Gesamttablettengewicht des Zweischichtablettenkerns (Durchmesser ca. 8 mm) zu erhalten.

Zur Herstellung der Hülle wurde Celluloseacetat (entsprechend der Ph. Eur. (Ausgabewerk 9) Monographie "Cellulose acetate") in Aceton aufgelöst. Eine wässrige Lösung enthaltend Polyethylenglykol 3350 (entsprechend der Ph. Eur. (Ausgabewerk 9) Monographie "Macrogols"; mittlere Molekularmasse 3350 g/mol) wurde der Celluloseacetat-Lösung zugesetzt und gemischt. Diese Lösung wurde mittels einer für organische Lackierungen geeigneten Lackiereinheit auf die Tablettenkerne der Zweischichttabletten aufgesprüht.

Ein Loch mit einer ungefähren Größe (Durchmesser) von 1 mm wurde mittels, z.B. halbautomatischer Bohrvorrichtung in die Hülle auf der Seite der Wirkstoffschicht gebohrt. Eine Unterscheidung der Wirkstoffschicht von der Osmoseschicht war aufgrund der Farbe möglich. Die Wirkstoffschicht war weiß bis leicht orange. Die Osmoseschicht war aufgrund des zugesetzten Eisenoxid orange-rot.

Optional kann ein Lack aufgetragen werden, der wiederrum optional Hilfsstoffe wie Pigmente zur Farbgebung enthalten kann. Dazu werden Polyvinylalkohol (entsprechend der Ph. Eur. (Ausgabewerk 9) Monographie "Poly(vinyl alcohol)") und Polyethylenglykol 3350 (entsprechend der Ph. Eur. (Ausgabewerk 9) Monographie "Macrogols"; mittlere Molekularmasse 3350 g/mol) in Wasser bei Raumtemperatur gelöst und unter Rühren gemischt. Unter Rühren wird schrittweise Talkum (entsprechend der Ph. Eur. (Ausgabewerk 9) Monographie "Tale"), Titandioxid (entsprechend der Ph. Eur. (Ausgabewerk 9) Monographie "Titanium dioxide") und Eisenoxid (beispielsweise CAS Nummer 1309-37-1 für Eisenoxid rot und CAS Nummern 51274-00-1 oder 20344-49-4 für Eisenoxid gelb) zugegeben. Die erhaltene Lacksuspension wird mit einer geeigneten Lackiereinheit, z.B. Glatt-Coater, auf die Tablettenkerne aufgetragen. Eine solche Lackierung wurde bei den Osmotischen Freisetzungssystemen 5 und 6 vorgenommen.

### Herstellung des Osmotischen Freisetzungssystems 7:

Zur Herstellung der Wirkstoffschicht wurde die Verbindung der Formel (II) in mikronisierter Form, Hydroxypropylmethylcellulose (entsprechend der Ph. Eur. (Ausgabewerk 9) Monographie "Hypromellose"; Viskosität 5 mPa·s; gemessen in 2 %iger wässrige Lösung, 25 °C) und Kollidon VA 64 (entsprechend Ph. Eur. (Ausgabewerk 9) Monographie "Copovidone") in einem Mischer gemischt. Diese Vormischung wurde gesiebt, erneut gemischt und anschließend mittels Walzengranulation trocken granuliert und abschließend gesiebt. Das erhaltene Granulat wurde mit hochdispersem Silika (entsprechend der Ph. Eur. (Ausgabewerk 9) Monographie "Silica, colloidal anhydrous"; Siliciumdioxid, Aerosil^{®} 200) gemischt. Nach Zugabe von vorher gesiebtem Magnesiumstearat (entsprechend der Ph. Eur. (Ausgabewerk 9) Monographie "Magnesium Stearate") erfolgte eine abschließende Mischung zur pressfertigen Mischung.

Zur Herstellung der Osmoseschicht wurden Eisenoxid rot (beispielsweise CAS Nummer 1309-37-1), Hydroxypropylmethylcellulose (entsprechend der Ph. Eur. (Ausgabewerk 9) Monographie "Hypromellose"; Viskosität 5mPa·s; gemessen in 2 %iger wässrige Lösung, 25 °C), Polyethylenoxid (entsprechend der Ph. Eur. (Ausgabewerk 9) Monographie "Macrogols, High Molecular Mass"; Viskosität 5000 bis 8000mPa·s; gemessen in 1%iger wässriger Lösung, 25 °C; POLYOX^{™} Water-Soluble Resin NF WSR N-80; Dow) und Natriumchlorid (entsprechend der Ph. Eur. (Ausgabewerk 9) Monographie "Sodium Chloride") in einem Mischer gemischt.

Diese Vormischung wurde trocken granuliert und anschließend gesiebt. Nach Zugabe von vorher gesiebtem Magnesiumstearat (entsprechend der Ph. Eur. (Ausgabewerk 9) Monographie "Magnesium Stearate") erfolgte eine abschließende Mischung zur pressfertigen Mischung.

Die Herstellung der Zweischichttabletten erfolgte durch Tablettierung auf einer Zweischichttablettenpresse. Zuerst wurde die Tablettenpresse auf das Tablettengewicht der Wirkstoffschicht (unterer Tablettenteil) eingestellt. Anschließend wurde das Granulat für die Osmoseschicht (oberer Tablettenteil) dem vorgepressten unteren Tablettenteil zugeführt, um das jeweilige Gesamttablettengewicht des Zweischichtablettenkerns (Durchmesser ca. 8 mm) zu erhalten.

Zur Herstellung der Hülle wurde Celluloseacetat (entsprechend der Ph. Eur. (Ausgabewerk 9) Monographie "Cellulose acetate") in Aceton aufgelöst. Eine wässrige Lösung enthaltend Polyethylenglykol 3350 (entsprechend der Ph. Eur. (Ausgabewerk 9) Monographie "Macrogols"; mittlere Molekularmasse 3350 g/mol) wurde der Celluloseacetat-Lösung zugesetzt und gemischt. Diese Lösung wurde mittels einer für organische Lackierungen geeigneten Lackiereinheit auf die Tablettenkerne der Zweischichttabletten aufgesprüht.

Ein Loch mit einer ungefähren Größe (Durchmesser) von 1 mm wurde mittels halbautomatischer Bohrvorrichtung in die Hülle auf der Seite der Wirkstoffschicht gebohrt. Eine Unterscheidung der Wirkstoffschicht von der Osmoseschicht war aufgrund der Farbe möglich. Die Wirkstoffschicht war weiß bis leicht orange. Die Osmoseschicht war aufgrund des zugesetzten Eisenoxid orange-rot.

Anschließend erfolgte eine Lackierung mit einem Lack, der Pigmente zur Farbgebung enthielt. Dazu wurden Polyvinylalkohol (entsprechend der Ph. Eur. (Ausgabewerk 9) Monographie "Poly(vinyl alcohol)") und Polyethylenglykol 3350 (entsprechend der Ph. Eur. (Ausgabewerk 9) Monographie "Macrogols"; mittlere Molekularmasse 3350 g/mol) in Wasser bei Raumtemperatur gelöst und unter Rühren gemischt. Unter Rühren wurde schrittweise Talkum (entsprechend der Ph. Eur. (Ausgabewerk 9) Monographie "Talc"), Titandioxid (entsprechend der Ph. Eur. (Ausgabewerk 9) Monographie "Titanium dioxide") und Eisenoxid (beispielsweise CAS Nummer 1309-37-1 für Eisenoxid rot und CAS Nummern 51274-00-1 oder 20344-49-4 für Eisenoxid gelb) zugegeben. Alternativ kann ein Fertiglack gleicher Zusammensetzung in Wasser suspendiert werden. Die erhaltene wässrige Lacksuspension wurde in einer geeigneten Lackiereinheit auf die Tablettenkerne aufgesprüht.

### Herstellung des Osmotischen Freisetzungssystems 8:

Die Verbindung der Formel (II) in mikronisierter Form, Xanthan ("Xanthan FN Lebensmittelqualität normal" (hergestellt durch Jungbunzlauer Ladenburg GmbH) entsprechend Ph. Eur. (Ausgabewerk 9) Monographie "Xanthan gum", Kollidon VA 64 (entsprechend Ph. Eur. (Ausgabewerk 9) Monographie "Copovidone"), Natriumchlorid (entsprechend der Ph. Eur. (Ausgabewerk 9) Monographie "Sodium Chloride"), Natriumbicarbonat und Natriumcarboxymethylstärke (Explotab) wurden in einem Mischer gemischt (Vormischung). Hydroxypropylmethylcellulose (entsprechend der Ph. Eur. (Ausgabewerk 9) Monographie "Hypromellose"; Viskosität 3mPa·s; gemessen in 2 %iger wässrige Lösung, 25 °C) wurde in Wasser gelöst (Granulierflüssigkeit).

Die Vormischung wurde in einen Wirbelschichtgranulator gegeben und in der Wirbelschicht mit der Granulierflüssigkeit granuliert. Anschließend wurde das Granulat in der Wirbelschicht getrocknet. Das getrocknete und gesiebte Granulat wurde mit hochdispersem Silika (entsprechend der Ph. Eur. (Ausgabewerk 9) Monographie "Silica, colloidal anhydrous"; Siliciumdioxid, Aerosil^{®} 200) gemischt. Nach Zugabe von vorher gesiebtem Magnesiumstearat (entsprechend der Ph. Eur. (Ausgabewerk 9) Monographie "Magnesium Stearate") erfolgte eine abschließende Mischung zur pressfertigen Mischung.

Die Tablettierung erfolgte mit einem Tablettendurchmesser von ca. 9mm und einer Tablettenbruchfestigkeit von ca. 50-60 N.

Zur Herstellung der Hülle wurde Celluloseacetat (entsprechend der Ph. Eur. (Ausgabewerk 9) Monographie "Cellulose acetate") in Aceton aufgelöst. Eine wässrige Lösung enthaltend Polyethylenglykol 3350 (entsprechend der Ph. Eur. (Ausgabewerk 9) Monographie "Macrogols"; mittlere Molekularmasse 3350 g/mol) wurde der Celluloseacetat-Lösung zugesetzt und gemischt. Diese Lösung wurde mittels einer für organische Lackierungen geeigneten Lackiereinheit auf die Tablettenkerne aufgesprüht.

Ein Loch mit einer ungefähren Größe (Durchmesser) von 1 mm wurde mittels halbautomatischer Bohrvorrichtung in die Hülle gebohrt.

### Herstellung des Osmotischen Freisetzungssystems 9:

Zur Herstellung der Tablettenkerne wurde die Verbindung der Formel (II) in mikronisierter Form, Hydroxypropylmethylcellulose (entsprechend der Ph. Eur. (Ausgabewerk 9) Monographie "Hypromellose"; Viskosität 5 mPa·s; gemessen in 2 %iger wässrige Lösung, 25 °C), Natriumchlorid (entsprechend der Ph. Eur. (Ausgabewerk 9) Monographie "Sodium Chloride") und Poyethylenoxid in einem Mischer gemischt. Diese Vormischung wurde gesiebt, erneut gemischt und anschließend mit hochdispersem Silika (entsprechend der Ph. Eur. (Ausgabewerk 9) Monographie "Silica, colloidal anhydrous"; Siliciumdioxid, Aerosil^{®} 200) gemischt. Nach Zugabe von vorher gesiebtem Magnesiumstearat (entsprechend der Ph. Eur. (Ausgabewerk 9) Monographie "Magnesium Stearate") erfolgte eine abschließende Mischung zur pressfertigen Mischung. Alternativ kann die Vormischung mittels Walzengranulation trocken granuliert und abschließend gesiebt werden.

Die Tablettierung erfolgte mit einem Tablettendurchmesser von 8 mm und einer Tablettenbruchfestigkeit von ca. 80-110 N.

Zur Herstellung der Hülle wurde Celluloseacetat (entsprechend der Ph. Eur. (Ausgabewerk 9) Monographie "Cellulose acetate") in Aceton aufgelöst. Eine wässrige Lösung enthaltend Polyethylenglykol 3350 (entsprechend der Ph. Eur. (Ausgabewerk 9) Monographie "Macrogols"; mittlere Molekularmasse 3350 g/mol) wurde der Celluloseacetat-Lösung zugesetzt und gemischt. Diese Lösung wurde mittels einer für organische Lackierungen geeigneten Lackiereinheit auf die Tablettenkerne aufgesprüht.

Ein Loch mit einer ungefähren Größe (Durchmesser) von 1 mm wurde mittels z.B. halbautomatischer Bohrvorrichtung in die Hülle gebohrt.

Falls nicht näher spezifiziert, bezeichnen die verwendeten Stoffe zur Herstellung der Osmotischen Freisetzungssysteme dem Fachmann unter der genannten Bezeichnung bekannte pharmazeutische Hilfsstoffe und genügen, wenn in einem der Arzneibücher aufgeführt, den jeweiligen Anforderungen der Arzneibuch-Monographien des europäischen (Ph. Eur 9), amerikanischen (USP 41 and NF 36) und / oder japanischen (JP, 17. Auflage) Arzneibuchs.

### Freisetzungsverhalten

Die Freisetzung des Wirkstoffs aus den Tabletten wurde nach der Methode der US Pharmacopoeia USP 39 (Kapitel <711> Dissolution) unter Verwendung der Apparatur 2 (Paddle Test) bestimmt. Zur Bestimmung der Freisetzungsgeschwindigkeit wurde in jedes Freisetzungsgefäß der USP Apparatur 2 eine Tablette eingebracht und die in Lösung gegangene Menge an Wirkstoff nach Abfiltrieren der ungelösten Bestandteile mittels HPLC bestimmt. Als Freisetzungsmedium wurde Phosphatpuffer pH 6.8 ohne Zusatz von Tensid verwendet und der Blattrührer der USP Apparatur 2 hatte eine Umdrehungsgeschwindigkeit von 100 Umdrehungen pro Minute. Wenn nicht anders angegeben wurde die Freisetzungsgeschwindigkeit von mindestens sechs Prüfkörpern bestimmt. Es wird jeweils der Mittelwert der freigesetzten Menge an Wirkstoff angegeben.

Fig. 12 zeigt die prozentuale Freisetzung der Verbindung der Formel (II) aus den Osmotischen Freisetzungssystem 1 in Abhängigkeit der Zeit.

Fig. 13 zeigt die prozentuale Freisetzung der Verbindung der Formel (II) aus den Osmotischen Freisetzungssystem 2 in Abhängigkeit der Zeit.

Fig. 14 zeigt die prozentuale Freisetzung der Verbindung der Formel (II) aus den Osmotischen Freisetzungssystem 3 in Abhängigkeit der Zeit.

Fig. 15 zeigt die prozentuale Freisetzung der Verbindung der Formel (II) aus den Osmotischen Freisetzungssystem 4 in Abhängigkeit der Zeit.

Fig. 16 zeigt die prozentuale Freisetzung der Verbindung der Formel (II) aus den Osmotischen Freisetzungssystem 5 in Abhängigkeit der Zeit.

Fig. 17 zeigt die prozentuale Freisetzung der Verbindung der Formel (II) aus den Osmotischen Freisetzungssystem 6 in Abhängigkeit der Zeit.

Fig. 18 zeigt die prozentuale Freisetzung der Verbindung der Formel (II) aus den Osmotischen Freisetzungssystem 7 in Abhängigkeit der Zeit.

Fig. 19 zeigt die prozentuale Freisetzung der Verbindung der Formel (II) aus den Osmotischen Freisetzungssystem 8 in Abhängigkeit der Zeit.

Fig. 20 zeigt die prozentuale Freisetzung der Verbindung der Formel (II) aus den Osmotischen Freisetzungssystem 9 in Abhängigkeit der Zeit.

### Thermoanalytische Untersuchung binärer physikalischer Mischungen

Um Kompartibilitäten in thermoanalytischen Untersuchungen darzustellen, wurden die Verbindungen der Formel (II) und (I) mit hydrophilen quellbaren Polymeren zu gleichen Teilen in einer Fantaschale vorgelegt und mittels Pistill zu einer homogenen Pulvermischung verrieben (Verreibung im Verhältnis 1:1, binäre Mischung). Als hydrophile quellbare Polymere wurden Polyethylenoxid (entsprechend der Ph. Eur. (Ausgabewerk 9) Monographie "Macrogols, High Molecular Mass"; Viskosität von 40 bis 100 mPa·s; gemessen in 5%iger wässriger Lösung, 25 °C; POLYOX^{™} Water-Soluble Resin NF WSR N-80; Dow), Xanthan ("Xanthan FN Lebensmittelqualität normal" hergestellt durch Jungbunzlauer Ladenburg GmbH), entsprechend Ph. Eur. (Ausgabewerk 9) Monographie "Xanthan gum"),Vinylpyrrolidon-Vinylacetat-Copolymer (Kollidon VA 64), entsprechend Ph. Eur. (Ausgabewerk 9) Monographie "Copovidone", Polyvinylpyrrolidon (PVP 25), entsprechend der Ph. Eur. (Ausgabewerk 9) Monographie "Povidone", Methacrylsäure-methylmethacrylat-Copolymer (Eudragit^{®} L100), entsprechend Ph. Eur. (Ausgabewerk 9) Monographie "Methacrylic acid - Methyl Methacrylate Copolymer (1:1), Methacrylsäure-methylmethacrylat-Copolymer (Eudragit^{®} RL PO) entsprechend Ph. Eur. (Ausgabewerk 9) Monographie "Ammonio Methacrylate Copolymer (TYPE A), Hydroxypropylcellulose (HPC LM Nisso), entsprechend der Ph. Eur. (Ausgabewerk 9) Monographie "Hydroxypropylcellulose" und Polyacrylsäure (entsprechend der Ph. Eur. (Ausgabewerk 9) Monographie "Carbomers"; Bezeichnung: *Polyacrylic acid, MW 1,080,000, aver. MN 135,000;* Acros Organics) untersucht.

Die pysikalischen Mischungen, sowie die jeweiligen Einzelkomponeneten wurden thermoanalytisch charakterisiert. Die Thermogramme wurden auf einem DSC (dynamisches Differenzkalorimeter, englisch: *differential scanning calorimeter*) aufgenommen. Dazu wurden jeweils ca. 5 mg der Probe in einem Aluminiumtiegel unter Stickstoff (50 ml/min) mit einer Heizrate von 10 K/min bis zum Ende der Schmelztemperatur der jeweiligen Verbindung erhitzt.

Fig. 1 zeigt Thermogramme der Verbindung der Formel (I), von Polyethylenoxid und von der binären Mischung aus der Verbindung der Formel (I) mit Polyethylenoxid.

Fig. 2 zeigt Thermogramme der Verbindung der Formel (II), von Polyethylenoxid und von der binären Mischung aus der Verbindung der Formel (II) mit Polyethylenoxid.

Fig. 3 zeigt Thermogramme der Verbindung der Formel (II), von Xanthan und von der binären Mischung aus der Verbindung der Formel (II) mit Xanthan.

Fig. 4 zeigt Thermogramme der Verbindung der Formel (II), von Vinylpyrrolidon-Vinylacetat-Copolymer und von der binären Mischung aus der Verbindung der Formel (II) mit Vinylpyrrolidon-Vinylacetat-Copolymer.

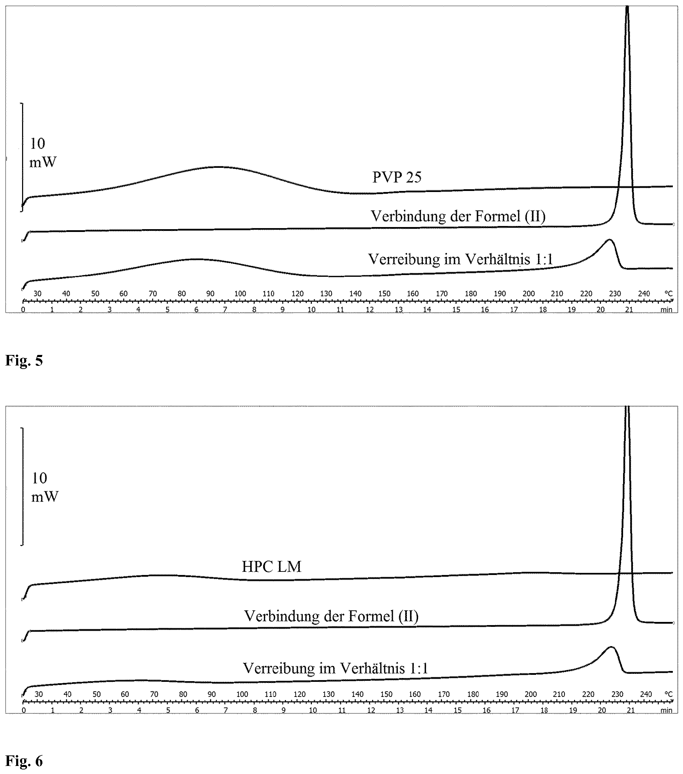

Fig. 5 zeigt Thermogramme der Verbindung der Formel (II), von PVP25 und von der binären Mischung aus der Verbindung der Formel (II) mit PVP25.

Fig. 6 zeigt Thermogramme der Verbindung der Formel (II), von HPC LM und von der binären Mischung aus der Verbindung der Formel (II) mit HPC LC.

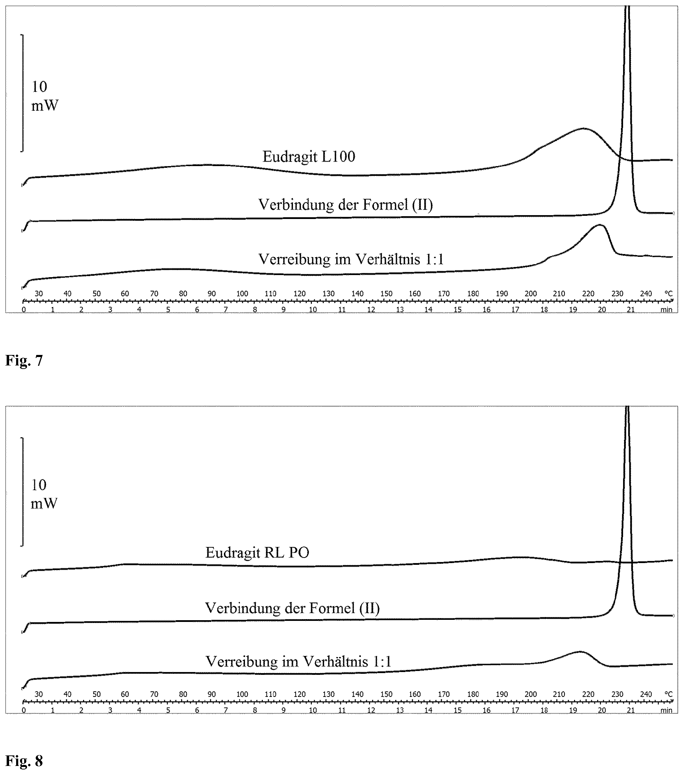

Fig. 7 zeigt Thermogramme der Verbindung der Formel (II), von Eudragit L100 und von der binären Mischung aus der Verbindung der Formel (II) mit Eudragit L100.

Fig. 8 zeigt Thermogramme der Verbindung der Formel (II), von Eudragit RL PO und von der binären Mischung aus der Verbindung der Formel (II) mit Eudragit RL PO.

Fig. 9 zeigt Thermogramme der Verbindung der Formel (II), von Polyacrylsäure und von der binären Mischung aus der Verbindung der Formel (II) mit Polyacrylsäure.

## Patentansprüche

1. Osmotisches Freisetzungssystem bestehend aus einem Kern und einer Hülle, wobei die Hülle aus einem wasserdurchlässigen, für die Komponenten des Kerns undurchlässigen Material mit mindestens einer Öffnung besteht und wobei der Kern Natrium-(3S)-3-(4-chlor-3-{[(2S,3R)-2-(4-chlorphenyl)-4,4,4-trifluor-3-methylbutanoyl]amino}phenyl)-3-cyclopropylpropanoat der Formel (II) 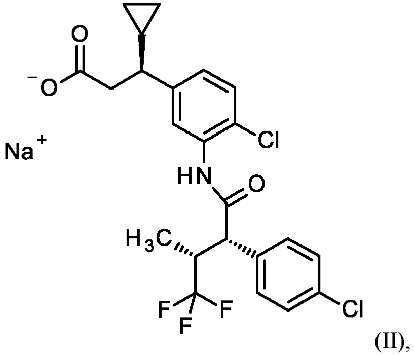 und mindestens ein hydrophiles, quellbares Polymer enthält, wobei das hydrophile, quellbare Polymer ausgewählt ist aus der Liste bestehend aus Polyethylenoxid, Xanthan, Cellulosederivate, beispielsweise Hydroxypropylcellulose, Hydroxypropylmethylcellulose oder Natriumcarboxymethylcellulose, Vinylpyrrolidon-Vinylacetat-Copolymer, Polyvinylpyrrolidon, Methacrylsäure-Copolymere, beispielsweise Methacrylsäure-methylmethacrylat-Copolymer und Polyacrylsäuren.

2. Osmotisches Freisetzungssystem gemäß Anspruch 1, wobei der Kern ein Zweikammersystem, bestehend aus einer Wirkstoffschicht und einer Osmoseschicht umfasst.

3. Osmotisches Freisetzungssystem gemäß Anspruch 2, wobei die Wirkstoffschicht
• 1 Gew.-% bis 50 Gew.-% der Verbindung der Formel (II),
• 20 Gew.-% bis 99 Gew.-% von mindestens einem hydrophilen quellbaren Polymer, gegebenenfalls mindestens einen osmotisch aktiven Zusatz und gegebenenfalls mindestens einen pharmazeutisch üblichen Hilfsstoff enthält,
und die Osmoseschicht
• 40 Gew.-% bis 90 Gew.-% von mindestens einem hydrophilen quellbaren Polymer,
• 10 Gew.-% bis 60 Gew.-% eines osmotischen aktiven Zusatzes,
und gegebenenfalls mindestens einen pharmazeutisch üblichen Hilfsstoff enthält.

4. Osmotisches Freisetzungssystem gemäß einem der Ansprüche 1 bis 3, wobei das mindestens eine hydrophile quellbare Polymer Polyethylenoxid ist.

5. Verfahren zur Herstellung eines osmotischen Freisetzungssystems gemäß einem der Ansprüche 1 bis 4, **dadurch gekennzeichnet, dass** die Komponenten des Kerns miteinander vermischt, granuliert und tablettiert werden, der so entstandene Kern mit einer Hülle beschichtet wird und die Hülle abschließend mit einer oder mehreren Öffnungen, geeignet zum Austritt der Verbindung der Formel (II), versehen wird.

6. Verfahren zur Herstellung eines osmotischen Freisetzungssystem gemäß einem der Ansprüche 2 bis 4, **dadurch gekennzeichnet, dass**
• die Komponenten der Wirkstoffschicht gemischt und granuliert werden und
• die Komponenten der Osmoseschicht gemischt und granuliert werden,
• anschließend auf einer Zweischichttablettenpresse beide Granulate zu einer Zweischichttablette verpresst werden,
• der so entstandene Kern dann mit der Hülle beschichtet wird und
• die Hülle auf der Wirkstoffseite mit einer oder mehreren Öffnungen versehen wird.

7. Verbindung der Formel (II) in kristalliner Form der Modifikation 1, **dadurch gekennzeichnet, dass** das Röntgendiffraktogramm der Verbindung Peakmaxima des 2 Theta Winkels bei 8,1; 17,2; 18,8; 22,3; 22,6° zeigt.

8. Verbindung der Formel (II), nach Anspruch 7, in kristalliner Form der Modifikation 1, **dadurch gekennzeichnet, dass** das IR-Spektrum der Verbindung Bandenmaxima bei 3381, 1691, 1565, 1524, 1419 cm⁻¹ zeigt.

9. Herstellung der Verbindung der Formel (II) in kristalliner Modifikation 1 nach einem der Ansprüche 7 oder 8, **dadurch gekennzeichnet, dass** die Verbindung der Formel (I) 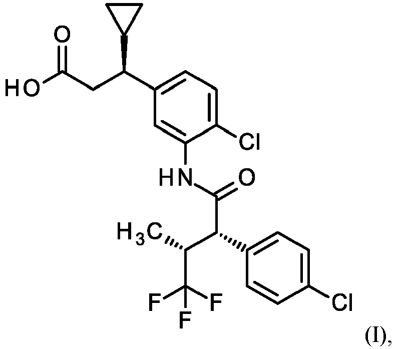 gelöst vorliegend in einem polaren und aprotischen Lösungsmittel, mit einer Base, ausgewählt aus einer Liste bestehend aus Natriumhydroxid oder einem sterisch anspruchsvollen Natriumalkoholat versetzt wird und nach Rühren der ausgefällte Feststoff isoliert und getrocknet wird.

10. Verbindung gemäß einem der Ansprüche 7 bis 8 zur Behandlung und/oder Prävention von Krankheiten.

11. Verbindung gemäß einem der Ansprüche 7 bis 8 zur Behandlung und/oder Prävention von renalen und kardiorenalen Erkrankungen, insbesondere des chronischen Nierenversagens (CKD) und der diabetischen Nierenkrankheit (DKD), kardialen und kardiovaskulären Erkrankungen, insbesondere der Herzinsuffizienz (HFpEF und HFrEF), Herzinfarkt, Angina pectoris, Kardiomyopathien, Hypertonie und Arteriosklerose, pulmonaler und kardiopulmonaler Erkrankungen, insbesondere pulmonaler Hypertonie (PH), ophthalmologischen Erkrankungen, insbesondere der nicht proliferativen diabetischen Retinopathie (NPDR) und des diabetisches Makula-Ödems (DME), Erkrankungen des Zentralnervensystems, insbesondere der Demenz, Knochenerkrankungen, insbesondere Osteogenesis imperfecta, thromboembolischen Erkrankungen, Muskeldystrophien, Ischämien, Gefäßerkrankungen, Mikrozirkulationsstörungen, fibrotischen Erkrankungen insbesondere der Systemischen Sklerose, inflammatorischen Erkrankungen, und Stoffwechselerkrankungen, insbesondere des Metabolischen Syndroms, der Dyslipidämie und Diabetes.

12. Arzneimittel enthaltend die Verbindung, wie in einem der Ansprüche 7 bis 9 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln, MR-Antagonisten, IP-Rezeptor-Agonisten, entzündungshemmenden Wirkstoffen, Antidementiva, Antidiabetika, den Fettstoffwechsel verändernden Wirkstoffen sowie Wirkstoffen zur Behandlung von Knochen- und Muskelerkrankungen.

13. Osmotisches Freisetzungssystem nach einem der Ansprüche 1 bis 4 zur Behandlung und/oder Prävention von renalen und kardiorenalen Erkrankungen, insbesondere des chronischen Nierenversagens (CKD) und der diabetischen Nierenkrankheit (DKD), kardialen und kardiovaskulären Erkrankungen, insbesondere der Herzinsuffizienz (HFpEF und HFrEF), Herzinfarkt, Angina pectoris, Kardiomyopathien, Hypertonie und Arteriosklerose, pulmonaler und kardiopulmonaler Erkrankungen, insbesondere pulmonaler Hypertonie (PH), ophthalmologischen Erkrankungen, insbesondere der nicht proliferativen diabetischen Retinopathie (NPDR) und des diabetisches Makula-Ödems (DME), Erkrankungen des Zentralnervensystems, insbesondere der Demenz, Knochenerkrankungen, insbesondere Osteogenesis imperfecta, thromboembolischen Erkrankungen, Muskeldystrophien, Ischämien, Gefäßerkrankungen, Mikrozirkulationsstörungen, fibrotischen Erkrankungen insbesondere der Systemischen Sklerose, inflammatorischen Erkrankungen, und Stoffwechselerkrankungen, insbesondere des Metabolischen Syndroms, der Dyslipidämie und Diabetes.

## Claims

1. Osmotic release system consisting of a core and a shell, where the shell consists of a water-permeable material impermeable for the components of the core and has at least one orifice, and where the core comprises sodium (3S)-3-(4-chloro-3-{[(2S,3R)-2-(4-chlorophenyl)-4,4,4-trifluoro-3-methylbutanoyl]amino}phenyl)-3-cyclopropyl-propanoate of the formula (II) 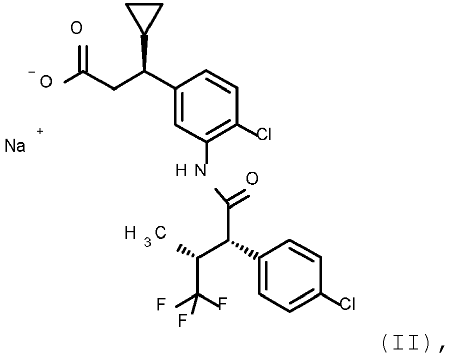 and at least one hydrophilic swellable polymer, where the hydrophilic swellable polymer is selected from the list consisting of polyethylene oxide, xanthan, cellulose derivatives, for example hydroxypropylcellulose, hydroxypropylmethylcellulose or sodium carboxymethylcellulose, vinylpyrrolidone/vinyl acetate copolymer, polyvinylpyrrolidone, methacrylic acid copolymers, for example methacrylic acid-methyl methacrylate copolymer, and polyacrylic acids.

2. Osmotic release system according to Claim 1, where the core comprises a two-chamber system consisting of an active ingredient layer and an osmosis layer.

3. Osmotic release system according to Claim 2, where the active ingredient layer comprises
• 1% by weight to 50% by weight of the compound of the formula (II),
• 20% by weight to 99% by weight of at least one hydrophilic swellable polymer,
optionally at least one osmotically active additive and optionally at least one pharmaceutically customary auxiliary,
and the osmosis layer comprises
• 40% by weight to 90% by weight of at least one hydrophilic swellable polymer,
• 10% by weight to 60% by weight of an osmotically active additive
and optionally at least one pharmaceutically customary auxiliary.

4. Osmotic release system according to any of Claims 1 to 3, where the at least one hydrophilic swellable polymer is polyethylene oxide.

5. Process for preparing an osmotic release system according to any of Claims 1 to 4, **characterized in that** the components of the core are mixed with one another, granulated and tabletted, the resulting core is coated with a shell and the shell is finally provided with one or more orifices suitable for the compound of the formula (II) exiting.

6. Process for preparing an osmotic release system according to any of Claims 2 to 4, **characterized in that**
• the components of the active ingredient layer are mixed and granulated and
• the components of the osmosis layer are mixed and granulated,
• both sets of granules are subsequently compressed on a bilayer tablet press to give a bilayer tablet,
• the resulting core is then coated with the shell and
• the shell is, on the active ingredient side, provided with one or more orifices.

7. Compound of the formula (II) in crystalline form of modification 1, **characterized in that** the X-ray diffractogram of the compound has peak maxima of the 2 theta angle at 8.1, 17.2, 18.8, 22.3 and 22.6°.

8. Compound of the formula (II) according to Claim 7, in crystalline form of modification 1, **characterized in that** the IR spectrum of the compound has band maxima at 3381, 1691, 1565, 1524 and 1419 cm⁻¹.

9. Preparation of the compound of the formula (II) in crystalline modification 1 according to any of Claims 7 or 8, **characterized in that** the compound of the formula (I) 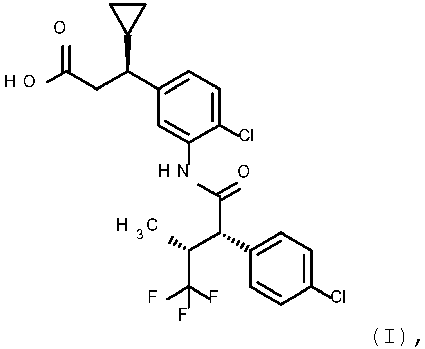 dissolved in the present case in a polar aprotic solvent, has a base selected from a list consisting of sodium hydroxide or a sterically demanding sodium alkoxide added to it and the precipitated solid is, after stirring, isolated and dried.

10. Compound according to any of Claims 7 to 8 for the treatment and/or prevention of diseases.

11. Compound according to any of Claims 7 to 8 for the treatment and/or prevention of renal and cardiorenal disorders, in particular chronic kidney disease (CKD) and diabetic kidney disease (DKD), cardiac and cardiovascular disorders, in particular heart failure (HFpEF and HFrEF), myocardial infarction, angina pectoris, cardiomyopathies, hypertension and arteriosclerosis, pulmonary and cardiopulmonary disorders, in particular pulmonary hypertension (PH), ophthalmic disorders, in particular non-proliferative diabetic retinopathy (NPDR) and diabetic macular oedema (DME), disorders of the central nervous system, in particular dementia, bone disorders, in particular osteogenesis imperfecta, thromboembolic disorders, muscular dystrophies, ischaemias, vascular disorders, impaired microcirculation, fibrotic disorders, in particular systemic sclerosis, inflammatory disorders, and metabolic disorders, in particular metabolic syndrome, dyslipidaemia and diabetes.

12. Medicament, comprising the compound as defined in any of Claims 7 to 8 in combination with one or more other active compounds selected from the group consisting of organic nitrates, NO donors, cGMP-PDE inhibitors, stimulators of guanylate cyclase, antithrombotics, antihypertensive agents, MR antagonists, IP receptor agonists, active compounds having anti-inflammatory action, antidementives, antidiabetics, active compounds which modify fat metabolism and active compounds for the treatment of bone and muscle disorders.

13. Osmotic release system according to any of Claims 1 to 4 for the treatment and/or prevention of renal and cardiorenal disorders, in particular chronic kidney disease (CKD) and diabetic kidney disease (DKD), cardiac and cardiovascular disorders, in particular heart failure (HFpEF and HFrEF), myocardial infarction, angina pectoris, cardiomyopathies, hypertension and arteriosclerosis, pulmonary and cardiopulmonary disorders, in particular pulmonary hypertension (PH), ophthalmic disorders, in particular non-proliferative diabetic retinopathy (NPDR) and diabetic macular oedema (DME), disorders of the central nervous system, in particular dementia, bone disorders, in particular osteogenesis imperfecta, thromboembolic disorders, muscular dystrophies, ischaemias, vascular disorders, impaired microcirculation, fibrotic disorders, in particular systemic sclerosis, inflammatory disorders, and metabolic disorders, in particular metabolic syndrome, dyslipidaemia and diabetes.

## Revendications

1. Système osmotique de libération, constitué par un noyau et une enveloppe, l'enveloppe étant constituée par un matériau perméable à l'eau, imperméable aux composants du noyau, présentant au moins une ouverture et le noyau contenant du (3S)-3-(4-chloro-3-{ [(2S,3R)-2-(4-chlorophényl)-4,4,4-trifluoro-3-méthylbutanoyl]amino}phényl)-3-cyclopropylpropanoate de sodium de formule (II) 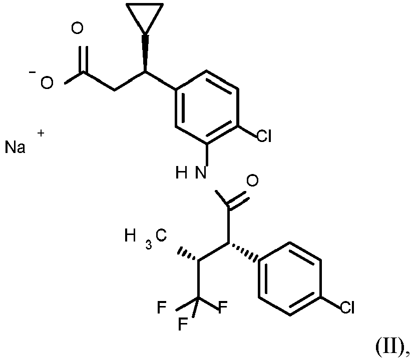 et au moins un polymère gonflable, hydrophile, le polymère gonflable, hydrophile étant choisi dans la liste constituée par le poly(oxyde d'éthylène), le xanthane, les dérivés de cellulose, par exemple l'hydroxypropylcellulose, l'hydroxypropylméthylcellulose ou la carboxyméthylcellulose sodique, le copolymère de vinylpyrrolidone-acétate de vinyle, la polyvinylpyrrolidone, les copolymères de l'acide méthacrylique, par exemple un copolymère de l'acide méthacrylique-méthacrylate de méthyle et les poly(acides acryliques).

2. Système osmotique de libération selon la revendication 1, le noyau comprenant un système à deux chambres, constitué par une couche de principe actif et une couche osmotique.

3. Système osmotique de libération selon la revendication 2, la couche de principe actif contenant
- 1% en poids à 50% en poids du composé de formule (II)
- 20% en poids à 99% en poids d'au moins un polymère gonflable hydrophile,
le cas échéant au moins un additif osmotiquement actif et le cas échéant au moins un adjuvant pharmaceutiquement usuel,
et la couche osmotique contenant
- 40% en poids à 90% en poids d'au moins un polymère gonflable hydrophile,
- 10 à 60% d'un additif osmotiquement actif
et le cas échéant au moins un adjuvant pharmaceutiquement usuel.

4. Système osmotique de libération selon l'une quelconque des revendications 1 à 3, ledit au moins un polymère gonflable hydrophile étant du poly(oxyde d'éthylène).

5. Procédé pour la préparation d'un système osmotique de libération selon l'une quelconque des revendications 1 à 4, **caractérisé en ce que** les composants du noyau sont mélangés les uns avec les autres, granulés et transformés en comprimés, le noyau ainsi formé est revêtu d'une enveloppe et l'enveloppe est ensuite pourvue d'une ou de plusieurs ouvertures appropriées pour la sortie du composé de formule (II).

6. Procédé pour la préparation d'un système osmotique de libération selon l'une quelconque des revendications 2 à 4, **caractérisé en ce que**
- les composants de la couche de principe actif sont mélangés et granulés et
- les composants de la couche osmotique sont mélangés et granulés,
- les deux granulats sont ensuite comprimés sur une presse à comprimés à deux couches en un comprimé à deux couches,
- le noyau ainsi formé est ensuite revêtu de l'enveloppe et
- l'enveloppe est pourvue d'une ou de plusieurs ouvertures sur le côté du principe actif.

7. Composé de formule (II) sous forme cristalline de la modification 1, **caractérisé en ce que** le diffractogramme aux rayons X du composé présente des maximums de pic de l'angle 2 thêta à 8,1 ; 17,2 ; 18,8 ; 22,3 ; 22,6°.

8. Composé de formule (II) selon la revendication 7, sous forme cristalline de la modification 1, **caractérisé en ce que** le spectre IR du composé présente des maximums de bande à 3381, 1691, 1565, 1524, 1419 cm⁻¹.

9. Préparation du composé de formule (II) sous forme cristalline de la modification 1 selon l'une quelconque des revendications 7 et 8, **caractérisé en ce que** le composé de formule (I) 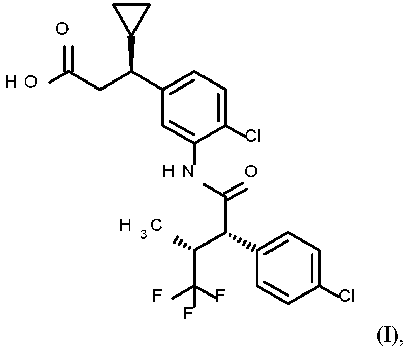 se trouvant sous forme dissoute dans un solvant polaire et aprotique, est additionné d'une base, choisie dans une liste constituée par l'hydroxyde de sodium ou un alcoolate sodique stérilement encombré et, après agitation, le solide précipité est isolé et séché.

10. Composé selon l'une quelconque des revendications 7 et 8 pour le traitement et/ou la prévention de maladies.

11. Composé selon l'une quelconque des revendications 7 et 8 pour le traitement et/ou la prévention de maladies rénales et cardio-rénales, en particulier l'insuffisance rénale chronique (IRC) et la maladie rénale diabétique (MRD), de maladies cardiaques et cardiovasculaires, en particulier l'insuffisance cardiaque (HFpEF et HFrEF), l'infarctus cardiaque, l'angine de poitrine, les cardiomyopathies, l'hypertonie et l'artériosclérose, de maladies pulmonaires et cardiopulmonaires, en particulier l'hypertonie pulmonaire (HP), de maladies ophtalmologiques, en particulier la rétinopathie diabétique non proliférante (RDNP) et l'œdème maculaire diabétique (OMD), de maladies du système nerveux central, en particulier la démence, de maladies osseuses, en particulier l'ostéogénèse imparfaite, de maladies thrombo-emboliques, des dystrophies musculaires, des ischémies, de maladies vasculaires, des troubles de la microcirculation, de maladies fibrotiques, en particulier la sclérose systémique, de maladies inflammatoires et de maladies du métabolisme, en particulier le syndrome métabolique, la dyslipidémie et le diabète.

12. Médicament contenant le composé tel que défini dans l'une quelconque des revendications 7 et 8, en combinaison avec un ou plusieurs autres principes actifs choisis dans le groupe constitué par les nitrates organiques, les donneurs de NO, les inhibiteurs de cGMP-PDE, les stimulateurs de la guanylate-cyclase, les agents à effet antithrombotique, les agents hypotenseurs, les antagonistes du RM, les agonistes du récepteur IP, les principes actifs anti-inflammatoires, les agents antidémence, les antidiabétiques, les agents modifiant le métabolisme ainsi que les principes actifs pour le traitement de maladies osseuses et musculaires.

13. Système osmotique de libération selon l'une quelconque des revendications 1 à 4 pour le traitement et/ou la prévention de maladies rénales et cardio-rénales, en particulier l'insuffisance rénale chronique (IRC) et la maladie rénale diabétique (MRD), de maladies cardiaques et cardiovasculaires, en particulier l'insuffisance cardiaque (HFpEF et HFrEF), l'infarctus cardiaque, l'angine de poitrine, les cardiomyopathies, l'hypertonie et l'artériosclérose, de maladies pulmonaires et cardiopulmonaires, en particulier l'hypertonie pulmonaire (HP), de maladies ophtalmologiques, en particulier la rétinopathie diabétique non proliférante (RDNP) et l'œdème maculaire diabétique (OMD), de maladies du système nerveux central, en particulier la démence, de maladies osseuses, en particulier l'ostéogénèse imparfaite, de maladies thrombo-emboliques, des dystrophies musculaires, des ischémies, de maladies vasculaires, des troubles de la microcirculation, de maladies fibrotiques, en particulier la sclérose systémique, de maladies inflammatoires et de maladies du métabolisme, en particulier le syndrome métabolique, la dyslipidémie et le diabète.
